(19) Europäisches Patentamt
European Patent Office
Office européen des brevets

(11) EP 1 524 275 A2

(12) **EUROPEAN PATENT APPLICATION**

(43) Date of publication:
**20.04.2005 Bulletin 2005/16**

(21) Application number: **04024673.8**

(22) Date of filing: **15.10.2004**

(51) Int Cl.[7]: **C07K 16/18**, C12N 15/63, C12N 15/13, C12N 5/10, A61K 39/395

(84) Designated Contracting States:
**AT BE BG CH CY CZ DE DK EE ES FI FR GB GR HU IE IT LI LU MC NL PL PT RO SE SI SK TR**
Designated Extension States:
**AL HR LT LV MK**

(30) Priority: **16.10.2003 EP 03023580**

(71) Applicants:
- **Micromet AG**
  **81477 München (DE)**
- **Biovation Limited**
  **Aberdeen AB22 8GU (GB)**

(72) Inventors:
- **Hofmeister, Robert**
  **82110 Germering (DE)**
- **Bäuerle, Patrick**
  **82131 Gauting (DE)**
- **Itin, Christian**
  **82166 Gräfelfing (DE)**
- **Hamilton, Anita A.**
  **Aberdeen AB15 4EQ (GB)**
- **Carr, Francis J.**
  **Aberdeenshire AB23 8XU (GB)**
- **Williams, Stephen**
  **Aberdeenshire AB33 8HJ (GB)**

(74) Representative: **VOSSIUS & PARTNER**
**Siebertstrasse 4**
**81675 München (DE)**

(54) **Deimmunized binding molecules to CD3**

(57) The invention provides CD3 specific binding molecules and nucleic acid sequences encoding said CD3 specific binding molecules. Further aspects of the invention are vectors and host cells comprising said nucleic acid sequence, a process for the production of the construct of the invention and compositions comprising said construct. The invention also provides the use of said constructs for the preparation of pharmaceutical compositions for the treatment of particular diseases and a kit comprising the binding construct of the invention.

EP 1 524 275 A2

SEQUENCE LISTING

<110> Micromet AG; Biovation Ltd.

<120> Deimmunized binding molecules to CD3

<130> H2783 EP

<160> 73

<170> PatentIn version 3.1

<210> 1
<211> 729
<212> DNA
<213> artificial sequence

<220>
<223> wt Anti-CD3 cassette

<400> 1
gatatcaaac tgcagcagtc aggggctgaa ctggcaagac ctggggcctc agtgaagatg 60

tcctgcaaga cttctggcta cacctttact aggtacacga tgcactgggt aaaacagagg 120

cctggacagg gtctggaatg gattggatac attaatccta gccgtggtta tactaattac 180

aatcagaagt tcaaggacaa ggccacattg actacagaca aatcctccag cacagcctac 240

atgcaactga gcagcctgac atctgaggac tctgcagtct attactgtgc aagatattat 300

gatgatcatt actgccttga ctactggggc caaggcacca ctctcacagt ctcctcagtc 360

gaaggtggaa gtggaggttc tggtggaagt ggaggttcag gtggagtcga cgacattcag 420

ctgacccagt ctccagcaat catgtctgca tctccagggg agaaggtcac catgacctgc 480

agagccagtt caagtgtaag ttacatgaac tggtaccagc agaagtcagg cacctccccc 540

aaaagatgga tttatgacac atccaaagtg gcttctggag tcccttatcg cttcagtggc 600

agtgggtctg ggacctcata ctctctcaca atcagcagca tggaggctga agatgctgcc 660

acttattact gccaacagtg gagtagtaac ccgctcacgt tcggtgctgg gaccaagctg 720

gagctgaaa 729

<210> 2
<211> 243
<212> PRT
<213> artificial sequence

<220>
<223> wt Anti-CD3 cassette

<400> 2

Asp Ile Lys Leu Gln Gln Ser Gly Ala Glu Leu Ala Arg Pro Gly Ala

```
1               5                   10                  15
Ser Val Lys Met Ser Cys Lys Thr Ser Gly Tyr Thr Phe Thr Arg Tyr
            20                  25                  30
Thr Met His Trp Val Lys Gln Arg Pro Gly Gln Gly Leu Glu Trp Ile
        35                  40                  45
Gly Tyr Ile Asn Pro Ser Arg Gly Tyr Thr Asn Tyr Asn Gln Lys Phe
    50                  55                  60
Lys Asp Lys Ala Thr Leu Thr Thr Asp Lys Ser Ser Ser Thr Ala Tyr
65                  70                  75                  80
Met Gln Leu Ser Ser Leu Thr Ser Glu Asp Ser Ala Val Tyr Tyr Cys
                85                  90                  95
Ala Arg Tyr Tyr Asp Asp His Tyr Cys Leu Asp Tyr Trp Gly Gln Gly
            100                 105                 110
Thr Thr Leu Thr Val Ser Ser Val Glu Gly Gly Ser Gly Gly Ser Gly
        115                 120                 125
Gly Ser Gly Gly Ser Gly Gly Val Asp Asp Ile Gln Leu Thr Gln Ser
    130                 135                 140
Pro Ala Ile Met Ser Ala Ser Pro Gly Glu Lys Val Thr Met Thr Cys
145                 150                 155                 160
Arg Ala Ser Ser Ser Val Ser Tyr Met Asn Trp Tyr Gln Gln Lys Ser
                165                 170                 175
Gly Thr Ser Pro Lys Arg Trp Ile Tyr Asp Thr Ser Lys Val Ala Ser
            180                 185                 190
Gly Val Pro Tyr Arg Phe Ser Gly Ser Gly Ser Gly Thr Ser Tyr Ser
        195                 200                 205
Leu Thr Ile Ser Ser Met Glu Ala Glu Asp Ala Ala Thr Tyr Tyr Cys
    210                 215                 220
Gln Gln Trp Ser Ser Asn Pro Leu Thr Phe Gly Ala Gly Thr Lys Leu
225                 230                 235                 240
```

Glu Leu Lys

<210> 3
<211> 18
<212> PRT
<213> artificial sequence

<220>
<223> deimmunized linker

<400> 3

Gly Glu Gly Thr Ser Thr Gly Ser Gly Gly Ser Gly Gly Ser Gly Gly
1 5 10 15

Ala Asp

<210> 4
<211> 747
<212> DNA
<213> artificial sequence

<220>
<223> VH1/VL1

<400> 4
tccggaggtg gtggctccga cgtccaactg gtgcagtcag ggctgaagt gaaaaaacct 60

ggggcctcag tgaaggtgtc ctgcaaggct tctggctaca ccgctactag gtacacgatg 120

cactgggtaa ggcaggcacc tggacagggt ctggaatgga ttggatacat taatcctagc 180

cgtggttata ctaattacgc agacagcgtc aagggccgct tcacaatcac tacagacaaa 240

tccaccagca cagcctacat ggaactgagc agcctgcgtt ctgaggacac tgcaacctat 300

tactgtgcaa gatattatga tgatcattac tgccttgact actggggcca agggaccacg 360

gtcaccgtct cctcaggcga aggtactagt actggttctg gtggaagtgg aggttcaggt 420

ggagcagacg acattcagat gacccagtct ccatctagcc tgtctgcatc tgtcggggac 480

cgtgtcacca tcacctgcag agccagtcaa agtgtaagtt acatgaactg gtaccagcag 540

aagccgggca aggcacccaa aagatggatt tatgacacat ccaaagtggc ttctggagtc 600

cctgctcgct tcagtggcag tgggtctggg accgactact ctctcacaat caacagcttg 660

gaggctgaag atgctgccac ttattactgc caacagtgga gtagtaaccc gctcacgttc 720

ggtggcggga ccaaggtgga gatcaaa 747

<210> 5

```
<211>  243
<212>  PRT
<213>  artificial sequence

<220>
<223>  VH1/VL1

<400>  5

Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Ala Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Asp Ser Val
    50                  55                  60

Lys Gly Arg Phe Thr Ile Thr Thr Asp Lys Ser Thr Ser Thr Ala Tyr
65                  70                  75                  80

Met Glu Leu Ser Ser Leu Arg Ser Glu Asp Thr Ala Thr Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Gln Met Thr Gln Ser
    130                 135                 140

Pro Ser Ser Leu Ser Ala Ser Val Gly Asp Arg Val Thr Ile Thr Cys
145                 150                 155                 160

Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205
```

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys

<210> 6
<211> 747
<212> DNA
<213> artificial sequence

<220>
<223> VH1/VL2

<400> 6
tccggaggtg gtggctccga cgtccaactg gtgcagtcag gggctgaagt gaaaaaacct 60

ggggcctcag tgaaggtgtc ctgcaaggct tctggctaca ccgctactag gtacacgatg 120

cactgggtaa ggcaggcacc tggacagggt ctggaatgga ttggatacat taatcctagc 180

cgtggttata ctaattacgc agacagcgtc aagggccgct tcacaatcac tacagacaaa 240

tccaccagca cagcctacat ggaactgagc agcctgcgtt ctgaggacac tgcaacctat 300

tactgtgcaa gatattatga tgatcattac tgccttgact actggggcca agggaccacg 360

gtcaccgtct cctcaggcga aggtactagt actggttctg gtggaagtgg aggttcaggt 420

ggagcagacg acattgtact gacccagtct ccagcaactc tgtctctgtc tccaggggag 480

cgtgccaccc tgagctgcag agccagtcaa agtgtaagtt acatgaactg gtaccagcag 540

aagccgggca aggcacccaa aagatggatt tatgacacat ccaaagtggc ttctggagtc 600

cctgctcgct tcagtggcag tgggtctggg accgactact ctctcacaat caacagcttg 660

gaggctgaag atgctgccac ttattactgc caacagtgga gtagtaaccc gctcacgttc 720

ggtggcggga ccaaggtgga gatcaaa 747

<210> 7
<211> 243
<212> PRT
<213> artificial sequence

<220>
<223> VH1/VL2

<400> 7

```
Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Ala Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Asp Ser Val
    50                  55                  60

Lys Gly Arg Phe Thr Ile Thr Thr Asp Lys Ser Thr Ser Thr Ala Tyr
65                  70                  75                  80

Met Glu Leu Ser Ser Leu Arg Ser Glu Asp Thr Ala Thr Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Val Leu Thr Gln Ser
    130                 135                 140

Pro Ala Thr Leu Ser Leu Ser Pro Gly Glu Arg Ala Thr Leu Ser Cys
145                 150                 155                 160

Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240
```

Glu Ile Lys

<210> 8
<211> 747
<212> DNA
<213> artificial sequence

<220>
<223> VH1/VL3

<400> 8
tccggaggtg gtggctccga cgtccaactg gtgcagtcag ggctgaagt gaaaaaacct 60

ggggcctcag tgaaggtgtc ctgcaaggct tctggctaca ccgctactag gtacacgatg 120

cactgggtaa ggcaggcacc tggacagggt ctggaatgga ttggatacat taatcctagc 180

cgtggttata ctaattacgc agacagcgtc aagggccgct tcacaatcac tacagacaaa 240

tccaccagca cagcctacat ggaactgagc agcctgcgtt ctgaggacac tgcaacctat 300

tactgtgcaa gatattatga tgatcattac tgccttgact actggggcca agggaccacg 360

gtcaccgtct cctcaggcga aggtactagt actggttctg gtggaagtgg aggttcaggt 420

ggagcagacg acattgtact gacccagtct ccagcaactc tgtctctgtc tccaggggag 480

cgtgccaccc tgagctgcag agccagtcaa agtgtaagtt acatgaactg gtaccagcag 540

aagccgggca aggcacccaa aagatggatt tatgacacat ccaaagtggc ttctggagtc 600

cctgctcgct tcagtggcag tgggtctggg accgactact ctctcacaat caacagcttg 660

gaggctgaag atgctgccac ttattactgc caacagtgga gtagtaaccc gctcacgttc 720

ggtggcggga ccaaggtgga gatcaaa 747

<210> 9
<211> 243
<212> PRT
<213> artificial sequence

<220>
<223> VH1/VL3

<400> 9

Asp Val Gln Leu Val Gln Ser Gly Ala Glu Val Lys Lys Pro Gly Ala
1 5 10 15

Ser Val Lys Val Ser Cys Lys Ala Ser Gly Tyr Thr Ala Thr Arg Tyr
20 25 30

```
Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Asp Ser Val
    50                  55                  60

Lys Gly Arg Phe Thr Ile Thr Thr Asp Lys Ser Thr Ser Thr Ala Tyr
65                  70                  75                  80

Met Glu Leu Ser Ser Leu Arg Ser Glu Asp Thr Ala Thr Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Val Leu Thr Gln Ser
    130                 135                 140

Pro Ala Thr Leu Ser Leu Ser Pro Gly Glu Arg Ala Thr Leu Thr Cys
145                 150                 155                 160

Arg Ala Ser Ser Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys
```

<210> 10
<211> 729

<212> DNA
<213> artificial sequence

<220>
<223> VH2/VL1

<400> 10
gacgtccaac tggtgcagtc aggggctgaa gtgaaaaaac ctggggcctc agtgaaggtg 60

tcctgcaagg cttctggcta caccgctact aggtacacga tgcactgggt aaggcaggca 120

cctggacagg gtctggaatg gattggatac attaatccta gccgtggtta tactaattac 180

gcacagaagt tgcagggccg cgtcacaatg actacagaca cttccaccag cacagcctac 240

atggaactga gcagcctgcg ttctgaggac actgcaacct attactgtgc aagatattat 300

gatgatcatt actgccttga ctactggggc caaggcacca cggtcaccgt ctcctcaggc 360

gaaggtacta gtactggttc tggtggaagt ggaggttcag gtggagcaga cgacattcag 420

atgacccagt ctccatctag cctgtctgca tctgtcgggg accgtgtcac catcacctgc 480

agagccagtc aaagtgtaag ttacatgaac tggtaccagc agaagccggg caaggcaccc 540

aaaagatgga tttatgacac atccaaagtg gcttctggag tccctgctcg cttcagtggc 600

agtgggtctg ggaccgacta ctctctcaca atcaacagct tggaggctga agatgctgcc 660

acttattact gccaacagtg gagtagtaac ccgctcacgt tcggtggcgg gaccaaggtg 720

gagatcaaa 729

<210> 11
<211> 243
<212> PRT
<213> artificial sequence

<220>
<223> VH2/VL1

<400> 11

Asp Val Gln Leu Val Gln Ser Gly Ala Glu Val Lys Lys Pro Gly Ala
1 5 10 15

Ser Val Lys Val Ser Cys Lys Ala Ser Gly Tyr Thr Ala Thr Arg Tyr
20 25 30

Thr Met His Trp Val Arg Gln Ala Pro Gly Gln Gly Leu Glu Trp Ile
35 40 45

Gly Tyr Ile Asn Pro Ser Arg Gly Tyr Thr Asn Tyr Ala Gln Lys Leu
50 55 60

```
Gln Gly Arg Val Thr Met Thr Thr Asp Thr Ser Thr Ser Thr Ala Tyr
65                  70                  75                  80

Met Glu Leu Ser Ser Leu Arg Ser Glu Asp Thr Ala Thr Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Gln Met Thr Gln Ser
    130                 135                 140

Pro Ser Ser Leu Ser Ala Ser Val Gly Asp Arg Val Thr Ile Thr Cys
145                 150                 155                 160

Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys


<210>  12
<211>  729
<212>  DNA
<213>  artificial sequence

<220>
<223>  VH2/VL2

<400>  12
gacgtccaac tggtgcagtc aggggctgaa gtgaaaaaac ctggggcctc agtgaaggtg      60
```

```
tcctgcaagg cttctggcta caccgctact aggtacacga tgcactgggt aaggcaggca    120
cctggacagg gtctggaatg gattggatac attaatccta gccgtggtta tactaattac    180
gcacagaagt tgcagggccg cgtcacaatg actacagaca cttccaccag cacagcctac    240
atggaactga gcagcctgcg ttctgaggac actgcaacct attactgtgc aagatattat    300
gatgatcatt actgccttga ctactggggc caaggcacca cggtcaccgt ctcctcaggc    360
gaaggtacta gtactggttc tggtggaagt ggaggttcag gtggagcaga cgacattgta    420
ctgacccagt ctccagcaac tctgtctctg tctccagggg agcgtgccac cctgagctgc    480
agagccagtc aaagtgtaag ttacatgaac tggtaccagc agaagccggg caaggcaccc    540
aaaagatgga tttatgacac atccaaagtg gcttctggag tccctgctcg cttcagtggc    600
agtgggtctg ggaccgacta ctctctcaca atcaacagct tggaggctga agatgctgcc    660
acttattact gccaacagtg gagtagtaac ccgctcacgt tcggtggcgg gaccaaggtg    720
gagatcaaa                                                            729
```

```
<210>  13
<211>  243
<212>  PRT
<213>  artificial sequence

<220>
<223>  VH2/VL2

<400>  13

Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Ala Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Ser Ser Leu Arg Ser Glu Asp Thr Ala Thr Tyr Tyr Cys
                85                  90                  95
```

```
Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Val Leu Thr Gln Ser
    130                 135                 140

Pro Ala Thr Leu Ser Leu Ser Pro Gly Glu Arg Ala Thr Leu Ser Cys
145                 150                 155                 160

Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys


<210>  14
<211>  729
<212>  DNA
<213>  artificial sequence

<220>
<223>  VH2/VL3

<400>  14
gacgtccaac tggtgcagtc aggggctgaa gtgaaaaaac ctggggcctc agtgaaggtg      60

tcctgcaagg cttctggcta caccgctact aggtacacga tgcactgggt aaggcaggca     120

cctggacagg gtctggaatg gattggatac attaatccta gccgtggtta tactaattac     180

gcacagaagt tgcagggccg cgtcacaatg actacagaca cttccaccag cacagcctac     240

atggaactga gcagcctgcg ttctgaggac actgcaacct attactgtgc aagatattat     300
```

```
gatgatcatt actgccttga ctactggggc caaggcacca cggtcaccgt ctcctcaggc    360

gaaggtacta gtactggttc tggtggaagt ggaggttcag gtggagcaga cgacattgta    420

ctgacccagt ctccagcaac tctgtctctg tctccagggg agcgtgccac cctgacctgc    480

agagccagtt caagtgtaag ttacatgaac tggtaccagc agaagccggg caaggcaccc    540

aaaagatgga tttatgacac atccaaagtg gcttctggag tccctgctcg cttcagtggc    600

agtgggtctg ggaccgacta ctctctcaca atcaacagct tggaggctga agatgctgcc    660

acttattact gccaacagtg gagtagtaac ccgctcacgt tcggtggcgg gaccaaggtg    720

gagatcaaa                                                            729


<210>  15
<211>  243
<212>  PRT
<213>  artificial sequence

<220>
<223>  VH2VL3

<400>  15

Asp Val Gln Leu Val Gln Ser Gly Ala Glu Val Lys Lys Pro Gly Ala
1               5                   10                  15


Ser Val Lys Val Ser Cys Lys Ala Ser Gly Tyr Thr Ala Thr Arg Tyr
            20                  25                  30


Thr Met His Trp Val Arg Gln Ala Pro Gly Gln Gly Leu Glu Trp Ile
        35                  40                  45


Gly Tyr Ile Asn Pro Ser Arg Gly Tyr Thr Asn Tyr Ala Gln Lys Leu
    50                  55                  60


Gln Gly Arg Val Thr Met Thr Thr Asp Thr Ser Thr Ser Thr Ala Tyr
65                  70                  75                  80


Met Glu Leu Ser Ser Leu Arg Ser Glu Asp Thr Ala Thr Tyr Tyr Cys
                85                  90                  95


Ala Arg Tyr Tyr Asp Asp His Tyr Cys Leu Asp Tyr Trp Gly Gln Gly
            100                 105                 110


Thr Thr Val Thr Val Ser Ser Gly Glu Gly Thr Ser Thr Gly Ser Gly
        115                 120                 125
```

```
Gly Ser Gly Gly Ser Gly Gly Ala Asp Asp Ile Val Leu Thr Gln Ser
    130                 135                 140

Pro Ala Thr Leu Ser Leu Ser Pro Gly Glu Arg Ala Thr Leu Thr Cys
145                 150                 155                 160

Arg Ala Ser Ser Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys


<210>  16
<211>  729
<212>  DNA
<213>  artificial sequence

<220>
<223>  VH3/VL1

<400>  16
gacgtccaac tggtgcagtc aggggctgaa gtgaaaaaac ctggggcctc agtgaaggtg      60

tcctgcaagg cttctggcta caccgctact aggtacacga tgcactgggt aaggcaggca     120

cctggacagg gtctggaatg gattggatac attaatccta gccgtggtta tactaattac     180

gcacagaagt tgcagggccg cgtcacaatg actacagaca cttccaccag cacagcctac     240

ctgcaaatga acagcctgaa aactgaggac actgcagtct attactgtgc aagatattat     300

gatgatcatt actgccttga ctactggggc caaggcacca cggtcaccgt ctcctcaggc     360

gaaggtacta gtactggttc tggtggaagt ggaggttcag gtggagcaga cgacattcag     420

atgacccagt ctccatctag cctgtctgca tctgtcgggg accgtgtcac catcacctgc     480

agagccagtc aaagtgtaag ttacatgaac tggtaccagc agaagccggg caaggcaccc     540
```

```
aaaagatgga tttatgacac atccaaagtg gcttctggag tccctgctcg cttcagtggc    600

agtgggtctg ggaccgacta ctctctcaca atcaacagct tggaggctga agatgctgcc    660

acttattact gccaacagtg gagtagtaac ccgctcacgt tcggtggcgg gaccaaggtg    720

gagatcaaa                                                            729


<210>  17
<211>  243
<212>  PRT
<213>  artificial sequence

<220>
<223>  VH3/VL1

<400>  17

Asp Val Gln Leu Val Gln Ser Gly Ala Glu Val Lys Lys Pro Gly Ala
1               5                   10                  15


Ser Val Lys Val Ser Cys Lys Ala Ser Gly Tyr Thr Ala Thr Arg Tyr
            20                  25                  30


Thr Met His Trp Val Arg Gln Ala Pro Gly Gln Gly Leu Glu Trp Ile
        35                  40                  45


Gly Tyr Ile Asn Pro Ser Arg Gly Tyr Thr Asn Tyr Ala Gln Lys Leu
    50                  55                  60


Gln Gly Arg Val Thr Met Thr Thr Asp Thr Ser Thr Ser Thr Ala Tyr
65                  70                  75                  80


Leu Gln Met Asn Ser Leu Lys Thr Glu Asp Thr Ala Val Tyr Tyr Cys
                85                  90                  95


Ala Arg Tyr Tyr Asp Asp His Tyr Cys Leu Asp Tyr Trp Gly Gln Gly
            100                 105                 110


Thr Thr Val Thr Val Ser Ser Gly Glu Gly Thr Ser Thr Gly Ser Gly
        115                 120                 125


Gly Ser Gly Gly Ser Gly Gly Ala Asp Asp Ile Gln Met Thr Gln Ser
    130                 135                 140


Pro Ser Ser Leu Ser Ala Ser Val Gly Asp Arg Val Thr Ile Thr Cys
145                 150                 155                 160
```

<210> 19
<211> 243
<212> PRT
<213> artificial sequence

<220>
<223> VH3/VL2

<400> 19

```
Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Ala Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Leu Gln Met Asn Ser Leu Lys Thr Glu Asp Thr Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Val Leu Thr Gln Ser
    130                 135                 140

Pro Ala Thr Leu Ser Leu Ser Pro Gly Glu Arg Ala Thr Leu Ser Cys
145                 150                 155                 160

Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190
```

```
Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys


<210>  18
<211>  729
<212>  DNA
<213>  artificial sequence

<220>
<223>  VH3/VL2

<400>  18
gacgtccaac tggtgcagtc aggggctgaa gtgaaaaaac ctggggcctc agtgaaggtg      60

tcctgcaagg cttctggcta caccgctact aggtacacga tgcactgggt aaggcaggca     120

cctggacagg gtctggaatg gattggatac attaatccta gccgtggtta tactaattac     180

gcacagaagt tgcagggccg cgtcacaatg actacagaca cttccaccag cacagcctac     240

ctgcaaatga acagcctgaa aactgaggac actgcagtct attactgtgc aagatattat     300

gatgatcatt actgccttga ctactggggc caaggcacca cggtcaccgt ctcctcaggc     360

gaaggtacta gtactggttc tggtggaagt ggaggttcag gtggagcaga cgacattgta     420

ctgacccagt ctccagcaac tctgtctctg tctccagggg agcgtgccac cctgagctgc     480

agagccagtc aaagtgtaag ttacatgaac tggtaccagc agaagccggg caaggcaccc     540

aaaagatgga tttatgacac atccaaagtg gcttctggag tccctgctcg cttcagtggc     600

agtgggtctg ggaccgacta ctctctcaca atcaacagct tggaggctga agatgctgcc     660

acttattact gccaacagtg gagtagtaac ccgctcacgt tcggtggcgg gaccaaggtg     720

gagatcaaa                                                              729
```

```
Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys


<210>  20
<211>  729
<212>  DNA
<213>  artificial sequence

<220>
<223>  VH3/VL3

<400>  20
gacgtccaac tggtgcagtc aggggctgaa gtgaaaaaac ctggggcctc agtgaaggtg      60

tcctgcaagg cttctggcta caccgctact aggtacacga tgcactgggt aaggcaggca     120

cctggacagg gtctggaatg gattggatac attaatccta gccgtggtta tactaattac     180

gcacagaagt tgcagggccg cgtcacaatg actacagaca cttccaccag cacagcctac     240

ctgcaaatga acagcctgaa aactgaggac actgcagtct attactgtgc aagatattat     300

gatgatcatt actgccttga ctactggggc caaggcacca cggtcaccgt ctcctcaggc     360

gaaggtacta gtactggttc tggtggaagt ggaggttcag gtggagcaga cgacattgta     420

ctgacccagt ctccagcaac tctgtctctg tctccagggg agcgtgccac cctgacctgc     480

agagccagtt caagtgtaag ttacatgaac tggtaccagc agaagccggg caaggcaccc     540

aaaagatgga tttatgacac atccaaagtg gcttctggag tccctgctcg cttcagtggc     600

agtgggtctg ggaccgacta ctctctcaca atcaacagct tggaggctga agatgctgcc     660

acttattact gccaacagtg gagtagtaac ccgctcacgt tcggtggcgg gaccaaggtg     720

gagatcaaa                                                             729


<210>  21
<211>  243
<212>  PRT
<213>  artificial sequence

<220>
```

```
Gln Gln Trp Ser Ser Asn Pro Leu Thr Phe Gly Gly Gly Thr Lys Val
225                 230                 235                 240

Glu Ile Lys


<210>  22
<211>  747
<212>  DNA
<213>  artificial sequence

<220>
<223>  VH4/VL1

<400>  22
tccggaggtg gtggctccga cgtccaactg gtgcagtcag ggctgaagt gaaaaaacct      60

ggggcctcag tgaaggtgtc ctgcaaggct tctggctaca ccgctactag gtacacgatg    120

cactgggtaa ggcaggcacc tggacagggt ctggaatgga ttggatacat taatcctagc    180

cgtggttata ctaattacgc agacagcgtc aagggccgct tcacaatcac tacagacaaa    240

tccaccagca cagcctacct gcaaatgaac agcctgaaaa ctgaggacac tgcagtctat    300

tactgtgcaa gatattatga tgatcattac tgccttgact actggggcca aggcaccacg    360

gtcaccgtct cctcaggcga aggtactagt actggttctg gtggaagtgg aggttcaggt    420

ggagcagacg acattcagat gacccagtct ccatctagcc tgtctgcatc tgtcggggac    480

cgtgtcacca tcacctgcag agccagtcaa agtgtaagtt acatgaactg gtaccagcag    540

aagccgggca aggcacccaa aagatggatt tatgacacat ccaaagtggc ttctggagtc    600

cctgctcgct tcagtggcag tgggtctggg accgactact ctctcacaat caacagcttg    660

gaggctgaag atgctgccac ttattactgc caacagtgga gtagtaaccc gctcacgttc    720

ggtggcggga ccaaggtgga gatcaaa                                         747


<210>  23
<211>  243
<212>  PRT
<213>  artificial sequence

<220>
<223>  VH4/VL1

<400>  23

Asp Val Gln Leu Val Gln Ser Gly Ala Glu Val Lys Lys Pro Gly Ala
1               5                   10                  15
```

<223> VH3/VL3

<400> 21

```
Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Ala Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Leu Gln Met Asn Ser Leu Lys Thr Glu Asp Thr Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Val Leu Thr Gln Ser
    130                 135                 140

Pro Ala Thr Leu Ser Leu Ser Pro Gly Glu Arg Ala Thr Leu Thr Cys
145                 150                 155                 160

Arg Ala Ser Ser Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220
```

```
Ser Val Lys Val Ser Cys Lys Ala Ser Gly Tyr Thr Ala Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Asp Ser Val
    50                  55                  60

Lys Gly Arg Phe Thr Ile Thr Thr Asp Lys Ser Thr Ser Thr Ala Tyr
65                  70                  75                  80

Leu Gln Met Asn Ser Leu Lys Thr Glu Asp Thr Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Gln Met Thr Gln Ser
    130                 135                 140

Pro Ser Ser Leu Ser Ala Ser Val Gly Asp Arg Val Thr Ile Thr Cys
145                 150                 155                 160

Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys
```

\

<210> 24
<211> 747
<212> DNA
<213> artificial sequence

<220>
<223> VH4/VL2

<400> 24
tccggaggtg gtggctccga cgtccaactg gtgcagtcag ggctgaagt gaaaaaacct 60

ggggcctcag tgaaggtgtc ctgcaaggct tctggctaca ccgctactag gtacacgatg 120

cactgggtaa ggcaggcacc tggacagggt ctggaatgga ttggatacat taatcctagc 180

cgtggttata ctaattacgc agacagcgtc aagggccgct tcacaatcac tacagacaaa 240

tccaccagca cagcctacct gcaaatgaac agcctgaaaa ctgaggacac tgcagtctat 300

tactgtgcaa gatattatga tgatcattac tgccttgact actggggcca aggcaccacg 360

gtcaccgtct cctcaggcga aggtactagt actggttctg gtggaagtgg aggttcaggt 420

ggagcagacg acattgtact gacccagtct ccagcaactc tgtctctgtc tccaggggag 480

cgtgccaccc tgagctgcag agccagtcaa agtgtaagtt acatgaactg gtaccagcag 540

aagccgggca aggcacccaa aagatggatt tatgacacat ccaaagtggc ttctggagtc 600

cctgctcgct tcagtggcag tgggtctggg accgactact ctctcacaat caacagcttg 660

gaggctgaag atgctgccac ttattactgc caacagtgga gtagtaaccc gctcacgttc 720

ggtggcggga ccaaggtgga gatcaaa 747

<210> 25
<211> 243
<212> PRT
<213> artificial sequence

<220>
<223> VH4/VL2

<400> 25

Asp Val Gln Leu Val Gln Ser Gly Ala Glu Val Lys Lys Pro Gly Ala
1 5 10 15

Ser Val Lys Val Ser Cys Lys Ala Ser Gly Tyr Thr Ala Thr Arg Tyr
20 25 30

Thr Met His Trp Val Arg Gln Ala Pro Gly Gln Gly Leu Glu Trp Ile
35 40 45

```
Gly Tyr Ile Asn Pro Ser Arg Gly Tyr Thr Asn Tyr Ala Asp Ser Val
    50                  55                  60

Lys Gly Arg Phe Thr Ile Thr Thr Asp Lys Ser Thr Ser Thr Ala Tyr
65                  70                  75                  80

Leu Gln Met Asn Ser Leu Lys Thr Glu Asp Thr Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Val Leu Thr Gln Ser
    130                 135                 140

Pro Ala Thr Leu Ser Leu Ser Pro Gly Glu Arg Ala Thr Leu Ser Cys
145                 150                 155                 160

Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys


<210>  26
<211>  747
<212>  DNA
<213>  artificial sequence

<220>
<223>  VH4/VL3
```

```
<400>  26
tccggaggtg gtggctccga cgtccaactg gtgcagtcag ggctgaagt gaaaaaacct      60

ggggcctcag tgaaggtgtc ctgcaaggct tctggctaca ccgctactag gtacacgatg     120

cactgggtaa ggcaggcacc tggacagggt ctggaatgga ttggatacat taatcctagc     180

cgtggttata ctaattacgc agacagcgtc aagggccgct tcacaatcac tacagacaaa     240

tccaccagca cagcctacct gcaaatgaac agcctgaaaa ctgaggacac tgcagtctat     300

tactgtgcaa gatattatga tgatcattac tgccttgact actggggcca aggcaccacg     360

gtcaccgtct cctcaggcga aggtactagt actggttctg gtggaagtgg aggttcaggt     420

ggagcagacg acattgtact gacccagtct ccagcaactc tgtctctgtc tccaggggag     480

cgtgccaccc tgacctgcag agccagttca agtgtaagtt acatgaactg gtaccagcag     540

aagccgggca aggcacccaa aagatggatt tatgacacat ccaaagtggc ttctggagtc     600

cctgctcgct tcagtggcag tgggtctggg accgactact ctctcacaat caacagcttg     660

gaggctgaag atgctgccac ttattactgc caacagtgga gtagtaaccc gctcacgttc     720

ggtggcggga ccaaggtgga gatcaaa                                         747


<210>  27
<211>  243
<212>  PRT
<213>  artificial sequence

<220>
<223>  VH4/VL3

<400>  27

Asp Val Gln Leu Val Gln Ser Gly Ala Glu Val Lys Lys Pro Gly Ala
1               5                   10                  15


Ser Val Lys Val Ser Cys Lys Ala Ser Gly Tyr Thr Ala Thr Arg Tyr
            20                  25                  30


Thr Met His Trp Val Arg Gln Ala Pro Gly Gln Gly Leu Glu Trp Ile
        35                  40                  45


Gly Tyr Ile Asn Pro Ser Arg Gly Tyr Thr Asn Tyr Ala Asp Ser Val
    50                  55                  60


Lys Gly Arg Phe Thr Ile Thr Thr Asp Lys Ser Thr Ser Thr Ala Tyr
65                  70                  75                  80
```

```
Leu Gln Met Asn Ser Leu Lys Thr Glu Asp Thr Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Val Leu Thr Gln Ser
    130                 135                 140

Pro Ala Thr Leu Ser Leu Ser Pro Gly Glu Arg Ala Thr Leu Thr Cys
145                 150                 155                 160

Arg Ala Ser Ser Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys
```

<210> 28
<211> 729
<212> DNA
<213> artificial sequence

<220>
<223> VH5/VL1

<400> 28

```
gacgtccaac tggtgcagtc aggggctgaa gtgaaaaaac ctggggcctc agtgaaggtg      60

tcctgcaagg cttctggcta cacctttact aggtacacga tgcactgggt aaggcaggca     120

cctggacagg gtctggaatg gattggatac attaatccta gccgtggtta tactaattac     180
```

gcagacagcg tcaagggccg cttcacaatc actacagaca aatccaccag cacagcctac 240

atggaactga gcagcctgcg ttctgaggac actgcaacct attactgtgc aagatattat 300

gatgatcatt actgccttga ctactggggc caaggcacca cggtcaccgt ctcctcaggc 360

gaaggtacta gtactggttc tggtggaagt ggaggttcag gtggagcaga cgacattcag 420

atgacccagt ctccatctag cctgtctgca tctgtcgggg accgtgtcac catcacctgc 480

agagccagtc aaagtgtaag ttacatgaac tggtaccagc agaagccggg caaggcaccc 540

aaaagatgga tttatgacac atccaaagtg gcttctggag tccctgctcg cttcagtggc 600

agtgggtctg ggaccgacta ctctctcaca atcaacagct tggaggctga agatgctgcc 660

acttattact gccaacagtg gagtagtaac ccgctcacgt tcggtggcgg gaccaaggtg 720

gagatcaaa 729

<210> 29
<211> 243
<212> PRT
<213> artificial sequence

<220>
<223> VH5/VL1

<400> 29

Asp Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Arg Tyr
20 25 30

Thr Met His Trp Val Arg Gln Ala Pro Gly Gln Gly Leu Glu Trp Ile
35 40 45

Gly Tyr Ile Asn Pro Ser Arg Gly Tyr Thr Asn Tyr Ala Asp Ser Val
50 55 60

Lys Gly Arg Phe Thr Ile Thr Thr Asp Lys Ser Thr Ser Thr Ala Tyr
65 70 75 80

Met Glu Leu Ser Ser Leu Arg Ser Glu Asp Thr Ala Thr Tyr Tyr Cys
85 90 95

Ala Arg Tyr Tyr Asp Asp His Tyr Cys Leu Asp Tyr Trp Gly Gln Gly
100 105 110

```
Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Gln Met Thr Gln Ser
    130                 135                 140

Pro Ser Ser Leu Ser Ala Ser Val Gly Asp Arg Val Thr Ile Thr Cys
145                 150                 155                 160

Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys


<210>  30
<211>  729
<212>  DNA
<213>  artificial sequence

<220>
<223>  VH5/VL2

<400>  30
gacgtccaac tggtgcagtc aggggctgaa gtgaaaaaac ctggggcctc agtgaaggtg     60

tcctgcaagg cttctggcta cacctttact aggtacacga tgcactgggt aaggcaggca    120

cctggacagg gtctggaatg gattggatac attaatccta gccgtggtta tactaattac    180

gcagacagcg tcaagggccg cttcacaatc actacagaca aatccaccag cacagcctac    240

atggaactga gcagcctgcg ttctgaggac actgcaacct attactgtgc aagatattat    300

gatgatcatt actgccttga ctactggggc caaggcacca cggtcaccgt ctcctcaggc    360

gaaggtacta gtactggttc tggtggaagt ggaggttcag gtggagcaga cgacattgta    420
```

```
ctgacccagt ctccagcaac tctgtctctg tctccagggg agcgtgccac cctgagctgc    480

agagccagtc aaagtgtaag ttacatgaac tggtaccagc agaagccggg caaggcaccc    540

aaaagatgga tttatgacac atccaaagtg gcttctggag tccctgctcg cttcagtggc    600

agtgggtctg ggaccgacta ctctctcaca atcaacagct tggaggctga agatgctgcc    660

acttattact gccaacagtg gagtagtaac ccgctcacgt tcggtggcgg gaccaaggtg    720

gagatcaaa                                                            729


<210>  31
<211>  243
<212>  PRT
<213>  artificial sequence

<220>
<223>  VH5/VL2

<400>  31

Asp Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Arg Tyr
            20                  25                  30


Thr Met His Trp Val Arg Gln Ala Pro Gly Gln Gly Leu Glu Trp Ile
        35                  40                  45


Gly Tyr Ile Asn Pro Ser Arg Gly Tyr Thr Asn Tyr Ala Asp Ser Val
    50                  55                  60


Lys Gly Arg Phe Thr Ile Thr Thr Asp Lys Ser Thr Ser Thr Ala Tyr
65                  70                  75                  80


Met Glu Leu Ser Ser Leu Arg Ser Glu Asp Thr Ala Thr Tyr Tyr Cys
                85                  90                  95


Ala Arg Tyr Tyr Asp Asp His Tyr Cys Leu Asp Tyr Trp Gly Gln Gly
            100                 105                 110


Thr Thr Val Thr Val Ser Ser Gly Glu Gly Thr Ser Thr Gly Ser Gly
        115                 120                 125


Gly Ser Gly Gly Ser Gly Gly Ala Asp Asp Ile Val Leu Thr Gln Ser
    130                 135                 140
```

```
Pro Ala Thr Leu Ser Leu Ser Pro Gly Glu Arg Ala Thr Leu Ser Cys
145                 150                 155                 160

Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys


<210>  32
<211>  729
<212>  DNA
<213>  artificial sequence

<220>
<223>  VH5/VL3

<400>  32
gacgtccaac tggtgcagtc aggggctgaa gtgaaaaaac ctggggcctc agtgaaggtg      60

tcctgcaagg cttctggcta cacctttact aggtacacga tgcactgggt aaggcaggca     120

cctggacagg gtctggaatg gattggatac attaatccta gccgtggtta tactaattac     180

gcagacagcg tcaagggccg cttcacaatc actacagaca aatccaccag cacagcctac     240

atggaactga gcagcctgcg ttctgaggac actgcaacct attactgtgc aagatattat     300

gatgatcatt actgccttga ctactggggc caaggcacca cggtcaccgt ctcctcaggc     360

gaaggtacta gtactggttc tggtggaagt ggaggttcag gtggagcaga cgacattgta     420

ctgacccagt ctccagcaac tctgtctctg tctccagggg agcgtgccac cctgacctgc     480

agagccagtt caagtgtaag ttacatgaac tggtaccagc agaagccggg caaggcaccc     540

aaaagatgga tttatgacac atccaaagtg gcttctggag tccctgctcg cttcagtggc     600

agtgggtctg ggaccgacta ctctctcaca atcaacagct tggaggctga agatgctgcc     660
```

```
acttattact gccaacagtg gagtagtaac ccgctcacgt tcggtggcgg gaccaaggtg    720

gagatcaaa                                                            729


<210>  33
<211>  243
<212>  PRT
<213>  artificial sequence

<220>
<223>  VH5VH3

<400>  33

Asp Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Thr Arg Tyr
            20                  25                  30


Thr Met His Trp Val Arg Gln Ala Pro Gly Gln Gly Leu Glu Trp Ile
        35                  40                  45


Gly Tyr Ile Asn Pro Ser Arg Gly Tyr Thr Asn Tyr Ala Asp Ser Val
    50                  55                  60


Lys Gly Arg Phe Thr Ile Thr Thr Asp Lys Ser Thr Ser Thr Ala Tyr
65                  70                  75                  80


Met Glu Leu Ser Ser Leu Arg Ser Glu Asp Thr Ala Thr Tyr Tyr Cys
                85                  90                  95


Ala Arg Tyr Tyr Asp Asp His Tyr Cys Leu Asp Tyr Trp Gly Gln Gly
            100                 105                 110


Thr Thr Val Thr Val Ser Ser Gly Glu Gly Thr Ser Thr Gly Ser Gly
        115                 120                 125


Gly Ser Gly Gly Ser Gly Gly Ala Asp Asp Ile Val Leu Thr Gln Ser
    130                 135                 140


Pro Ala Thr Leu Ser Leu Ser Pro Gly Glu Arg Ala Thr Leu Thr Cys
145                 150                 155                 160


Arg Ala Ser Ser Ser Val Ser Tyr Met Asn Trp Tyr Gln Gln Lys Pro
                165                 170                 175
```

Gly Lys Ala Pro Lys Arg Trp Ile Tyr Asp Thr Ser Lys Val Ala Ser
180 185 190

Gly Val Pro Ala Arg Phe Ser Gly Ser Gly Ser Gly Thr Asp Tyr Ser
195 200 205

Leu Thr Ile Asn Ser Leu Glu Ala Glu Asp Ala Ala Thr Tyr Tyr Cys
210 215 220

Gln Gln Trp Ser Ser Asn Pro Leu Thr Phe Gly Gly Gly Thr Lys Val
225 230 235 240

Glu Ile Lys

<210> 34
<211> 747
<212> DNA
<213> artificial sequence

<220>
<223> VH6/VL1

<400> 34
tccggaggtg gtggctccga cgtccaactg gtgcagtcag ggctgaagt gaaaaaacct 60

ggggcctcag tgaaggtgtc ctgcaaggct tctggctaca cctttactag gtacacgatg 120

cactgggtaa ggcaggcacc tggacagggt ctggaatgga ttggatacat taatcctagc 180

cgtggttata ctaattacgc agacagcgtc aagggccgct tcacaatcac tacagacaaa 240

tccaccagca cagcctacat ggaactgagc agcctgcgtt ctgaggacac tgcaacctat 300

tactgtgcaa gatattatga tgatcattac tgccttgact actggggcca aggcaccacg 360

gtcaccgtct cctcaggcga aggtactagt actggttctg gtggaagtgg aggttcaggt 420

ggagcagacg acattcagat gacccagtct ccatctagcc tgtctgcatc tgtcggggac 480

cgtgtcacca tcacctgcag agccagtcaa agtgtaagtt acatgaactg gtaccagcag 540

aagccgggca aggcacccaa aagatggatt tatgacacat ccaaagtggc ttctggagtc 600

cctgctcgct tcagtggcag tgggtctggg accgactact ctctcacaat caacagcttg 660

gaggctgaag atgctgccac ttattactgc caacagtgga gtagtaaccc gctcacgttc 720

ggtggcggga ccaaggtgga gatcaaa 747

<210> 35
<211> 243
<212> PRT

<213> artificial sequence

<220>
<223> VH6/VL1

<400> 35

```
Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Ser Ser Leu Arg Ser Glu Asp Thr Ala Thr Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Gln Met Thr Gln Ser
    130                 135                 140

Pro Ser Ser Leu Ser Ala Ser Val Gly Asp Arg Val Thr Ile Thr Cys
145                 150                 155                 160

Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205
```

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys

<210> 36
<211> 747
<212> DNA
<213> artificial sequence

<220>
<223> VH6/VL2

<400> 36
tccggaggtg gtggctccga cgtccaactg gtgcagtcag ggctgaagt gaaaaaacct 60

ggggcctcag tgaaggtgtc ctgcaaggct tctggctaca cctttactag gtacacgatg 120

cactgggtaa ggcaggcacc tggacagggt ctggaatgga ttggatacat taatcctagc 180

cgtggttata ctaattacgc acagaagttg cagggccgcg tcacaatgac tacagacact 240

tccaccagca cagcctacat ggaactgagc agcctgcgtt ctgaggacac tgcaacctat 300

tactgtgcaa gatattatga tgatcattac tgccttgact actggggcca aggcaccacg 360

gtcaccgtct cctcaggcga aggtactagt actggttctg gtggaagtgg aggttcaggt 420

ggagcagacg acattgtact gacccagtct ccagcaactc tgtctctgtc tccaggggag 480

cgtgccaccc tgagctgcag agccagtcaa agtgtaagtt acatgaactg gtaccagcag 540

aagccgggca aggcacccaa aagatggatt tatgacacat ccaaagtggc ttctggagtc 600

cctgctcgct tcagtggcag tgggtctggg accgactact ctctcacaat caacagcttg 660

gaggctgaag atgctgccac ttattactgc caacagtgga gtagtaaccc gctcacgttc 720

ggtggcggga ccaaggtgga gatcaaa 747

<210> 37
<211> 243
<212> PRT
<213> artificial sequence

<220>
<223> VH6/VL2

<400> 37

Asp Val Gln Leu Val Gln Ser Gly Ala Glu Val Lys Lys Pro Gly Ala

```
1               5                   10                  15
Ser Val Lys Val Ser Cys Lys Ala Ser Gly Tyr Thr Phe Thr Arg Tyr
            20                  25                  30
Thr Met His Trp Val Arg Gln Ala Pro Gly Gln Gly Leu Glu Trp Ile
        35                  40                  45
Gly Tyr Ile Asn Pro Ser Arg Gly Tyr Thr Asn Tyr Ala Gln Lys Leu
    50                  55                  60
Gln Gly Arg Val Thr Met Thr Thr Asp Thr Ser Thr Ser Thr Ala Tyr
65                  70                  75                  80
Met Glu Leu Ser Ser Leu Arg Ser Glu Asp Thr Ala Thr Tyr Tyr Cys
                85                  90                  95
Ala Arg Tyr Tyr Asp Asp His Tyr Cys Leu Asp Tyr Trp Gly Gln Gly
            100                 105                 110
Thr Thr Val Thr Val Ser Ser Gly Glu Gly Thr Ser Thr Gly Ser Gly
        115                 120                 125
Gly Ser Gly Gly Ser Gly Gly Ala Asp Asp Ile Val Leu Thr Gln Ser
    130                 135                 140
Pro Ala Thr Leu Ser Leu Ser Pro Gly Glu Arg Ala Thr Leu Ser Cys
145                 150                 155                 160
Arg Ala Ser Gln Ser Val Ser Tyr Met Asn Trp Tyr Gln Gln Lys Pro
                165                 170                 175
Gly Lys Ala Pro Lys Arg Trp Ile Tyr Asp Thr Ser Lys Val Ala Ser
            180                 185                 190
Gly Val Pro Ala Arg Phe Ser Gly Ser Gly Ser Gly Thr Asp Tyr Ser
        195                 200                 205
Leu Thr Ile Asn Ser Leu Glu Ala Glu Asp Ala Ala Thr Tyr Tyr Cys
    210                 215                 220
Gln Gln Trp Ser Ser Asn Pro Leu Thr Phe Gly Gly Gly Thr Lys Val
225                 230                 235                 240
```

Glu Ile Lys

<210> 38
<211> 747
<212> DNA
<213> artificial sequence

<220>
<223> VH6/VL3

<400> 38
tccggaggtg gtggctccga cgtccaactg gtgcagtcag ggctgaagt gaaaaaacct 60

ggggcctcag tgaaggtgtc ctgcaaggct tctggctaca cctttactag gtacacgatg 120

cactgggtaa ggcaggcacc tggacagggt ctggaatgga ttggatacat taatcctagc 180

cgtggttata ctaattacgc acagaagttg cagggccgcg tcacaatgac tacagacact 240

tccaccagca cagcctacat ggaactgagc agcctgcgtt ctgaggacac tgcaacctat 300

tactgtgcaa gatattatga tgatcattac tgccttgact actggggcca aggcaccacg 360

gtcaccgtct cctcaggcga aggtactagt actggttctg gtggaagtgg aggttcaggt 420

ggagcagacg acattgtact gacccagtct ccagcaactc tgtctctgtc tccaggggag 480

cgtgccaccc tgacctgcag agccagttca agtgtaagtt acatgaactg gtaccagcag 540

aagccgggca aggcacccaa aagatggatt tatgacacat ccaaagtggc ttctggagtc 600

cctgctcgct tcagtggcag tgggtctggg accgactact ctctcacaat caacagcttg 660

gaggctgaag atgctgccac ttattactgc caacagtgga gtagtaaccc gctcacgttc 720

ggtggcggga ccaaggtgga gatcaaa 747

<210> 39
<211> 243
<212> PRT
<213> artificial sequence

<220>
<223> VH6/VL3

<400> 39

Asp Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Arg Tyr
20 25 30

Thr Met His Trp Val Arg Gln Ala Pro Gly Gln Gly Leu Glu Trp Ile

```
        35                  40                  45

Gly Tyr Ile Asn Pro Ser Arg Gly Tyr Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Ser Ser Leu Arg Ser Glu Asp Thr Ala Thr Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Val Leu Thr Gln Ser
    130                 135                 140

Pro Ala Thr Leu Ser Leu Ser Pro Gly Glu Arg Ala Thr Leu Thr Cys
145                 150                 155                 160

Arg Ala Ser Ser Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys
```

<210>  40
<211>  729
<212>  DNA
<213>  artificial sequence

<220>
<223> VH7/VL1

<400> 40
gacgtccaac tggtgcagtc aggggctgaa gtgaaaaaac ctggggcctc agtgaaggtg 60

tcctgcaagg cttctggcta cacctttact aggtacacga tgcactgggt aaggcaggca 120

cctggacagg gtctggaatg gattggatac attaatccta gccgtggtta tactaattac 180

aatcagaagt tcaaggaccg cgtcacaatc actacagaca aatccaccag cacagcctac 240

atggaactga gcagcctgcg ttctgaggac actgcagtct attactgtgc aagatattat 300

gatgatcatt actgccttga ctactggggc caaggcacca cggtcaccgt ctcctcaggc 360

gaaggtacta gtactggttc tggtggaagt ggaggttcag gtggagcaga cgacattcag 420

atgacccagt ctccatctag cctgtctgca tctgtcgggg accgtgtcac catcacctgc 480

agagccagtc aaagtgtaag ttacatgaac tggtaccagc agaagccggg caaggcaccc 540

aaaagatgga tttatgacac atccaaagtg gcttctggag tccctgctcg cttcagtggc 600

agtgggtctg ggaccgacta ctctctcaca atcaacagct tggaggctga agatgctgcc 660

acttattact gccaacagtg gagtagtaac ccgctcacgt tcggtggcgg gaccaaggtg 720

gagatcaaa 729

<210> 41
<211> 243
<212> PRT
<213> artificial sequence

<220>
<223> VH7/VL1

<400> 41

Asp Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Arg Tyr
20 25 30

Thr Met His Trp Val Arg Gln Ala Pro Gly Gln Gly Leu Glu Trp Ile
35 40 45

Gly Tyr Ile Asn Pro Ser Arg Gly Tyr Thr Asn Tyr Asn Gln Lys Phe
50 55 60

Lys Asp Arg Val Thr Ile Thr Thr Asp Lys Ser Thr Ser Thr Ala Tyr

65 70 75 80

Met Glu Leu Ser Ser Leu Arg Ser Glu Asp Thr Ala Val Tyr Tyr Cys
85 90 95

Ala Arg Tyr Tyr Asp Asp His Tyr Cys Leu Asp Tyr Trp Gly Gln Gly
100 105 110

Thr Thr Val Thr Val Ser Ser Gly Glu Gly Thr Ser Thr Gly Ser Gly
115 120 125

Gly Ser Gly Gly Ser Gly Gly Ala Asp Asp Ile Gln Met Thr Gln Ser
130 135 140

Pro Ser Ser Leu Ser Ala Ser Val Gly Asp Arg Val Thr Ile Thr Cys
145 150 155 160

Arg Ala Ser Gln Ser Val Ser Tyr Met Asn Trp Tyr Gln Gln Lys Pro
165 170 175

Gly Lys Ala Pro Lys Arg Trp Ile Tyr Asp Thr Ser Lys Val Ala Ser
180 185 190

Gly Val Pro Ala Arg Phe Ser Gly Ser Gly Ser Gly Thr Asp Tyr Ser
195 200 205

Leu Thr Ile Asn Ser Leu Glu Ala Glu Asp Ala Ala Thr Tyr Tyr Cys
210 215 220

Gln Gln Trp Ser Ser Asn Pro Leu Thr Phe Gly Gly Gly Thr Lys Val
225 230 235 240

Glu Ile Lys

<210> 42
<211> 729
<212> DNA
<213> artificial sequence

<220>
<223> VH7/VL2

<400> 42
gacgtccaac tggtgcagtc aggggctgaa gtgaaaaaac ctggggcctc agtgaaggtg 60

tcctgcaagg cttctggcta cacctttact aggtacacga tgcactgggt aaggcaggca 120

```
cctggacagg gtctggaatg gattggatac attaatccta gccgtggtta tactaattac     180
aatcagaagt tcaaggaccg cgtcacaatc actacagaca aatccaccag cacagcctac     240
atggaactga gcagcctgcg ttctgaggac actgcagtct attactgtgc aagatattat     300
gatgatcatt actgccttga ctactggggc caaggcacca cggtcaccgt ctcctcaggc     360
gaaggtacta gtactggttc tggtggaagt ggaggttcag gtggagcaga cgacattgta     420
ctgacccagt ctccagcaac tctgtctctg tctccagggg agcgtgccac cctgagctgc     480
agagccagtc aaagtgtaag ttacatgaac tggtaccagc agaagccggg caaggcaccc     540
aaaagatgga tttatgacac atccaaagtg gcttctggag tccctgctcg cttcagtggc     600
agtgggtctg ggaccgacta ctctctcaca atcaacagct tggaggctga agatgctgcc     660
acttattact gccaacagtg gagtagtaac ccgctcacgt tcggtggcgg gaccaaggtg     720
gagatcaaa                                                             729
```

```
<210>  43
<211>  243
<212>  PRT
<213>  artificial sequence

<220>
<223>  VH7/VL2

<400>  43

Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Arg Val Thr Ile Thr Thr Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Tyr Tyr Asp Asp His Tyr Cys Leu Asp Tyr Trp Gly Gln Gly
```

```
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Val Leu Thr Gln Ser
    130                 135                 140

Pro Ala Thr Leu Ser Leu Ser Pro Gly Glu Arg Ala Thr Leu Ser Cys
145                 150                 155                 160

Arg Ala Ser Gln Ser Val Ser Tyr Met Asn Trp Tyr Gln Gln Lys Pro
                165                 170                 175

Gly Lys Ala Pro Lys Arg Trp Ile Tyr Asp Thr Ser Lys Val Ala Ser
            180                 185                 190

Gly Val Pro Ala Arg Phe Ser Gly Ser Gly Ser Gly Thr Asp Tyr Ser
        195                 200                 205

Leu Thr Ile Asn Ser Leu Glu Ala Glu Asp Ala Ala Thr Tyr Tyr Cys
    210                 215                 220

Gln Gln Trp Ser Ser Asn Pro Leu Thr Phe Gly Gly Gly Thr Lys Val
225                 230                 235                 240

Glu Ile Lys


<210>  44
<211>  729
<212>  DNA
<213>  artificial sequence

<220>
<223>  VH7/VL3

<400>  44
gacgtccaac tggtgcagtc aggggctgaa gtgaaaaaac ctggggcctc agtgaaggtg      60

tcctgcaagg cttctggcta cacctttact aggtacacga tgcactgggt aaggcaggca     120

cctggacagg gtctggaatg gattggatac attaatccta gccgtggtta tactaattac     180

aatcagaagt tcaaggaccg cgtcacaatc actacagaca aatccaccag cacagcctac     240

atggaactga gcagcctgcg ttctgaggac actgcagtct attactgtgc aagatattat     300

gatgatcatt actgccttga ctactggggc caaggcacca cggtcaccgt ctcctcaggc     360
```

```
gaaggtacta gtactggttc tggtggaagt ggaggttcag gtggagcaga cgacattgta    420
ctgacccagt ctccagcaac tctgtctctg tctccagggg agcgtgccac cctgacctgc    480
agagccagtt caagtgtaag ttacatgaac tggtaccagc agaagccggg caaggcaccc    540
aaaagatgga tttatgacac atccaaagtg gcttctggag tccctgctcg cttcagtggc    600
agtgggtctg ggaccgacta ctctctcaca atcaacagct tggaggctga agatgctgcc    660
acttattact gccaacagtg gagtagtaac ccgctcacgt tcggtggcgg gaccaaggtg    720
gagatcaaa                                                            729
```

```
<210>  45
<211>  243
<212>  PRT
<213>  artificial sequence

<220>
<223>  VH7/VL3

<400>  45

Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Arg Val Thr Ile Thr Thr Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser Gly Glu Gly Thr Ser Thr Gly Ser Gly
        115                 120                 125

Gly Ser Gly Gly Ser Gly Gly Ala Asp Asp Ile Val Leu Thr Gln Ser
```

130 135 140

Pro Ala Thr Leu Ser Leu Ser Pro Gly Glu Arg Ala Thr Leu Thr Cys
145 150 155 160

Arg Ala Ser Ser Ser Val Ser Tyr Met Asn Trp Tyr Gln Gln Lys Pro
165 170 175

Gly Lys Ala Pro Lys Arg Trp Ile Tyr Asp Thr Ser Lys Val Ala Ser
180 185 190

Gly Val Pro Ala Arg Phe Ser Gly Ser Gly Ser Gly Thr Asp Tyr Ser
195 200 205

Leu Thr Ile Asn Ser Leu Glu Ala Glu Asp Ala Ala Thr Tyr Tyr Cys
210 215 220

Gln Gln Trp Ser Ser Asn Pro Leu Thr Phe Gly Gly Gly Thr Lys Val
225 230 235 240

Glu Ile Lys

<210> 46
<211> 20
<212> DNA
<213> artificial sequence

<220>
<223> Sequencing primer

<400> 46
cctcagacag tggttcaaag 20

<210> 47
<211> 18
<212> DNA
<213> artificial sequence

<220>
<223> Sequencing primer

<400> 47
agccgccacg tgggcctc 18

<210> 48
<211> 18
<212> PRT
<213> artificial sequence

<220>
<223>  non-deimmunized linker sequence

<400>  48

Val Glu Gly Gly Ser Gly Gly Ser Gly Gly Ser Gly Gly Ser Gly Gly
1               5                   10                  15

Val Asp

<210>  49
<211>  357
<212>  DNA
<213>  artificial sequence

<220>
<223>  anti-CD3 VH1

<400>  49
gacgtccaac tggtgcagtc aggggctgaa gtgaaaaaac ctggggcctc agtgaaggtg     60

tcctgcaagg cttctggcta caccgctact aggtacacga tgcactgggt aaggcaggca    120

cctggacagg gtctggaatg gattggatac attaatccta gccgtggtta tactaattac    180

gcagacagcg tcaagggccg cttcacaatc actacagaca aatccaccag cacagcctac    240

atggaactga gcagcctgcg ttctgaggac actgcaacct attactgtgc aagatattat    300

gatgatcatt actgccttga ctactggggc caagggacca cggtcaccgt ctcctca       357

<210>  50
<211>  119
<212>  PRT
<213>  artificial sequence

<220>
<223>  anti-CD3 VH1

<400>  50

Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Ala Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Asp Ser Val

50 55 60

Lys Gly Arg Phe Thr Ile Thr Thr Asp Lys Ser Thr Ser Thr Ala Tyr
65 70 75 80

Met Glu Leu Ser Ser Leu Arg Ser Glu Asp Thr Ala Thr Tyr Tyr Cys
85 90 95

Ala Arg Tyr Tyr Asp Asp His Tyr Cys Leu Asp Tyr Trp Gly Gln Gly
100 105 110

Thr Thr Val Thr Val Ser Ser
115

<210> 51
<211> 357
<212> DNA
<213> artificial sequence

<220>
<223> anti-CD3 VH2

<400> 51
gacgtccaac tggtgcagtc aggggctgaa gtgaaaaaac ctggggcctc agtgaaggtg 60

tcctgcaagg cttctggcta caccgctact aggtacacga tgcactgggt aaggcaggca 120

cctggacagg gtctggaatg gattggatac attaatccta gccgtggtta tactaattac 180

gcacagaagt tgcagggccg cgtcacaatg actacagaca cttccaccag cacagcctac 240

atggaactga gcagcctgcg ttctgaggac actgcaacct attactgtgc aagatattat 300

gatgatcatt actgccttga ctactggggc caaggcacca cggtcaccgt ctcctca 357

<210> 52
<211> 119
<212> PRT
<213> artificial sequence

<220>
<223> anti-CD3 VH2

<400> 52

Asp Val Gln Leu Val Gln Ser Gly Ala Glu Val Lys Lys Pro Gly Ala
1 5 10 15

Ser Val Lys Val Ser Cys Lys Ala Ser Gly Tyr Thr Ala Thr Arg Tyr
20 25 30

```
Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Ser Ser Leu Arg Ser Glu Asp Thr Ala Thr Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser
        115


<210>  53
<211>  357
<212>  DNA
<213>  artificial sequence

<220>
<223>  anti-CD3 VH3

<400>  53
gacgtccaac tggtgcagtc aggggctgaa gtgaaaaaac ctggggcctc agtgaaggtg      60

tcctgcaagg cttctggcta caccgctact aggtacacga tgcactgggt aaggcaggca     120

cctggacagg gtctggaatg gattggatac attaatccta gccgtggtta tactaattac     180

gcacagaagt tgcagggccg cgtcacaatg actacagaca cttccaccag cacagcctac     240

ctgcaaatga acagcctgaa aactgaggac actgcagtct attactgtgc aagatattat     300

gatgatcatt actgccttga ctactggggc caaggcacca cggtcaccgt ctcctca        357


<210>  54
<211>  119
<212>  PRT
<213>  artificial sequence

<220>
<223>  anti-CD3 VH3

<400>  54

Asp Val Gln Leu Val Gln Ser Gly Ala Glu Val Lys Lys Pro Gly Ala
1               5                   10                  15
```

```
Ser Val Lys Val Ser Cys Lys Ala Ser Gly Tyr Thr Ala Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Leu Gln Met Asn Ser Leu Lys Thr Glu Asp Thr Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser
        115
```

```
<210>  55
<211>  357
<212>  DNA
<213>  artificial sequence

<220>
<223>  anti-CD3 VH4

<400>  55
gacgtccaac tggtgcagtc aggggctgaa gtgaaaaaac ctggggcctc agtgaaggtg      60

tcctgcaagg cttctggcta caccgctact aggtacacga tgcactgggt aaggcaggca     120

cctggacagg gtctggaatg gattggatac attaatccta gccgtggtta tactaattac     180

gcagacagcg tcaagggccg cttcacaatc actacagaca aatccaccag cacagcctac     240

ctgcaaatga acagcctgaa aactgaggac actgcagtct attactgtgc aagatattat     300

gatgatcatt actgccttga ctactggggc caaggcacca cggtcaccgt ctcctca        357
```

```
<210>  56
<211>  119
<212>  PRT
<213>  artificial sequence

<220>
<223>  anti-CD3 VH4
```

<400> 56

```
Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Ala Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Asp Ser Val
    50                  55                  60

Lys Gly Arg Phe Thr Ile Thr Thr Asp Lys Ser Thr Ser Thr Ala Tyr
65                  70                  75                  80

Leu Gln Met Asn Ser Leu Lys Thr Glu Asp Thr Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser
        115
```

<210> 57
<211> 357
<212> DNA
<213> artificial sequence

<220>
<223> anti-CD3 VH5

<400> 57

```
gacgtccaac tggtgcagtc aggggctgaa gtgaaaaaac ctggggcctc agtgaaggtg     60

tcctgcaagg cttctggcta cacctttact aggtacacga tgcactgggt aaggcaggca    120

cctggacagg gtctggaatg gattggatac attaatccta gccgtggtta tactaattac    180

gcagacagcg tcaagggccg cttcacaatc actacagaca aatccaccag cacagcctac    240

atggaactga gcagcctgcg ttctgaggac actgcaacct attactgtgc aagatattat    300

gatgatcatt actgccttga ctactggggc caaggcacca cggtcaccgt ctcctca       357
```

<210> 58
<211> 119
<212> PRT

<213> artificial sequence

<220>
<223> anti-CD3 VH5

<400> 58

Asp Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Arg Tyr
20 25 30

Thr Met His Trp Val Arg Gln Ala Pro Gly Gln Gly Leu Glu Trp Ile
35 40 45

Gly Tyr Ile Asn Pro Ser Arg Gly Tyr Thr Asn Tyr Ala Asp Ser Val
50 55 60

Lys Gly Arg Phe Thr Ile Thr Thr Asp Lys Ser Thr Ser Thr Ala Tyr
65 70 75 80

Met Glu Leu Ser Ser Leu Arg Ser Glu Asp Thr Ala Thr Tyr Tyr Cys
85 90 95

Ala Arg Tyr Tyr Asp Asp His Tyr Cys Leu Asp Tyr Trp Gly Gln Gly
100 105 110

Thr Thr Val Thr Val Ser Ser
115

<210> 59
<211> 357
<212> DNA
<213> artificial sequence

<220>
<223> anti-CD3 VH6

<400> 59
gacgtccaac tggtgcagtc aggggctgaa gtgaaaaaac ctggggcctc agtgaaggtg 60

tcctgcaagg cttctggcta cacctttact aggtacacga tgcactgggt aaggcaggca 120

cctggacagg gtctggaatg gattggatac attaatccta gccgtggtta tactaattac 180

gcacagaagt tgcagggccg cgtcacaatg actacagaca cttccaccag cacagcctac 240

atggaactga gcagcctgcg ttctgaggac actgcaacct attactgtgc aagatattat 300

gatgatcatt actgccttga ctactggggc caaggcacca cggtcaccgt ctcctca 357

<210> 60
<211> 119
<212> PRT
<213> artificial sequence

<220>
<223> anti-CD3 VH6

<400> 60

```
Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Ser Ser Leu Arg Ser Glu Asp Thr Ala Thr Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser
        115
```

<210> 61
<211> 357
<212> DNA
<213> artificial sequence

<220>
<223> anti-CD3 VH7

<400> 61

```
gacgtccaac tggtgcagtc aggggctgaa gtgaaaaaac ctggggcctc agtgaaggtg     60

tcctgcaagg cttctggcta cacctttact aggtacacga tgcactgggt aaggcaggca    120

cctggacagg gtctggaatg gattggatac attaatccta gccgtggtta tactaattac    180
```

aatcagaagt tcaaggaccg cgtcacaatc actacagaca aatccaccag cacagcctac 240

atggaactga gcagcctgcg ttctgaggac actgcagtct attactgtgc aagatattat 300

gatgatcatt actgccttga ctactggggc caaggcacca cggtcaccgt ctcctca 357

<210> 62
<211> 119
<212> PRT
<213> artificial sequence

<220>
<223> anti-CD3 VH7

<400> 62

```
Asp Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Arg Tyr
            20                  25                  30

Thr Met His Trp Val Arg Gln Ala Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Arg Val Thr Ile Thr Thr Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Val Thr Val Ser Ser
        115
```

<210> 63
<211> 318
<212> DNA
<213> artificial sequence

<220>
<223> anti-CD3 VL1

<400> 63
gacattcaga tgacccagtc tccatctagc ctgtctgcat ctgtcgggga ccgtgtcacc 60

```
atcacctgca gagccagtca aagtgtaagt tacatgaact ggtaccagca gaagccgggc    120

aaggcaccca aaagatggat ttatgacaca tccaaagtgg cttctggagt ccctgctcgc    180

ttcagtggca gtgggtctgg gaccgactac tctctcacaa tcaacagctt ggaggctgaa    240

gatgctgcca cttattactg ccaacagtgg agtagtaacc cgctcacgtt cggtggcggg    300

accaaggtgg agatcaaa                                                  318


<210>  64
<211>  106
<212>  PRT
<213>  artificial sequence

<220>
<223>  anti-CD3 VL1

<400>  64

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Val Ser Tyr Met
            20                  25                  30


Asn Trp Tyr Gln Gln Lys Pro Gly Lys Ala Pro Lys Arg Trp Ile Tyr
        35                  40                  45


Asp Thr Ser Lys Val Ala Ser Gly Val Pro Ala Arg Phe Ser Gly Ser
    50                  55                  60


Gly Ser Gly Thr Asp Tyr Ser Leu Thr Ile Asn Ser Leu Glu Ala Glu
65                  70                  75                  80


Asp Ala Ala Thr Tyr Tyr Cys Gln Gln Trp Ser Ser Asn Pro Leu Thr
                85                  90                  95


Phe Gly Gly Gly Thr Lys Val Glu Ile Lys
            100                 105


<210>  65
<211>  318
<212>  DNA
<213>  artificial sequence

<220>
<223>  anti-CD3 VL2

<400>  65
```

```
gacattgtac tgacccagtc tccagcaact ctgtctctgt ctccagggga gcgtgccacc     60

ctgagctgca gagccagtca aagtgtaagt tacatgaact ggtaccagca gaagccgggc    120

aaggcaccca aaagatggat ttatgacaca tccaaagtgg cttctggagt ccctgctcgc    180

ttcagtggca gtgggtctgg gaccgactac tctctcacaa tcaacagctt ggaggctgaa    240

gatgctgcca cttattactg ccaacagtgg agtagtaacc cgctcacgtt cggtggcggg    300

accaaggtgg agatcaaa                                                  318
```

```
<210>  66
<211>  106
<212>  PRT
<213>  artificial sequence

<220>
<223>  anti-CD3 VL2

<400>  66

Asp Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Tyr Met
            20                  25                  30

Asn Trp Tyr Gln Gln Lys Pro Gly Lys Ala Pro Lys Arg Trp Ile Tyr
        35                  40                  45

Asp Thr Ser Lys Val Ala Ser Gly Val Pro Ala Arg Phe Ser Gly Ser
    50                  55                  60

Gly Ser Gly Thr Asp Tyr Ser Leu Thr Ile Asn Ser Leu Glu Ala Glu
65                  70                  75                  80

Asp Ala Ala Thr Tyr Tyr Cys Gln Gln Trp Ser Ser Asn Pro Leu Thr
                85                  90                  95

Phe Gly Gly Gly Thr Lys Val Glu Ile Lys
            100                 105


<210>  67
<211>  318
<212>  DNA
<213>  artificial sequence

<220>
<223>  anti-CD3 VL3
```

<400> 67
gacattgtac tgacccagtc tccagcaact ctgtctctgt ctccagggga gcgtgccacc 60

ctgacctgca gagccagttc aagtgtaagt tacatgaact ggtaccagca gaagccgggc 120

aaggcaccca aaagatggat ttatgacaca tccaaagtgg cttctggagt ccctgctcgc 180

ttcagtggca gtgggtctgg gaccgactac tctctcacaa tcaacagctt ggaggctgaa 240

gatgctgcca cttattactg ccaacagtgg agtagtaacc cgctcacgtt cggtggcggg 300

accaaggtgg agatcaaa 318

<210> 68
<211> 106
<212> PRT
<213> artificial sequence

<220>
<223> anti-CD3 VL3

<400> 68

Asp Ile Val Leu Thr Gln Ser Pro Ala Thr Leu Ser Leu Ser Pro Gly
1               5                   10                  15

Glu Arg Ala Thr Leu Thr Cys Arg Ala Ser Ser Ser Val Ser Tyr Met
            20                  25                  30

Asn Trp Tyr Gln Gln Lys Pro Gly Lys Ala Pro Lys Arg Trp Ile Tyr
        35                  40                  45

Asp Thr Ser Lys Val Ala Ser Gly Val Pro Ala Arg Phe Ser Gly Ser
    50                  55                  60

Gly Ser Gly Thr Asp Tyr Ser Leu Thr Ile Asn Ser Leu Glu Ala Glu
65                  70                  75                  80

Asp Ala Ala Thr Tyr Tyr Cys Gln Gln Trp Ser Ser Asn Pro Leu Thr
                85                  90                  95

Phe Gly Gly Gly Thr Lys Val Glu Ile Lys
            100                 105

<210> 69
<211> 357
<212> DNA
<213> artificial sequence

<220>
<223> wild type anti-CD3 VH

<400> 69
gatatcaaac tgcagcagtc aggggctgaa ctggcaagac ctggggcctc agtgaagatg 60

tcctgcaaga cttctggcta cacctttact aggtacacga tgcactgggt aaaacagagg 120

cctggacagg gtctggaatg gattggatac attaatccta gccgtggtta tactaattac 180

aatcagaagt tcaaggacaa ggccacattg actacagaca aatcctccag cacagcctac 240

atgcaactga gcagcctgac atctgaggac tctgcagtct attactgtgc aagatattat 300

gatgatcatt actgccttga ctactggggc caaggcacca ctctcacagt ctcctca 357

<210> 70
<211> 119
<212> PRT
<213> artificial sequence

<220>
<223> wild type anti-CD3 VH

<400> 70

Asp Ile Lys Leu Gln Gln Ser Gly Ala Glu Leu Ala Arg Pro Gly Ala
1               5                   10                  15

Ser Val Lys Met Ser Cys Lys Thr Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Leu Thr Val Ser Ser
        115

<210> 71
<211> 318

```
<212>  DNA
<213>  artificial sequence

<220>
<223>  wild type anti-CD3 VK

<400>  71
gacattcagc tgacccagtc tccagcaatc atgtctgcat ctccagggga gaaggtcacc      60

atgacctgca gagccagttc aagtgtaagt tacatgaact ggtaccagca gaagtcaggc     120

acctccccca aaagatggat ttatgacaca tccaaagtgg cttctggagt cccttatcgc     180

ttcagtggca gtgggtctgg gacctcatac tctctcacaa tcagcagcat ggaggctgaa     240

gatgctgcca cttattactg ccaacagtgg agtagtaacc cgctcacgtt cggtgctggg     300

accaagctgg agctgaaa                                                   318


<210>  72
<211>  106
<212>  PRT
<213>  artificial sequence

<220>
<223>  wild type anti-CD3 VK

<400>  72

Asp Ile Gln Leu Thr Gln Ser Pro Ala Ile Met Ser Ala Ser Pro Gly
1               5                   10                  15


Glu Lys Val Thr Met Thr Cys Arg Ala Ser Ser Ser Val Ser Tyr Met
            20                  25                  30


Asn Trp Tyr Gln Gln Lys Ser Gly Thr Ser Pro Lys Arg Trp Ile Tyr
        35                  40                  45


Asp Thr Ser Lys Val Ala Ser Gly Val Pro Tyr Arg Phe Ser Gly Ser
    50                  55                  60


Gly Ser Gly Thr Ser Tyr Ser Leu Thr Ile Ser Ser Met Glu Ala Glu
65                  70                  75                  80


Asp Ala Ala Thr Tyr Tyr Cys Gln Gln Trp Ser Ser Asn Pro Leu Thr
                85                  90                  95


Phe Gly Ala Gly Thr Lys Leu Glu Leu Lys
            100                 105


<210>  73
```

```
<211>  54
<212>  DNA
<213>  artificial sequence

<220>
<223>  deimmunized linker

<400>  73
ggcgaaggta ctagtactgg ttctggtgga agtggaggtt caggtggagc agac          54
```

## Description

The present invention relates to CD3 specific binding molecules and nucleic acid sequences encoding said CD3 specific binding molecules. Further aspects of the invention are vectors and host cells comprising said nucleic acid sequence, a process for the production of the binding molecules of the invention and compositions comprising said binding molecules. The invention also provides the use of said binding molecules for the preparation of pharmaceutical compositions for the treatment of particular diseases and a kit comprising the binding molecules of the invention.

Human CD3 denotes an antigen which is expressed on T cells as part of the multimolecular T cell complex and which consists of three different chains: CD3-ε, CD3-δ and CD3-γ. Clustering of CD3 on T cells, e.g, by immobilized anti-CD3 antibodies leads to T cell activation similar to the engagement of the T cell receptor but independent of its clone-typical specificity; see WO 99/54440 or Hoffman (1985) J. Immunol. 135:5-8.

Antibodies which specifically recognize CD3 antigen are described in the prior art, e.g. in Traunecker, EMBO J 10 (1991), 3655-9 and Kipriyanov, Int. J. Cancer 77 (1998), 763-772. Lately, antibodies directed against CD3 have been proposed in the treatment of a variety of diseases. These antibodies or antibody constructs act as either T-cell depleting agents or as mitogenic agents, as disclosed in EP 1 025 854. Human/rodent hybrid antibodies which specifically bind to the human CD3 antigen complex are disclosed in WO 00/05268 and are proposed as immunosuppressive agents, for example, for the treatment of rejection episodes following the transplantation of the renal, septic and cardiac allografts.

However, prior art antibodies directed against CD3 are derived from non-human sources. This leads to several serious problems when using such anti-CD3 antibodies as part of a therapeutic regimen in humans.

One such problem is "cytokine release syndrome (CRS)". CRS is a clinical syndrome which has been observed following the administration of the first few doses of anti-CD3 antibodies and is related to the fact that many antibodies directed against CD3 are mitogenic. In vitro, mitogenic antibodies directed against CD3 induce T cell proliferation and cytokine production. In vivo this mitogenic activity leads to the large-scale release of cytokines, including many T cell-derived cytokines, within the initial hours after the first injection of antibody. The mitogenic capacity of CD3-specific antibodies is monocyte/macrophage dependent and it involves the production of IL-6 and IL-1β by these cells.

CRS symptoms range from frequently reported mild "flu-like" symptoms to less frequently reported severe "shock-like" reactions (which may include cardiovascular and central nervous system manifestations). Symptoms include, inter alia, headache, tremor, nausea/vomiting, diarrhoea, abdominal pain, malaise and muscle/joint aches and pains, generalized weakness, cardiorespiratory events as well as neuropsychiatric events. Severe pulmonary oedema has occurred in patients with fluid overload and in those who appeared not to have a fluid overload. Another serious problem hampering the therapeutic use of, especially, murine monoclonal antibodies is the mounting of a humoral immune response against such antibodies, resulting in the production of human anti-mouse antibodies ("HAMAs") (Schroff (1985) Cancer Res.45:879-885, Shawler (1985) J. Immunol. 135:1530-1535). HAMAs are typically generated during the second week of treatment with the murine antibody and neutralize the murine antibodies, thereby blocking their ability to bind to their intended target. The HAMA response can depend on the murine constant ("Fc") antibody regions or/and the nature of the murine variable ("V") regions.

The prior art contains various approaches to reducing or preventing the production of HAMAs by modifying monoclonal antibodies of non-human origin.

One approach to reducing the immunogenicity of such antibodies is by humanization, as for example described in WO 91/09968 and US 6,407,213. In general, humanization entails substitutions of non-human antibody sequences, e.g. of the framework regions, for corresponding human sequences, as for example is the case with CDR-grafting.

Another approach to reducing the immunogenicity of such antibodies is by deimmunization, as for example described in WO 00/34317, WO 98/52976, WO 02/079415, WO 02/012899 and WO 02/069232. In general, deimmunization entails carrying out substitutions of amino acids within potential T cell epitopes. In this way, the likelihood that a given sequence will give rise to T cell epitopes upon intracellular protein processing is reduced. Moreover, WO 92/10755 describes an approach in which antigenic determinants on proteins are engineered. Particularly, proteins are epitope mapped and their amino acid sequence is changed through genetic engineering.

However, humanized antibodies often exhibit a decreased binding affinity with respect to their target as compared to their non-humanized parent antibodies and also often are still somewhat immunogenic in a human host.

Therefore, the technical problem of the present invention was the provision of means and methods for the treatment of and/or the amelioration of a proliferative disease, a tumorous disease, an inflammatory disease, an immunological disorder, an autoimmune disease, an infectious disease, viral disease, allergic reactions, parasitic reactions, graft-versus-host diseases or host-versus-graft diseases by induction of T cell mediated immune response. The above-mentioned means and methods should overcome the recited disadvantages of known antibody-based therapies.

The solution to said technical problem is achieved by providing the embodiments characterized in the claims.

Accordingly, the present invention relates to CD3 specific binding molecule selected from the group consisting of

(a) a polypeptide having the amino acid sequence of SEQ ID NO.:5, 7, 9, 11, 13, 15, 17, 19, 21, 23, 25, 27, 29, 31, 33, 35, 37, 39, 41, 43 and 45,
(b) a polypeptide encoded by a nucleic acid sequence selected from the group consisting of SEQ ID Nos.: 4, 6, 8, 10, 12, 14, 16, 18, 20, 22, 24, 26, 28, 30, 32, 34, 36, 38, 40, 42 and 44;
(c) a polypeptide encoded by a nucleic acid sequence which is degenerated as a result of the genetic code to a nucleic acid sequence of (b).

In accordance with the present invention the term "CD3 specific binding molecule" relates to a molecule, i.e. a proteinaceous structure/polypeptide, which is capable of binding to and/or interacting with CD3 and/or the CD3 complex or parts of said CD3 and/or parts of said CD3 complex. Most preferably said CD3 specific binding molecules bind to/interact with human CD3 (or parts thereof) and or with the human CD3 complex. Accordingly, the herein defined CD3 binding molecules are active binding molecules in the sense that these CD3 binders show reduced cellular T cell response in vivo (reduced T cell activation) in comparison to a non-deimmunized molecule. The term "deimmunized" is defined herein below.

The term "binding to/interacting with" as used in the context with the present invention defines a binding/ interaction of at least two "antigen-interaction-sites" with each other. The term "antigen-interaction-site" defines, in accordance with the present invention, a motif of a polypeptide which shows the capacity of specific interaction with a specific antigen or a specific group of antigens. Said binding/interaction is also understood to define a "specific recognition". The term "specifically recognizing" means in accordance with this invention that the antibody molecule is capable of specifically interacting with and/or binding to at least two amino acids of each of the human target molecule as defined herein, namely CD3. Antibodies can recognize, interact and/or bind to different epitopes on the same target molecule. Said term relates to the specificity of the antibody molecule, i.e. to its ability to discriminate between the specific regions of the human target molecule as defined herein. The specific interaction of the antigen-interaction-site with its specific antigen may result in an initiation of a signal, e.g. due to the induction of a change of the conformation of the antigen, an oligomerization of the antigen, etc. Thus, specific motifs in the amino acid sequence of the antigen-interaction-site are a result of their primary, secondary or tertiary structure as well as the result of secondary modifications of said structure.

The term "specific interaction" as used in accordance with the present invention means that the CD3 specific binding molecule of the invention does not or essentially does not cross-react with (poly)peptides of similar structures. Cross-reactivity of a panel of binding molecules under investigation may be tested, for example, by assessing binding of said panel of single-chain binding molecules (i.e. CD3 specific binding molecules of the invention in a single-chain context) under conventional conditions (see, e.g., Harlow and Lane, Antibodies: A Laboratory Manual, Cold Spring Harbor Laboratory Press, 1988 and Using Antibodies: A Laboratory Manual, Cold Spring Harbor Laboratory Press, 1999) to the (poly)peptide of interest as well as to a number of more or less (structurally and/or functionally) closely related (poly)peptides. Only those constructs (i.e. antibodies, scFvs and the like) that bind to the (poly)peptide/protein of interest but do not or do not essentially bind to any of the other (poly)peptides which are preferably expressed by the same tissue as the (poly)peptide of interest, e.g. by the cells of the heart tissue, are considered specific for the (poly)peptide/protein of interest and selected for further studies in accordance with the method provided herein and illustrated in the appended examples. These methods may comprise, inter alia, binding studies, blocking and competition studies with structurally and/or functionally closely related molecules. These binding studies also comprise FACS analysis, surface plasmon resonance (SPR, e.g. with BIAcore®), analytical ultracentrifugation, isothermal titration calorimetry, fluorescence anisotropy, fluorescence spectroscopy or by radiolabeled ligand binding assays. Accordingly, examples for the specific interaction of an antigen-interaction-site with a specific antigen may comprise the specificity of a ligand for its receptor. Said definition particularly comprises the interaction of ligands which induce a signal upon binding to its specific receptor. Examples for corresponding ligands comprise cytokines which interact/bind with/to its specific cytokine-receptors. An other example for said interaction, which is also particularly comprised by said definition, is the interaction of an antigenic determinant (epitope) with the antigenic binding site of an antibody.

The term "binding to/interacting with" relates not only to a linear epitope but may also relate to a conformational epitope, a structural epitope or a discontinuous epitope consisting of two regions of the human target molecules or parts thereof. In context of this invention, a conformational epitope is defined by two or more discrete amino acid sequences separated in the primary sequence which come together on the surface of the molecule when the polypeptide folds to the native protein (Sela, (1969) Science 166, 1365 and Laver, (1990) Cell 61, 553-6).

The term "discontinuous epitope" means in context of the invention non-linear epitopes that are assembled from residues from distant portions of the polypeptide chain. These residues come together on the surface when the polypeptide chain folds into a three-dimensional structure to constitute a conformational/structural epitope.

The binding molecules of the present invention are also envisaged to specifically bind to/interact with a conformational/structural epitope(s) composed of and/or comprising the human CD3 complex or parts thereof as disclosed herein below.

Accordingly, specificity can be determined experimentally by methods known in the art and methods as disclosed and described herein. Such methods comprise, but are not limited to Western blots, ELISA-, RIA-, ECL-, IRMA-tests and peptide scans.

The inventive constructs provided herein have the surprising feature of being modified and "deimmunized" yet of being still capable of binding to and/or interacting with CD3 and/or CD3 complex.

The term "deimmunized" as used herein relates to the above-identified inventive CD3 binding molecule, which is modified compared to an original wild type molecule by rendering said wild type molecule non-immunogenic or less immunogenic in humans. Deimmunized molecules according to the invention relate to antibodies or parts thereof (like frameworks and/or CDRs) of non-human origin. Corresponding examples are antibodies or fragments thereof as described in US 4,361,549. The term "deimmunized" also relates to molecules, which show reduced propensity to generate T cell epitopes. In accordance with this invention, the term "reduced propensity to generate T cell epitopes" relates to the removal of T-cell epitopes leading to specific T-cell activation. Furthermore, reduced propensity to generate T cell epitopes means substitution of amino acids contributing to the formation of T cell epitopes, i.e. substitution of amino acids, which are essential for formation of a T cell epitope. In other words, reduced propensity to generate T cell epitopes relates to reduced immunogenicity or reduced capacity to induce antigen independent T cell proliferation. In addition, reduced propensity to generate T cell epitopes relates to deimmunisation, which means loss or reduction of potential T cell epitopes of amino acid sequences inducing antigen independent T cell proliferation. According to the invention, a CD3 binding molecule, which has reduced propensity to generate T cell epitopes is less or preferably non immunogenic compared to a non-deimmunized molecule but which has still retained its capacity to binding to CD3.

The term "T cell epitope" as used herein relates to short peptide sequences which can be released during the degradation of peptides, polypeptide or proteins within cells and subsequently be presented by molecules of the major histocompatibility complex (MHC) in order to trigger the activation of T cells; see inter alia WO 02/066514. For peptides presented by MHC class II such activation of T cells can then induce an antibody response by direct stimulation of B cells to produce said antibodies.

"Reduced propensity to generate T-cell epitopes" and/or "deimmunization" may be measured by techniques known in the art. Preferably, deimmunization of proteins may be tested in vitro by T cell proliferation assay. In this assay PBMCs from donors representing > 80 % of HLA-DR alleles in the world are screened for proliferation in response to either wild type or deimmunized peptides. Ideally cell proliferation is only detected upon loading of the antigen-presenting cells with wild type peptides. Alternatively, one may test deimmunization by expressing HLA-DR tetramers representing all haplotypes. In order to test if de-immunized peptides are presented on HLA-DR haplotypes, binding of e.g. fluorescence-labeled peptides on PBMCs can be measured. Furthermore, deimmunization can be proven by determining whether antibodies against the deimmunized molecules have been generated after administration in patients. The T-cell proliferation assay has been illustrated in the appended examples. A particular preferred method is a T-cell proliferation assay as, inter alia, shown in appended example 2.

The term "CDR" as employed herein relates to "complementary determining region", which is well known in the art. The CDRs are parts of immunoglobulins that determine the specificity of said molecules and make contact with a specific ligand. The CDRs are the most variable part of the molecule and contribute to the diversity of these molecules. There are three CDR regions CDR1, CDR2 and CDR3 in each V domain. CDR-H depicts a CDR region of a variable heavy chain and CDR-L relates to a CDR region of a variable light chain. H means the variable heavy chain and L means the variable light chain. The CDR regions of an Ig-derived region may be determined as described in Kabat (1991). Sequences of Proteins of Immunological Interest, 5th edit., NIH Publication no. 91-3242 U.S. Department of Health and Human Services, Chothia (1987). J. Mol. Biol. 196, 901-917 and Chothia (1989) Nature, 342, 877-883.

In accordance with this invention, a framework region relates to a region in the V domain (VH or VL domain) of immunoglobulins that provides a protein scaffold for the hypervariable complementarity determining regions (CDRs) that make contact with the antigen. In each V domain, there are four framework regions designated FR1, FR2, FR3 and FR4. Framework 1 encompasses the region from the N-terminus of the V domain until the beginning of CDR1, framework 2 relates to the region between CDR1 and CDR2, framework 3 encompasses the region between CDR2 and CDR3 and framework 4 means the region from the end of CDR3 until the C-terminus of the V domain; see, inter alia, Janeway, Immunobiology, Garland Publishing, 2001, 5th ed. Thus, the framework regions encompass all the regions outside the CDR regions in VH or VL domains.

The person skilled in the art is readily in a position to deduce from a given sequence the framework regions and, the CDRs; see Kabat (1991) Sequences of Proteins of Immunological Interest, 5th edit., NIH Publication no. 91-3242 U.S. Department of Health and Human Services, Chothia (1987). J. Mol. Biol. 196, 901-917 and Chothia (1989) Nature, 342, 877-883.

In accordance with the present invention, the CD3 binding molecule of the invention specifically binding to/interacting with human CD3 and having a reduced propensity to generate T cell epitopes, comprises CDR-H1, CDR-H2 and CDR-H3 regions as defined herein and VH-frameworks (frameworks 1, 2, 3, 4) as defined above.

The CD3 binding molecule of the invention comprises a VH-region as depicted in SEQ ID NO.: 50, 52, 54, 56, 58, 60 or 62. Particularly preferred CD3 binding molecules comprise a VH-region as shown in SEQ ID NO.: 58 and 62. Corresponding nucleic acid molecules are shown in SEQ ID NO.: 57 and 61.

The CD3 specific binding molecule of the invention comprises a VL region in its CD3-specific portion, wherein said VL region is selected from the group consisting of SEQ ID NO.: 64 , SEQ ID NO.: 66 or SEQ ID NO.: 68. VL1 as characterized in SEQ ID NO.:64, VL2 as characterized in SEQ ID NO.:66 and VL 3 as characterized in SEQ ID NO.: 68 relate to full deimmunized VL regions in accordance with this invention, and they may be used in various combinations with the above described VH regions.

The term "single-chain" as used in accordance with the present invention means that the VH and VL domain of the CD3 binding molecule are covalently linked, preferably in the form of a co-linear amino acid sequence encoded by a single nucleic acid molecule.

It was surprisingly found that specific CD3 binding molecules having the sequences SEQ ID NO.: 5, 7, 9, 11, 13, 15, 17, 19, 21, 23, 25, 27, 29, 31, 33, 35, 37, 39, 41, 43 and 45 showed reduced immunogenicity, thus being very suitable for the treatment, prevention or amelioration of various diseases. Amino acid substitutions were introduced into the wild type CD3 antibody (SEQ ID NO.:70 and 72) generating CD3 specific binding molecules with reduced immunogenicity. Removal of potential T cell epitopes was shown in T cell proliferation assays with overlapping peptides. In this context of this invention particularly preferred CD3 binding molecules are the molecules as defined by amino acid sequences shown in SEQ ID NOs.: 5, 7, 9, 23, 25, 27, 29, 31, 33, 35, 37, 39, 41, 43 and 45. Most preferred CD3 binding molecules are the molecules as defined by amino acid sequences shown in SEQ ID NOs.: 29, 31, 33, 41, 43 and 45.

In a further embodiment, the invention encompasses a nucleic acid sequence encoding a CD3 specific binding molecule of the invention.

Preferably, said nucleic acid sequence is selected from the group consisting of SEQ ID NO.: 4, 6, 8, 10, 12, 14, 16, 18, 20, 22, 24, 26, 28, 30, 32, 34, 36, 38, 40, 42 and 44. Particularly preferred nucleic acid sequences are sequences as shown in SEQ ID NOs.: 4, 6, 8, 22, 24, 26, 28, 30, 32, 34, 36, 38, 40, 42 and 44. Most preferred are nucleic acid sequences as shown in SEQ ID NOs.: 28, 30, 32, 40, 42, and 44.

It is evident to the person skilled in the art that regulatory sequences may be added to the nucleic acid molecule of the invention. For example, promoters, transcriptional enhancers and/or sequences which allow for induced expression of the polynucleotide of the invention may be employed. A suitable inducible system is for example tetracycline-regulated gene expression as described, e.g., by Gossen and Bujard (Proc. Natl. Acad. Sci. USA 89 (1992), 5547-5551) and Gossen et al. (Trends Biotech. 12 (1994), 58-62), or a dexamethasone-inducible gene expression system as described, e.g. by Crook (1989) EMBO J. 8, 513-519.

Furthermore, it is envisaged for further purposes that nucleic acid molecules may contain, for example, thioester bonds and/or nucleotide analogues. Said modifications may be useful for the stabilization of the nucleic acid molecule against endo- and/or exonucleases in the cell. Said nucleic acid molecules may be transcribed by an appropriate vector containing a chimeric gene which allows for the transcription of said nucleic acid molecule in the cell. In this respect, it is also to be understood that such polynucleotide can be used for "gene targeting" or "gene therapeutic" approaches. In another embodiment said nucleic acid molecules are labeled. Methods for the detection of nucleic acids are well known in the art, e.g., Southern and Northern blotting, PCR or primer extension. This embodiment may be useful for screening methods for verifying successful introduction of the nucleic acid molecules described above during gene therapy approaches.

Said nucleic acid molecule(s) may be a recombinantly produced chimeric nucleic acid molecule comprising any of the aforementioned nucleic acid molecules either alone or in combination. Preferably, the nucleic acid molecule is part of a vector.

The present invention therefore also relates to a vector comprising the nucleic acid molecule described in the present invention.

Many suitable vectors are known to those skilled in molecular biology, the choice of which would depend on the function desired and include plasmids, cosmids, viruses, bacteriophages and other vectors used conventionally in genetic engineering. Methods which are well known to those skilled in the art can be used to construct various plasmids and vectors; see, for example, the techniques described in Sambrook et al. (loc cit.) and Ausubel, Current Protocols in Molecular Biology, Green Publishing Associates and Wiley Interscience, N.Y. (1989), (1994). Alternatively, the polynucleotides and vectors of the invention can be reconstituted into liposomes for delivery to target cells. As discussed in further details below, a cloning vector was used to isolate individual sequences of DNA. Relevant sequences can be transferred into expression vectors where expression of a particular polypeptide is required. Typical cloning vectors include pBluescript SK, pGEM, pUC9, pBR322 and pGBT9. Typical expression vectors include pTRE, pCAL-n-EK, pESP-1, pOP13CAT.

Preferably said vector comprises a nucleic acid sequence which is a regulatory sequence operably linked to said nucleic acid sequence encoding a single chain antibody construct defined herein.

Such regulatory sequences (control elements) are known to the skilled artisan and may include a promoter, a splice cassette, translation initiation codon, translation and insertion site for introducing an insert into the vector. Preferably, said nucleic acid molecule is operatively linked to said expression control sequences allowing expression in eukaryotic or prokaryotic cells.

It is envisaged that said vector is an expression vector comprising the nucleic acid molecule encoding a single chain antibody construct defined herein.

The term "regulatory sequence" refers to DNA sequences, which are necessary to effect the expression of coding sequences to which they are ligated. The nature of such control sequences differs depending upon the host organism. In prokaryotes, control sequences generally include promoter, ribosomal binding site, and terminators. In eukaryotes generally control sequences include promoters, terminators and, in some instances, enhancers, transactivators or transcription factors. The term "control sequence" is intended to include, at a minimum, all components the presence of which are necessary for expression, and may also include additional advantageous components.

The term "operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences. In case the control sequence is a promoter, it is obvious for a skilled person that double-stranded nucleic acid is preferably used.

Thus, the recited vector is preferably an expression vector. An "expression vector" is a construct that can be used to transform a selected host and provides for expression of a coding sequence in the selected host. Expression vectors can for instance be cloning vectors, binary vectors or integrating vectors. Expression comprises transcription of the nucleic acid molecule preferably into a translatable mRNA. Regulatory elements ensuring expression in prokaryotes and/or eukaryotic cells are well known to those skilled in the art. In the case of eukaryotic cells they comprise normally promoters ensuring initiation of transcription and optionally poly-A signals ensuring termination of transcription and stabilization of the transcript. Possible regulatory elements permitting expression in prokaryotic host cells comprise, e.g., the $P_L$, *lac*, *trp* or *tac* promoter in *E. coli*, and examples of regulatory elements permitting expression in eukaryotic host cells are the *AOX1* or *GAL1* promoter in yeast or the CMV-, SV40-, RSV-promoter (Rous sarcoma virus), CMV-enhancer, SV40-enhancer or a globin intron in mammalian and other animal cells.

Beside elements which are responsible for the initiation of transcription such regulatory elements may also comprise transcription termination signals, such as the SV40-poly-A site or the tk-poly-A site, downstream of the polynucleotide. Furthermore, depending on the expression system used leader sequences capable of directing the polypeptide to a cellular compartment or secreting it into the medium may be added to the coding sequence of the recited nucleic acid sequence and are well known in the art; see also, e.g., appended example 1. The leader sequence(s) is (are) assembled in appropriate phase with translation, initiation and termination sequences, and preferably, a leader sequence capable of directing secretion of translated protein, or a portion thereof, into the periplasmic space or extracellular medium. Optionally, the heterologous sequence can encode a fusion protein including an N-terminal identification peptide imparting desired characteristics, e.g., stabilization or simplified purification of expressed recombinant product; see supra. In this context, suitable expression vectors are known in the art such as Okayama-Berg cDNA expression vector pcDV1 (Pharmacia), pCDM8, pRc/CMV, pcDNA1, pcDNA3 (In-vitrogene), pEF-DHFR, pEF-ADA or pEF-neo (Raum et al. Cancer Immunol Immunother (2001) 50(3), 141-150) or pSPORT1 (GIBCO BRL).

Preferably, the expression control sequences will be eukaryotic promoter systems in vectors capable of transforming of transfecting eukaryotic host cells, but control sequences for prokaryotic hosts may also be used. Once the vector has been incorporated into the appropriate host, the host is maintained under conditions suitable for high level expression of the nucleotide sequences, and as desired, the collection and purification of the polypeptide of the invention may follow; see, e.g., the appended examples.

An alternative expression system which could be used is an insect system. In one such system, *Autographa californica* nuclear polyhedrosis virus (AcNPV) is used as a vector to express foreign genes in *Spodoptera frugiperda* cells or in *Trichoplusia* larvae. The coding sequence of a recited nucleic acid molecule may be cloned into a nonessential region of the virus, such as the polyhedrin gene, and placed under control of the polyhedrin promoter. Successful insertion of said coding sequence will render the polyhedrin gene inactive and produce recombinant virus lacking coat protein coat. The recombinant viruses are then used to infect S. *frugiperda* cells or *Trichoplusia* larvae in which the protein of the invention is expressed (Smith, J. Virol. 46 (1983), 584; Engelhard, Proc. Nat. Acad. Sci. USA 91 (1994), 3224-3227).

Additional regulatory elements may include transcriptional as well as translational enhancers. Advantageously, the above-described vectors of the invention comprises a selectable and/or scorable marker.

Selectable marker genes useful for the selection of transformed cells and, e.g., plant tissue and plants are well known to those skilled in the art and comprise, for example, antimetabolite resistance as the basis of selection for dhfr, which confers resistance to methotrexate (Reiss, Plant Physiol. (Life Sci. Adv.) 13 (1994), 143-149); npt, which confers resistance to the aminoglycosides neomycin, kanamycin and paromycin (Herrera-Estrella, EMBO J. 2 (1983), 987-995) and hygro, which confers resistance to hygromycin (Marsh, Gene 32 (1984), 481-485). Additional selectable genes have been described, namely trpB, which allows cells to utilize indole in place of tryptophan; hisD, which allows cells to utilize histinol in place of histidine (Hartman, Proc. Natl. Acad. Sci. USA 85 (1988), 8047); mannose-6-phosphate isomerase which allows cells to utilize mannose (WO 94/20627) and ODC (ornithine decarboxylase) which confers resistance to the ornithine decarboxylase inhibitor, 2-(difluoromethyl)-DL-ornithine, DFMO (McConlogue, 1987, In: Current Communications in Molecular Biology, Cold Spring Harbor Laboratory ed.) or deaminase from Aspergillus terreus which confers resistance to Blasticidin S (Tamura, Biosci. Biotechnol. Biochem. 59 (1995), 2336-2338).

Useful scorable markers are also known to those skilled in the art and are commercially available. Advantageously, said marker is a gene encoding luciferase (Giacomin, Pl. Sci. 116 (1996), 59-72; Scikantha, J. Bact. 178 (1996), 121), green fluorescent protein (Gerdes, FEBS Lett. 389 (1996), 44-47) or ß-glucuronidase (Jefferson, EMBO J. 6 (1987), 3901-3907). This embodiment is particularly useful for simple and rapid screening of cells, tissues and organisms containing a recited vector.

As described above, the recited nucleic acid molecule can be used alone or as part of a vector to express the encoded CD3 specific construct in cells, for, e.g., purification but also for gene therapy purposes. The nucleic acid molecules or vectors containing the DNA sequence(s) encoding any one of the above described CD3 binding molecule is introduced into the cells which in turn produce the polypeptide of interest. Gene therapy, which is based on introducing therapeutic genes into cells by ex-vivo or in-vivo techniques is one of the most important applications of gene transfer. Suitable vectors, methods or gene-delivery systems for in-vitro or in-vivo gene therapy are described in the literature and are known to the person skilled in the art; see, e.g., Giordano, Nature Medicine 2 (1996), 534-539; Schaper, Circ. Res. 79 (1996), 911-919; Anderson, Science 256 (1992), 808-813; Verma, Nature 389 (1994), 239; Isner, Lancet 348 (1996), 370-374; Muhlhauser, Circ. Res. 77 (1995), 1077-1086; Onodera, Blood 91 (1998), 30-36; Verma, Gene Ther. 5 (1998), 692-699; Nabel, Ann. N.Y. Acad. Sci. 811 (1997), 289-292; Verzeletti, Hum. Gene Ther. 9 (1998), 2243-51; Wang, Nature Medicine 2 (1996), 714-716; WO 94/29469; WO 97/00957, US 5,580,859; US 5,589,466; or Schaper, Current Opinion in Biotechnology 7 (1996), 635-640. The recited nucleic acid molecules and vectors may be designed for direct introduction or for introduction via liposomes, or viral vectors (e.g., adenoviral, retroviral) into the cell. Preferably, said cell is a germ line cell, embryonic cell, or egg cell or derived therefrom, most preferably said cell is a stem cell. An example for an embryonic stem cell can be, inter alia, a stem cell as described in, Nagy, Proc. Natl. Acad. Sci. USA 90 (1993), 8424-8428.

In accordance with the above, the present invention relates to methods to derive vectors, particularly plasmids, cosmids, viruses and bacteriophages used conventionally in genetic engineering that comprise a nucleic acid molecule encoding the polypeptide sequence of a single chain antibody construct defined herein. Preferably, said vector is an expression vector and/or a gene transfer or targeting vector. Expression vectors derived from viruses such as retroviruses, vaccinia virus, adeno-associated virus, herpes viruses, or bovine papilloma virus, may be used for delivery of the recited polynucleotides or vector into targeted cell populations. Methods which are well known to those skilled in the art can be used to construct recombinant vectors; see, for example, the techniques described in Sambrook et al. (loc cit.), Ausubel (1989, loc cit.) or other standard text books. Alternatively, the recited nucleic acid molecules and vectors can be reconstituted into liposomes for delivery to target cells. The vectors containing the nucleic acid molecules of the invention can be transferred into the host cell by well-known methods, which vary depending on the type of cellular host. For example, calcium chloride transfection is commonly utilized for prokaryotic cells, whereas calcium phosphate treatment or electroporation may be used for other cellular hosts; see Sambrook, supra.

The recited vector may, inter alia, be the pEF-DHFR, pEF-ADA or pEF-neo. The vectors pEF-DHFR, pEF-ADA and pEF-neo have been described in the art, e.g. in Mack et al. (PNAS (1995) 92, 7021-7025) and Raum et al. (Cancer Immunol Immunother (2001) 50(3), 141-150).

The invention also provides for a host transformed or transfected with a vector as described herein. Said host may be produced by introducing said at least one of the above described vector or at least one of the above described nucleic acid molecules into the host. The presence of said at least one vector or at least one nucleic acid molecule in the host may mediate the expression of a gene encoding the above described single chain antibody constructs.

The described nucleic acid molecule or vector which is introduced in the host may either integrate into the genome of the host or it may be maintained extrachromosomally.

The host can be any prokaryotic or eukaryotic cell.

The term "prokaryote" is meant to include all bacteria which can be transformed or transfected with DNA or RNA molecules for the expression of a protein of the invention. Prokaryotic hosts may include gram negative as well as gram positive bacteria such as, for example, E. coli, S. typhimurium, Serratia marcescens and Bacillus subtilis. The term "eukaryotic" is meant to include yeast, higher plant, insect and preferably mammalian cells. Depending upon the host employed in a recombinant production procedure, the protein encoded by the polynucleotide of the present invention may be glycosylated or may be non-glycosylated. Especially preferred is the use of a plasmid or a virus containing the coding sequence of the polypeptide of the invention and genetically fused thereto an N-terminal FLAG-tag and/or C-terminal His-tag. Preferably, the length of said FLAG-tag is about 4 to 8 amino acids, most preferably 8 amino acids. An above described polynucleotide can be used to transform or transfect the host using any of the techniques commonly known to those of ordinary skill in the art. Furthermore, methods for preparing fused, operably linked genes and expressing them in, e.g., mammalian cells and bacteria are well-known in the art (Sambrook, loc cit.).

Preferably, said the host is a bacteria, an insect, fungal, plant or animal cell.

It is particularly envisaged that the recited host may be a mammalian cell, more preferably a human cell or human cell line.

Particularly preferred host cells comprise CHO cells, COS cells, myeloma cell lines like SP2/0 or NS/0. As illustrated in the appended examples, particularly preferred are CHO-cells as hosts.

In a further embodiment, the present invention thus relates to a process for the production of a CD3 specific binding molecule described above comprising cultivating a cell and/or the host of the invention under conditions suitable for the expression/allowing the expression of said CD3 specific binding molecule and isolating/recovering the CD3 specific binding molecule from the cell or the culture/culture medium.

The transformed hosts can be grown in fermentors and cultured according to techniques known in the art to achieve optimal cell growth. The polypeptide of the invention can then be isolated from the growth medium, cellular lysates, or cellular membrane fractions. The isolation and purification of the, e.g., microbially expressed polypeptides of the invention may be by any conventional means such as, for example, preparative chromatographic separations and immunological separations such as those involving the use of monoclonal or polyclonal antibodies directed, e.g., against a tag of the polypeptide of the invention or as described in the appended examples.

Furthermore, the invention provides for a composition comprising a (human) CD3-specific binding molecule as defined herein or a (human) CD3-specific binding molecule as produced by the process disclosed above, a nucleic acid molecule of the invention, a vector or a host of the invention. Said composition may, optionally, also comprise a proteinaceous compound capable of providing an activation signal for immune effector cells. Most preferably, said composition is a pharmaceutical composition further comprising, optionally, suitable formulations of carrier, stabilizers and/or excipients.

In accordance with this invention, the term "pharmaceutical composition" relates to a composition for administration to a patient, preferably a human patient. In a preferred embodiment, the pharmaceutical composition comprises a composition for parenteral, transdermal, intraluminal, intra arterial, intrathecal administration or by direct injection into the tissue or tumour. It is in particular envisaged that said pharmaceutical composition is administered to a patient via infusion or injection. Administration of the suitable compositions may be effected by different ways, e.g., by intravenous, intraperitoneal, subcutaneous, intramuscular, topical or intradermal administration. The pharmaceutical composition of the present invention may further comprise a pharmaceutically acceptable carrier. Examples of suitable pharmaceutical carriers are well known in the art and include phosphate buffered saline solutions, water, emulsions, such as oil/water emulsions, various types of wetting agents, sterile solutions, etc. Compositions comprising such carriers can be formulated by well known conventional methods. These pharmaceutical compositions can be administered to the subject at a suitable dose. The dosage regimen will be determined by the attending physician and clinical factors. As is well known in the medical arts, dosages for any one patient depends upon many factors, including the patient's size, body surface area, age, the particular compound to be administered, sex, time and route of administration, general health, and other drugs being administered concurrently. Generally, the regimen as a regular administration of the pharmaceutical composition should be in the range of 1 μg to 5 g units per day. However, a more preferred dosage for continuous infusion might be in the range of 0.01 μg to 2 mg, preferably 0.01 μg to 1 mg, more preferably 0.01 μg to 100 μg, even more preferably 0.01 μg to 50 μg and most preferably 0.01 μg to 10 μg units per kilogram of body weight per hour. Particularly preferred dosages are recited herein below. Progress can be monitored by periodic assessment. Dosages will vary but a preferred dosage for intravenous administration of DNA is from approximately $10^6$ to $10^{12}$ copies of the DNA molecule. The compositions of the invention may be administered locally or systematically. Administration will generally be parenterally, e.g., intravenously; DNA may also be administered directed to the target site, e.g., by biolistic delivery to an internal or external target site or by catheter to a site in an artery. Preparations for parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's, or fixed oils. Intravenous vehicles include fluid and nutrient replenishes, electrolyte replenishers (such as those based on Ringer's dextrose), and the like. Preservatives and other additives may also be present such as, for example, antimicrobials, anti-oxidants, chelating agents, and inert gases and the like. In addition, the pharmaceutical composition of the present invention might comprise proteinaceous carriers, like, e.g., serum albumine or immunoglobuline, preferably of human origin. It is envisaged that the pharmaceutical composition of the invention might comprise, in addition to the proteinaceous CD3 binding molecules or nucleic acid molecules or vectors encoding the same (as described in this invention), further biologically active agents, depending on the intended use of the pharmaceutical composition. Such agents might be drugs acting on the gastro-intestinal system, drugs acting as cytostatica, drugs preventing hyperurikemia, drugs inhibiting immunereactions (e.g. corticosteroids), drugs acting on the circulatory system and/or agents such as T-cell co-stimulatory molecules or cytokines known in the art.

Possible indications for administration of the composition(s) of the invention are tumorous diseases, cancers, especially epithelial cancers/carcinomas such as breast cancer, colon cancer, prostate cancer, head and neck cancer, skin cancer (melanoma), cancers of the genito-urinary tract, e.g. ovarial cancer, endometrial cancer, cervix cancer and kidney cancer, lung cancer, gastric cancer, cancer of the small intestine, liver cancer, pancreas cancer, gall bladder cancer, cancers of the bile duct, esophagus cancer, cancer of the salivatory glands and cancer of the thyroid gland or other tumorous diseases like haematological tumors, gliomas, sarcomas or osteosarcomas.

The composition of the invention as described above may also be a diagnostic composition further comprising, optionally, means and methods for detection.

The CD3-specific binding molecules provided herein are also suited for use in immunoassays in which they can be utilized in liquid phase or bound to a solid phase carrier. Examples of immunoassays which can utilize the polypeptide of the invention are competitive and non-competitive immunoassays in either a direct or indirect format. Examples of such immunoassays are the enzyme linked immunosorbent assay (ELISA), enzyme immunoassay (EIA), radioimmunoassay (RIA), the sandwich (immunometric assay) and the Western blot assay.

The CD3 specific binding molecules of the invention can be bound to many different carriers and used to isolate cells specifically bound to said polypeptides. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble, e.g. as beads, for the purposes of the invention.

There are many different labels and methods of labeling known to those of ordinary skill in the art. Examples of the types of labels which can be used in the present invention include enzymes, radioisotopes, colloidal metals, fluorescent compounds, chemiluminescent compounds, and bioluminescent compounds; see also the embodiments discussed hereinabove.

In a most preferred embodiment of the present invention, the use of a CD3 specific binding molecule of the invention, of a vector or of a host of the invention for the preparation of a pharmaceutical composition is envisaged. Said pharmaceutical composition may be employed in the prevention, treatment or amelioration of a proliferative disease, a tumorous disease, an inflammatory disease, an immunological disorder, an autoimmune disease, an infectious disease, viral disease, allergic reactions, parasitic reactions, graft-versus-host diseases or host-versus-graft diseases.

The invention is also useful in a method for the prevention, treatment or amelioration of a proliferative disease, a tumorous disease, an inflammatory disease, an immunological disorder, an autoimmune disease, an infectious disease, viral disease, allergic reactions, parasitic reactions, graft-versus-host diseases or host-versus-graft diseases comprising the administration of a CD3 specific binding molecule of the invention or a CD3 specific binding molecule as produced by the process described herein, of a nucleic acid molecule, a vector or a host of the invention to a subject in need of such a prevention, treatment or amelioration . Preferably, said subject is a human.

Finally, the invention provides for a kit comprising the CD3 specific binding molecule, a nucleic acid molecule, a vector or a host of the invention.

Said kit is particularly useful in the preparation of the pharmaceutical composition of the present invention and may, inter alia, consist of a container useful for injections or infusions. Advantageously, the kit of the present invention further comprises, optionally (a) buffer(s), storage solutions and/or remaining reagents or materials required for the conduct of medical or scientific purposes. Furthermore, parts of the kit of the invention can be packaged individually in vials or bottles or in combination in containers or multicontainer units. The kit of the present invention may be advantageously used, inter alia, for carrying out the method of the invention and could be employed in a variety of applications referred herein, e.g., as research tools or medical tools. The manufacture of the kits preferably follows standard procedures which are known to the person skilled in the art.

These and other embodiments are disclosed and encompassed by the description and Examples of the present invention. Further literature concerning any one of the antibodies, methods, uses and compounds to be employed in accordance with the present invention may be retrieved from public libraries and databases, using for example electronic devices. For example, the public database "Medline", available on the Internet, may be utilized, for example under http://www.ncbi.nlm.nih.gov/PubMed/medline.html. Further databases and addresses, such as http://www.ncbi.nlm.nih.gov/, http://www.infobio-gen.fr/, http://www.fmi.ch/biology/research tools.html, http://www.tigr.org/, are known to the person skilled in the art and can also be obtained using, e.g., http://www.lycos.com or http://www.google.com.

The figures show:

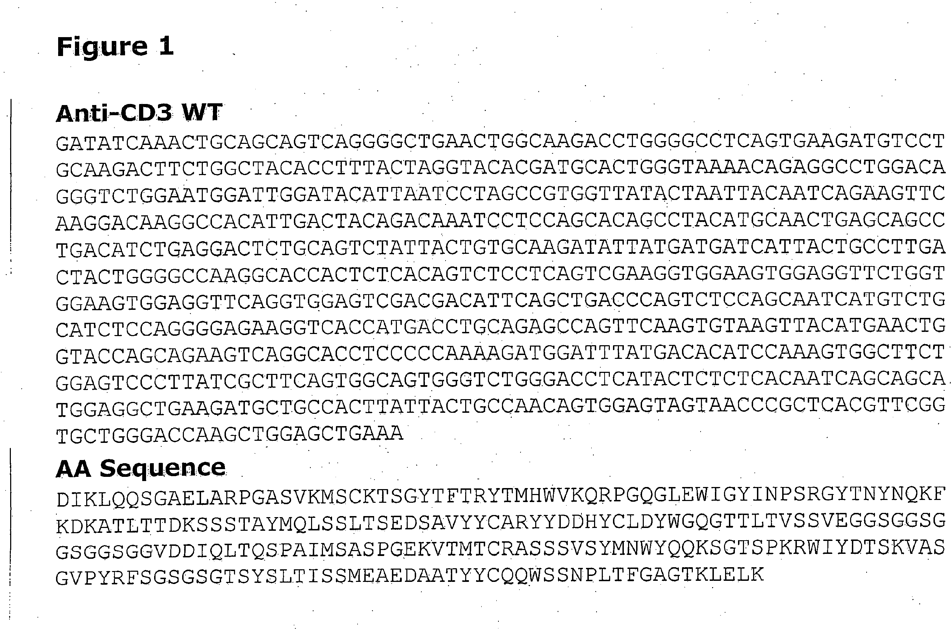

**Figure 1**. DNA and amino acid sequences of non-deimmunized anti-CD3 cassette (SEQ ID Nos.: 1 and 2).

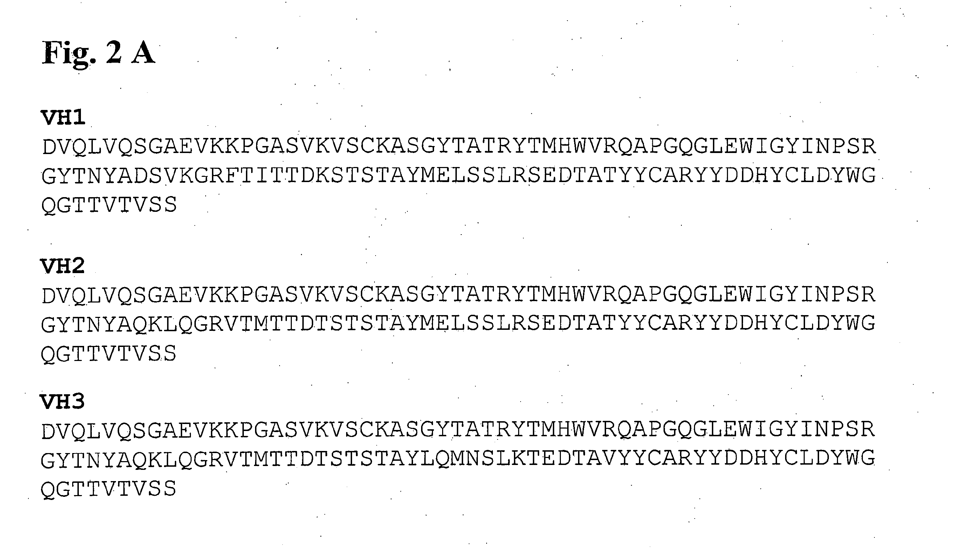

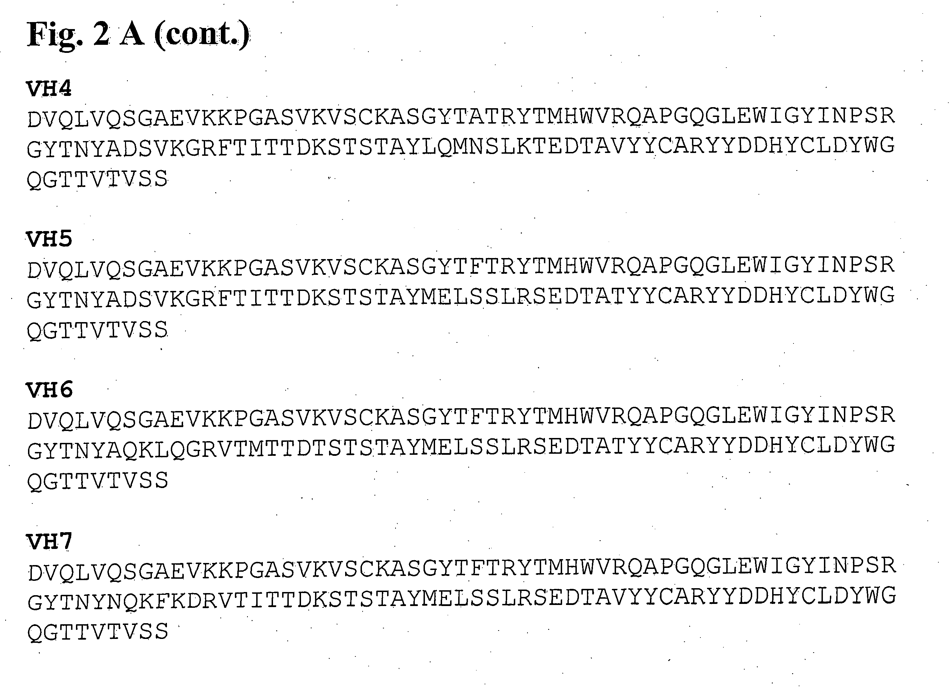

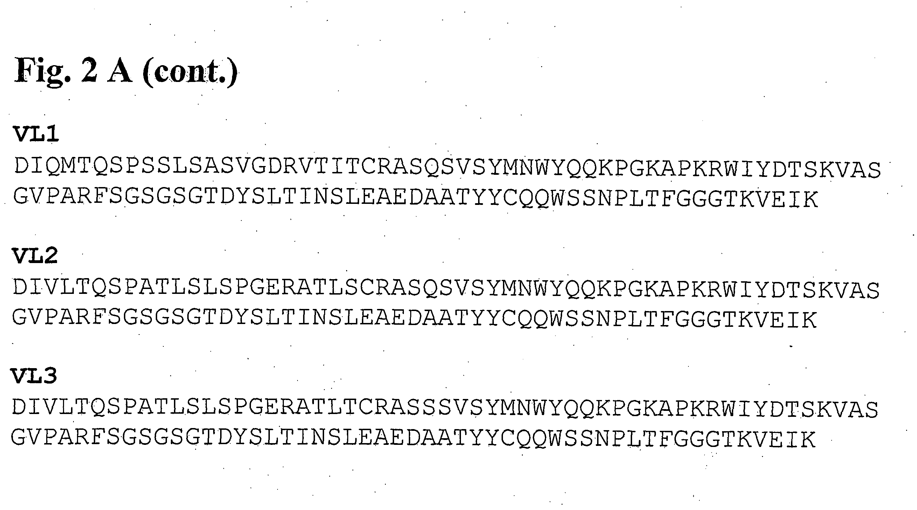

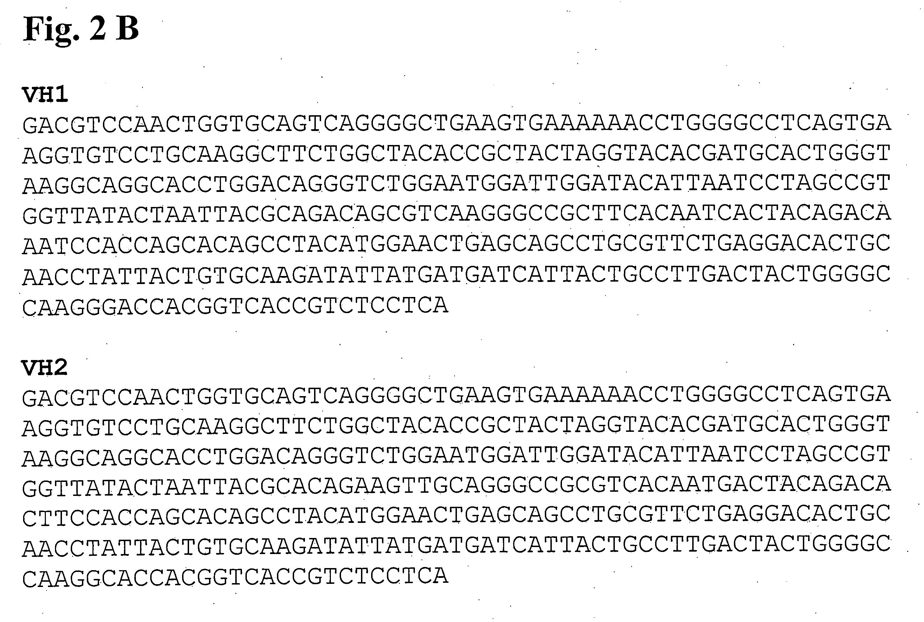

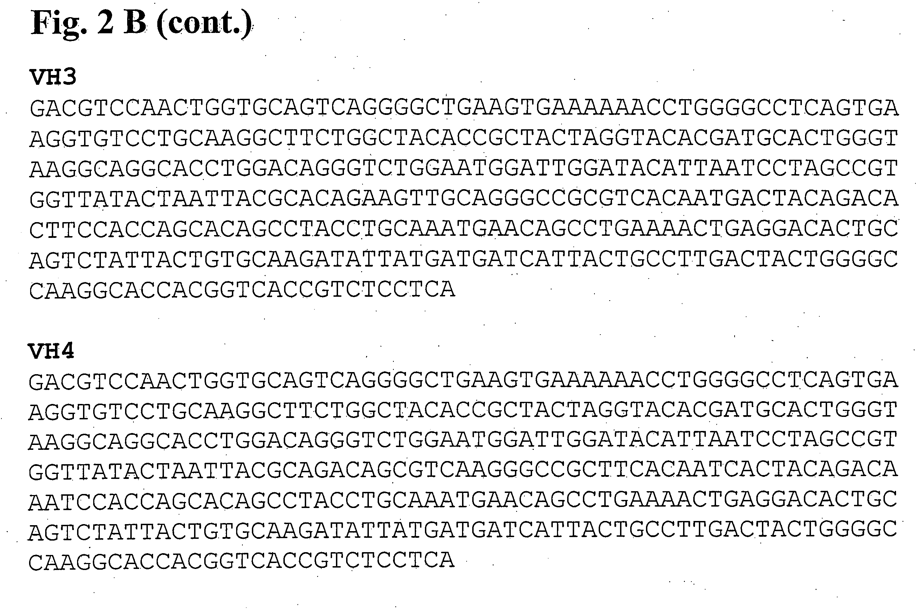

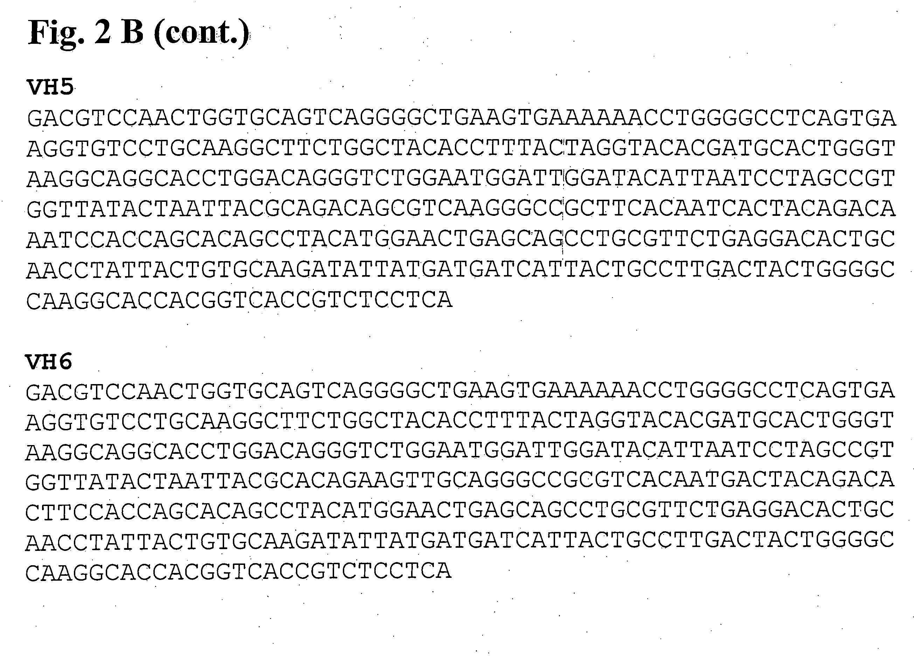

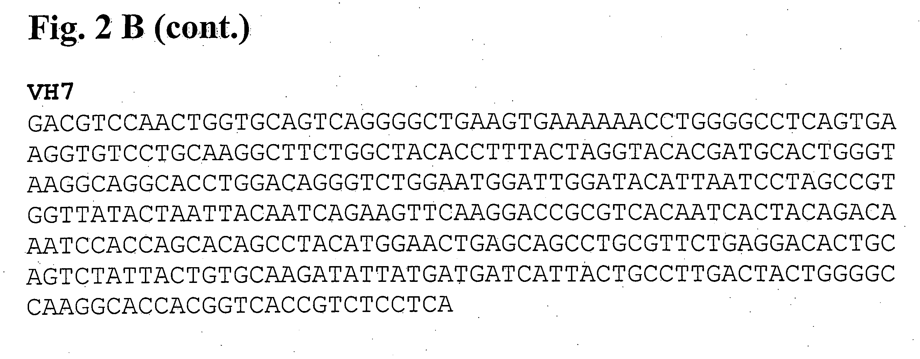

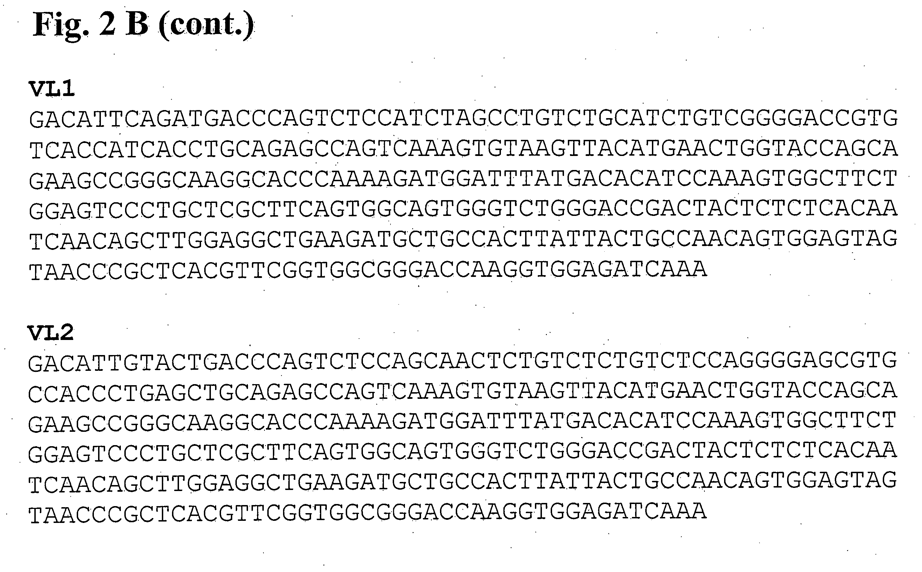

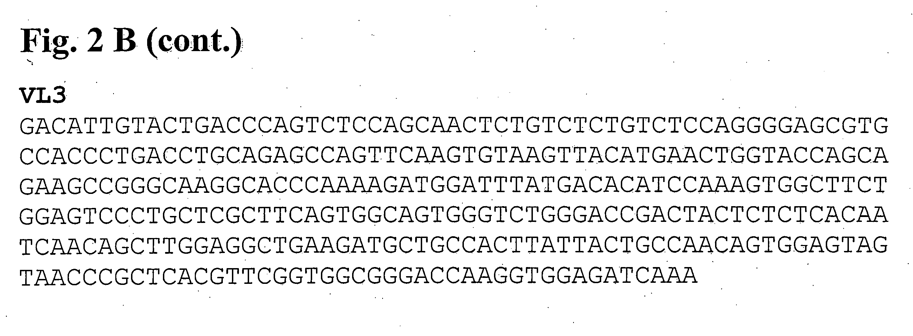

**Figure 2.** DNA and amino acid sequences of deimmunized CD3 specific binding molecules. (A) Amino acid sequences of the heavy chains VH1 (SEQ ID NO.:50), VH2 (SEQ ID NO.:52), VH3 (SEQ ID NO.:54), VH4 (SEQ ID NO.:56) VH5 (SEQ ID NO.:58), VH6 (SEQ ID NO.:60) and VH7 (SEQ ID NO.:62) and light chains VL1 (SEQ ID NO.:64), VL2 (SEQ ID NO.:66) and VL3 (SEQ ID NO.:68), respectively. B) Nucleotide sequences of the heavy chains VH1 (SEQ ID NO.:49), VH2 (SEQ ID NO.:51), VH3 (SEQ ID NO.:53), VH4 (SEQ ID NO.:55), VH5 (SEQ ID NO.:57), VH6 (SEQ ID NO.:59) and VH7 (SEQ ID NO.:61) and light chains VL1 (SEQ ID NO.:63), VL2 (SEQ ID NO.:65) and VL3 (SEQ ID NO.:67), respectively,

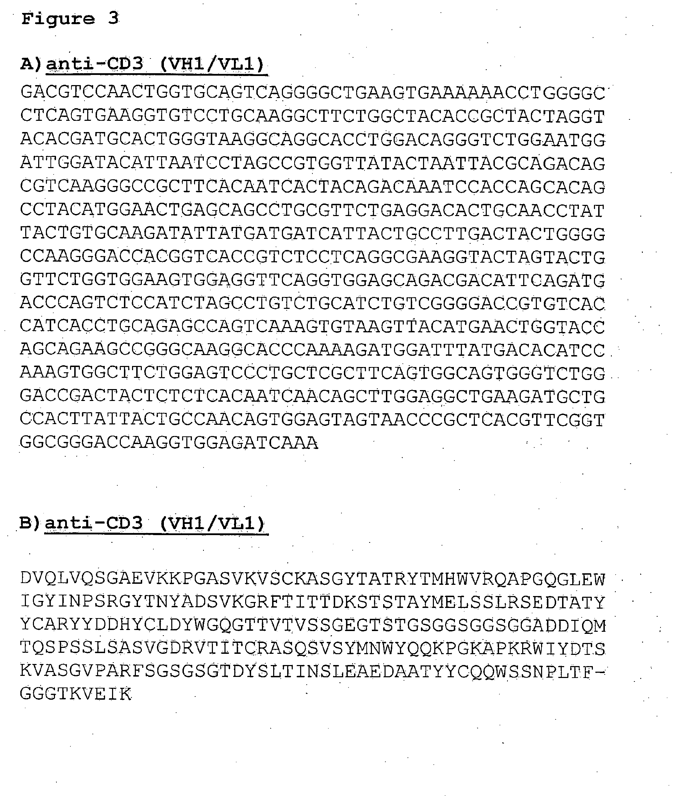

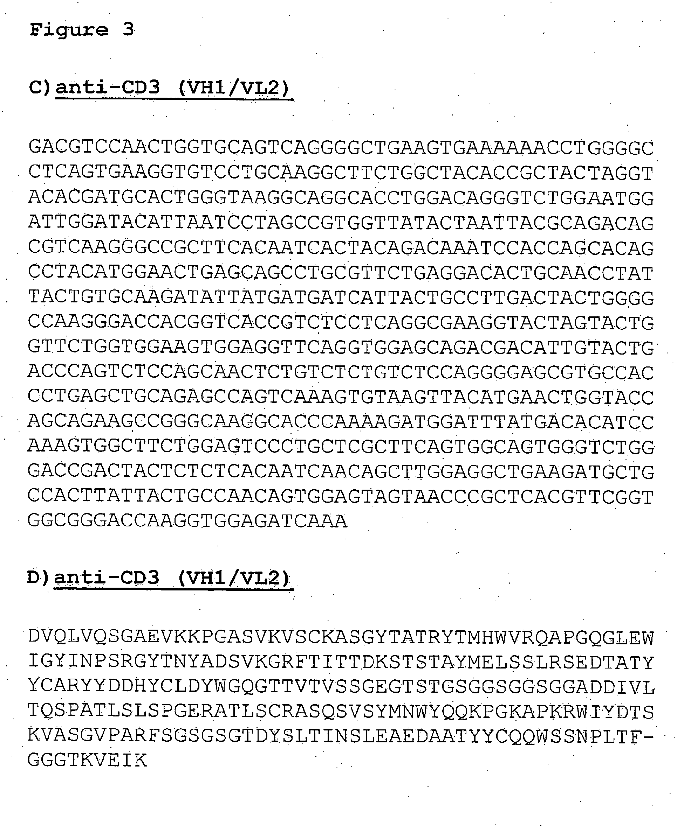

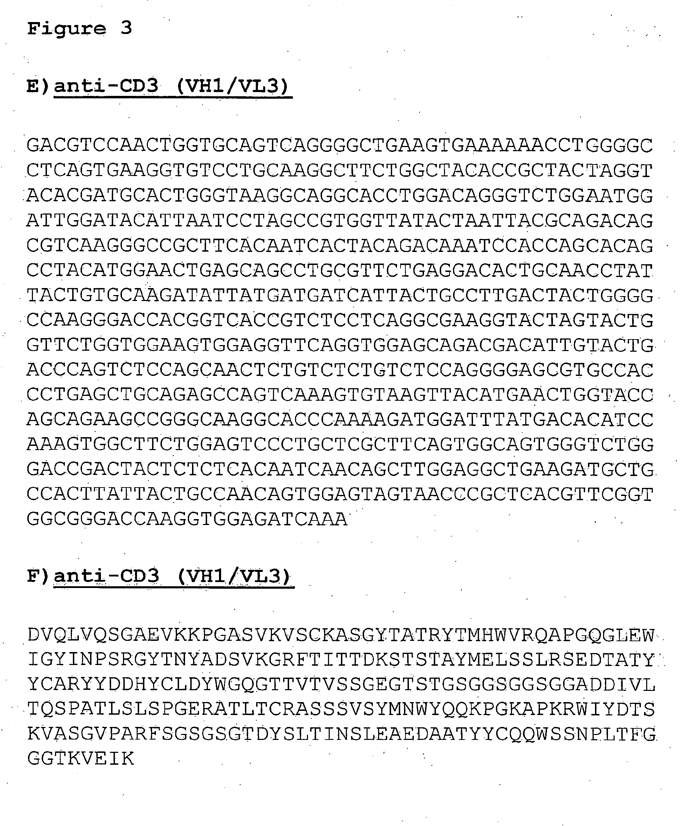

**Figure 3.** DNA and amino acid sequences of deimmunized CD3 specific binding molecules. (A) Nucleotide sequence of anti-CD3 (VH1/VL1) (SEQ ID NO.:4) B) Amino acid sequence of anti-CD3 (VH1/VL1) (SEQ ID NO.:5) C) Nucleotide sequence of anti-CD3 (VH1/VL2) (SEQ ID NO.:6) D) Amino acid sequence of anti-CD3 (VH1/VL2) (SEQ ID NO.:7) E) Nucleotide sequence of anti-CD3 (VH1/VL3) (SEQ ID NO.:8) F) Amino acid sequence of anti-CD3 (VH1/VL3) (SEQ ID NO.:9).

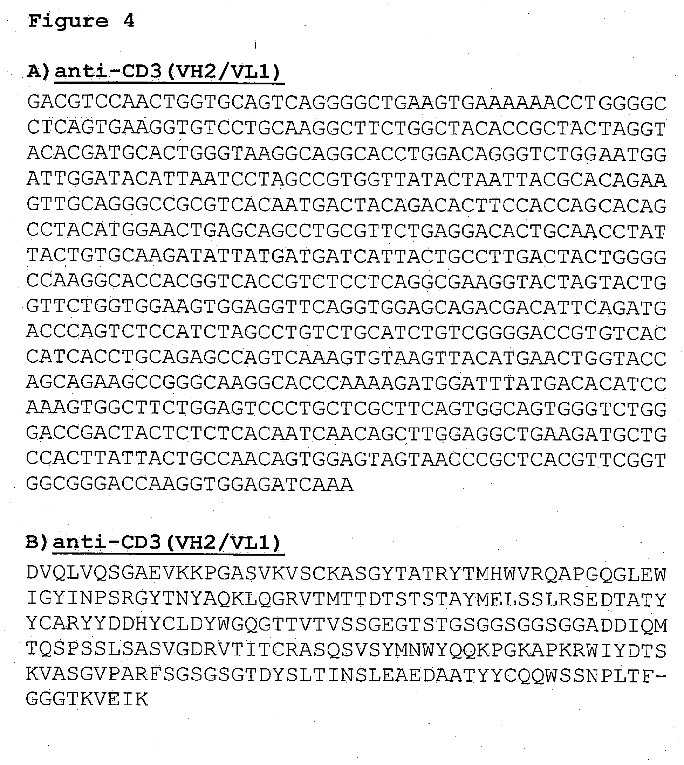

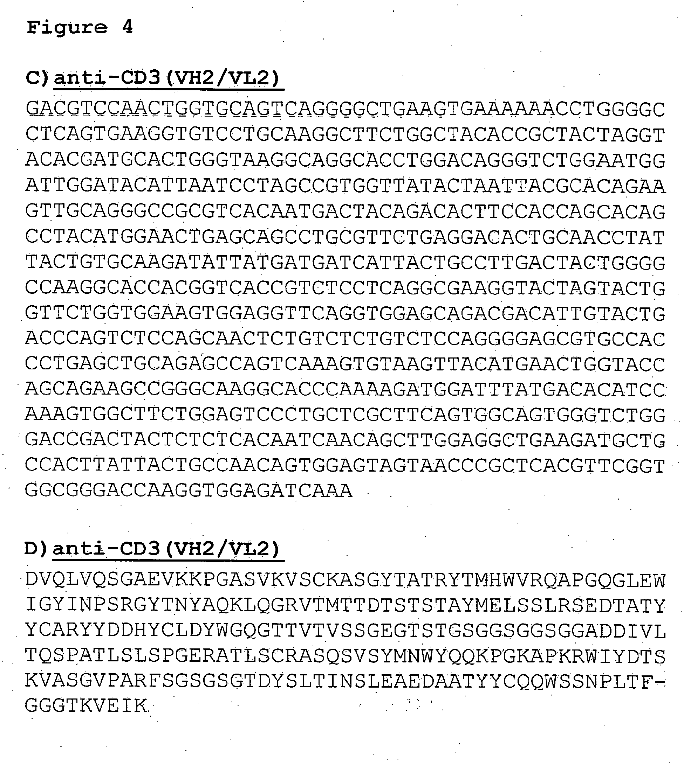

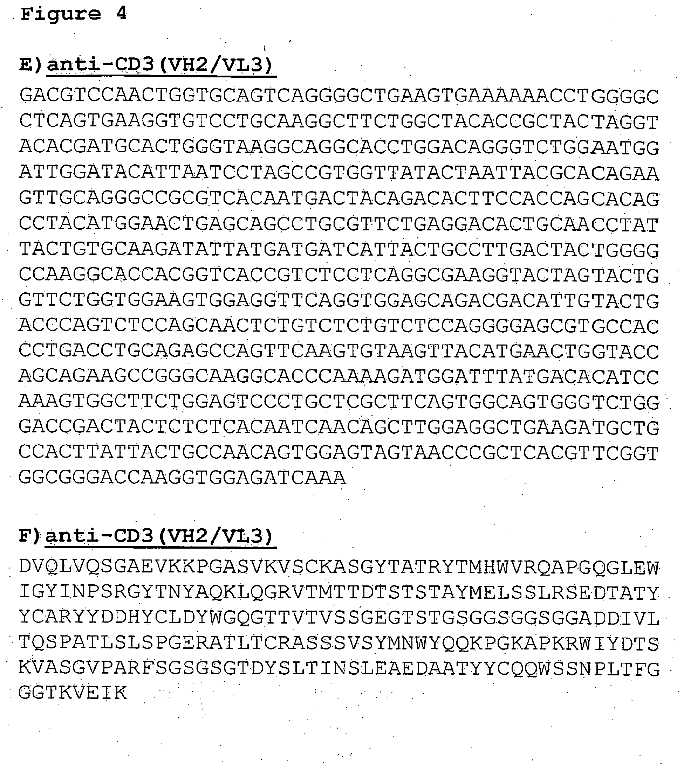

**Figure 4.** DNA and amino acid sequences of deimmunized CD3 specific binding molecules. (A) Nucleotide sequence of anti-CD3 (VH2/VL1) (SEQ ID NO.:10) B) Amino acid sequence of anti-CD3 (VH2/VL1) (SEQ ID NO.: 11) C) Nucleotide sequence of anti-CD3 (VH2/VL2) (SEQ ID NO.:12) D) Amino acid sequence of anti-CD3 (VH2/VL2) (SEQ ID NO.:13) E) Nucleotide sequence of anti-CD3 (VH2/VL3) (SEQ ID NO.:14) F) Amino acid sequence of anti-CD3 (VH2/VL3) (SEQ ID NO.:15).

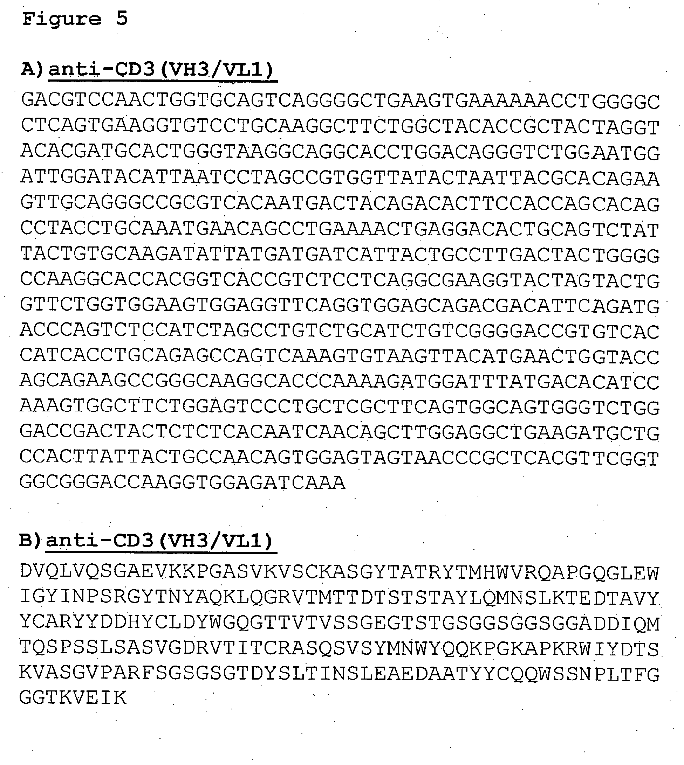

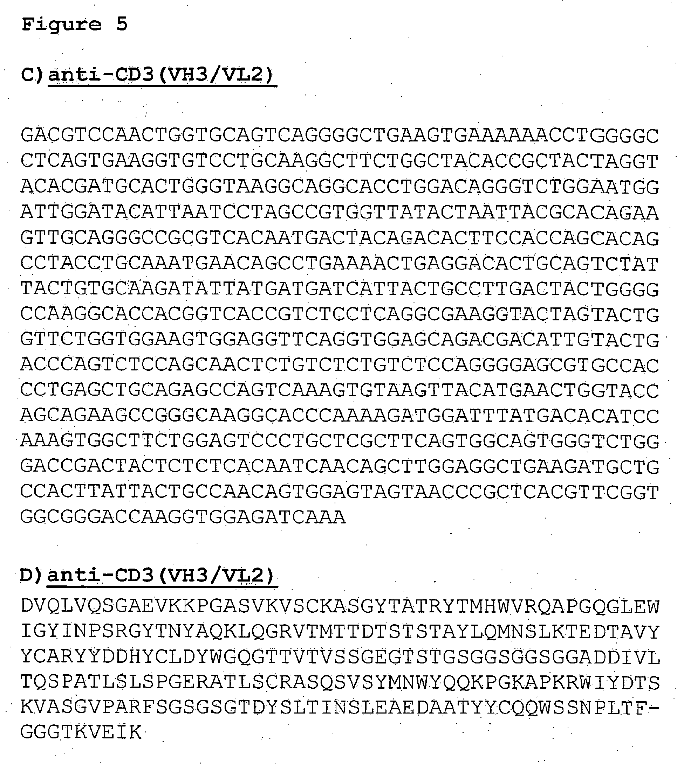

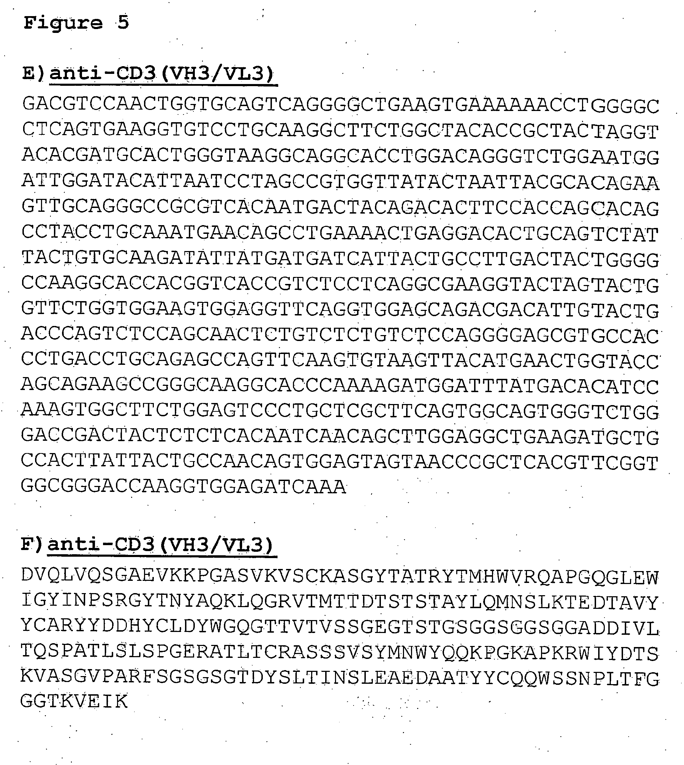

**Figure 5.** DNA and amino acid sequences of deimmunized CD3 specific binding molecules. (A) Nucleotide sequence of anti-CD3 (VH3/VL1) (SEQ ID NO.:16) B) Amino acid sequence of anti-CD3 (VH3/VL1) (SEQ ID NO.: 17) C) Nucleotide sequence of anti-CD3 (VH3/VL2) (SEQ ID NO.:18) D) Amino acid sequence of anti-CD3 (VH3/VL2) (SEQ ID NO.:19) E) Nucleotide sequence of anti-CD3 (VH3/VL3) (SEQ ID NO.:20) F) Amino acid sequence of anti-CD3 (VH3/VL3) (SEQ ID NO.:21).

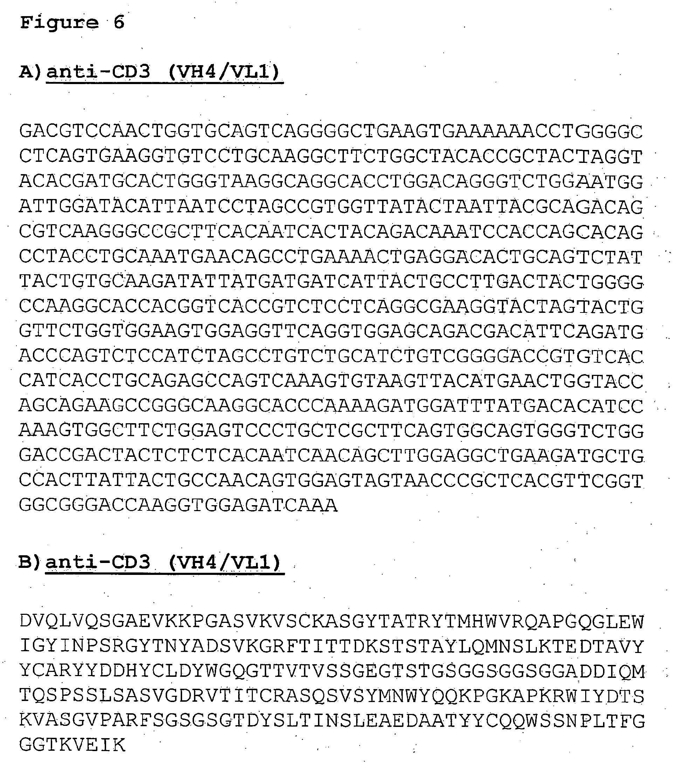

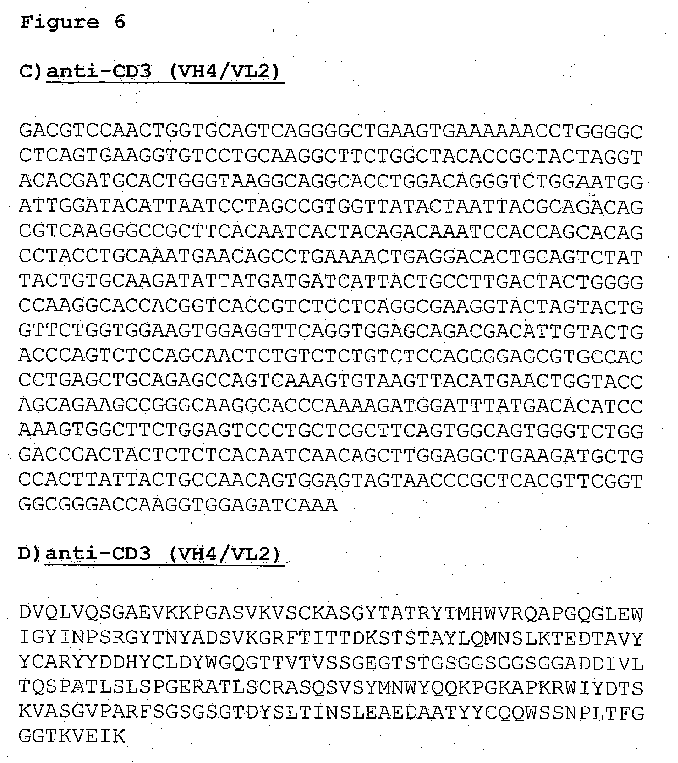

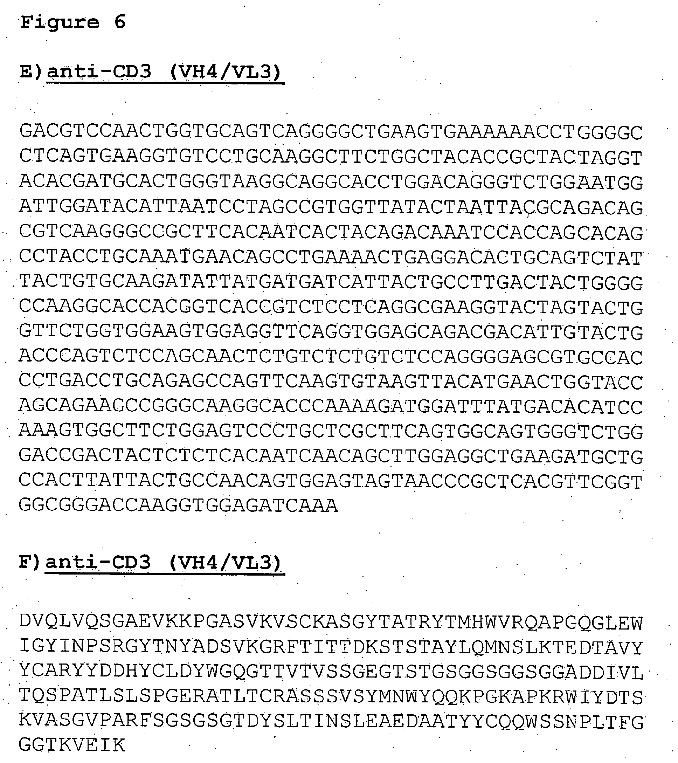

**Figure 6.** DNA and amino acid sequences of deimmunized CD3 specific binding molecules. (A) Nucleotide sequence of anti-CD3 (VH4/VL1) (SEQ ID NO.:22) B) Amino acid sequence of anti-CD3 (VH4/VL1) (SEQ ID NO.: 23) C) Nucleotide sequence of anti-CD3 (VH4/VL2) (SEQ ID NO.:24) D) Amino acid sequence of anti-CD3 (VH4/VL2) (SEQ ID NO.:25) E) Nucleotide sequence of anti-CD3 (VH4/VL3) (SEQ ID NO.:26) F) Amino acid sequence of anti-CD3 (VH4/VL3) (SEQ ID NO.:27).

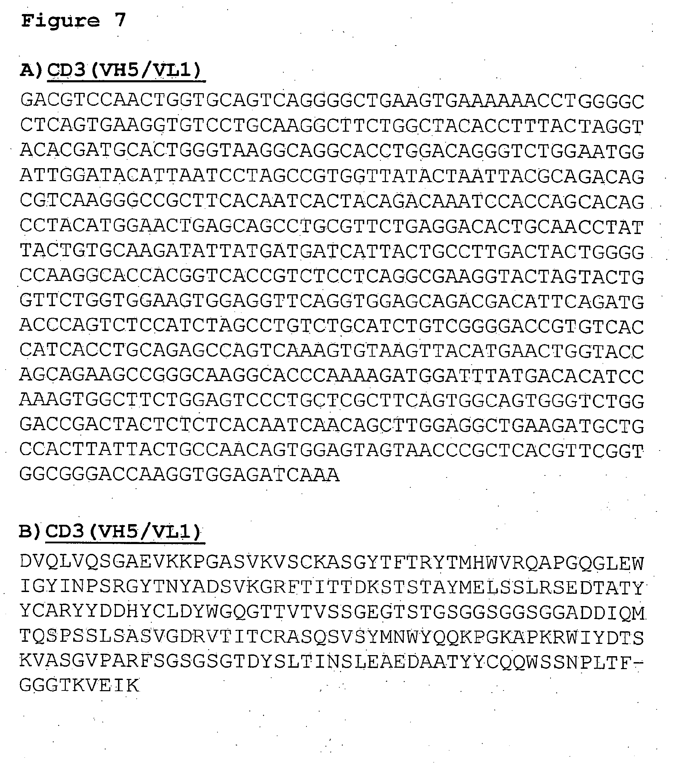

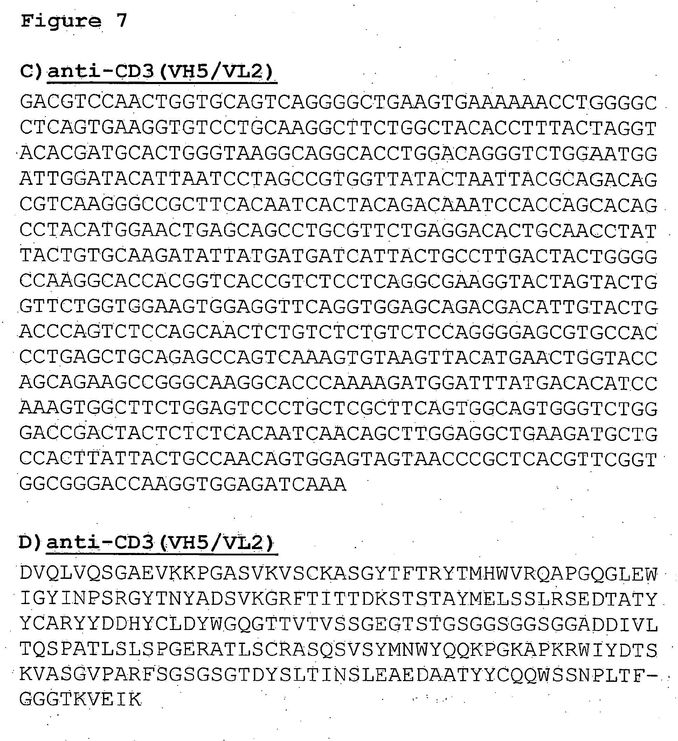

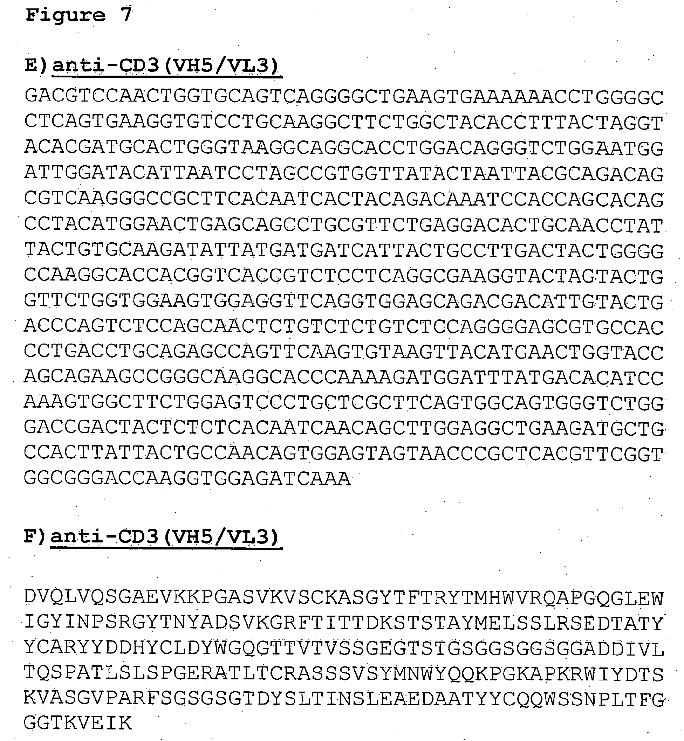

**Figure 7.** DNA and amino acid sequences of deimmunized CD3 specific binding molecules. (A) Nucleotide sequence of anti-CD3 (VH5/VL1) (SEQ ID NO.:28) B) Amino acid sequence of anti-CD3 (VH5/VL1) (SEQ ID NO.: 29) C) Nucleotide sequence of anti-CD3 (VH5/VL2) (SEQ ID NO.:30) D) Amino acid sequence of anti-CD3 (VH5xVL2) (SEQ ID NO.:31) E) Nucleotide sequence of anti-CD3 (VH5/VL3) (SEQ ID NO.:32) F) Amino acid sequence of anti-CD3 (VH5/VL3) (SEQ ID NO.:33).

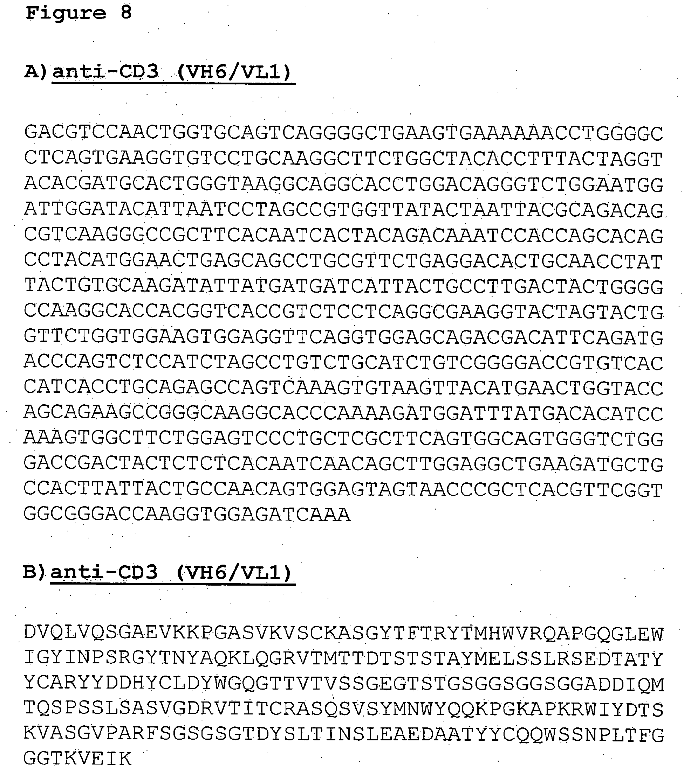

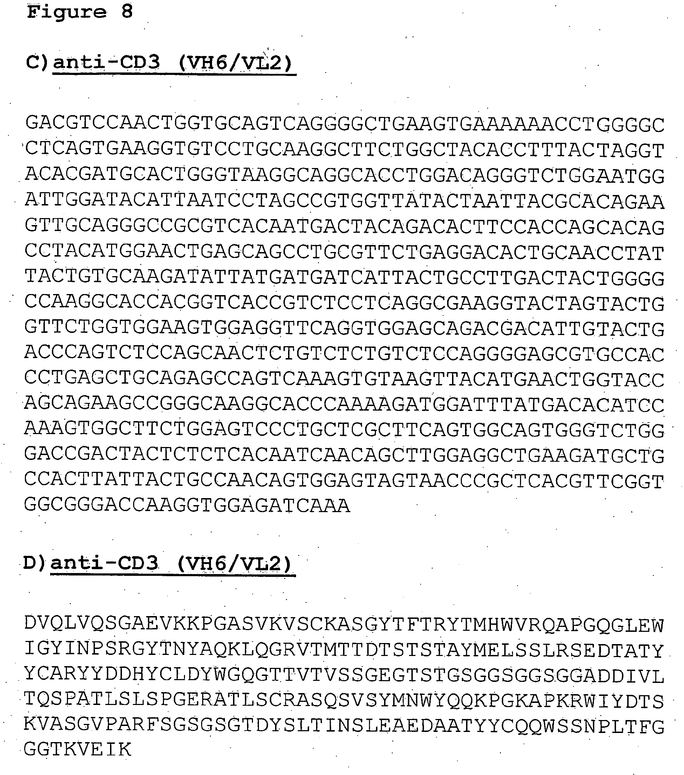

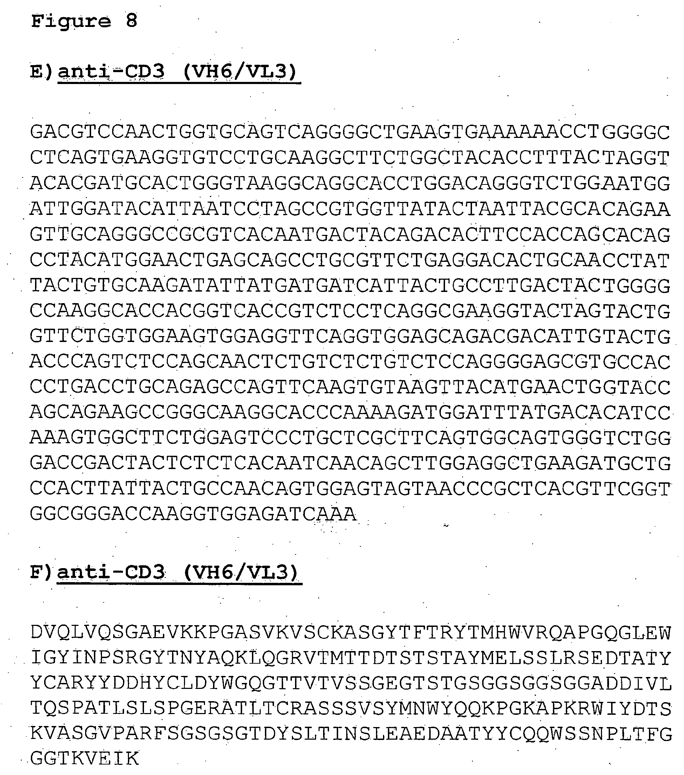

**Figure 8.** DNA and amino acid sequences of deimmunized CD3 specific binding molecules. (A) Nucleotide sequence of anti-CD3 (VH6/VL1) (SEQ ID NO.:34) B) Amino acid sequence of anti-CD3 (VH6/VL1) (SEQ ID NO.: 35) C) Nucleotide sequence of anti-CD3 (VH6/VL2) (SEQ ID NO.:36) D) Amino acid sequence of anti-CD3 (VH6xVL2) (SEQ ID NO.:37) E) Nucleotide sequence of anti-CD3 (VH6/VL3) (SEQ ID NO.:38) F) Amino acid sequence of anti-CD3 (VH6/VL3) (SEQ ID NO.:39).

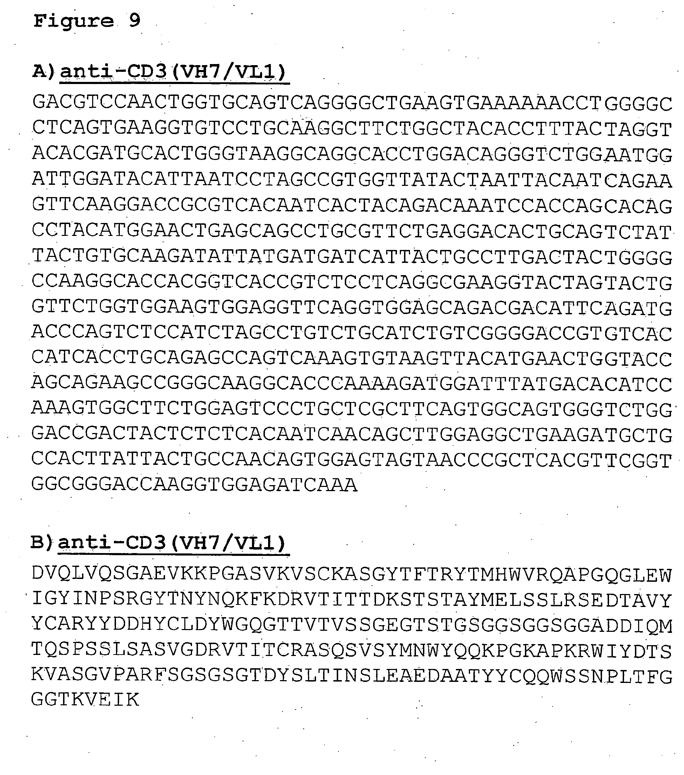

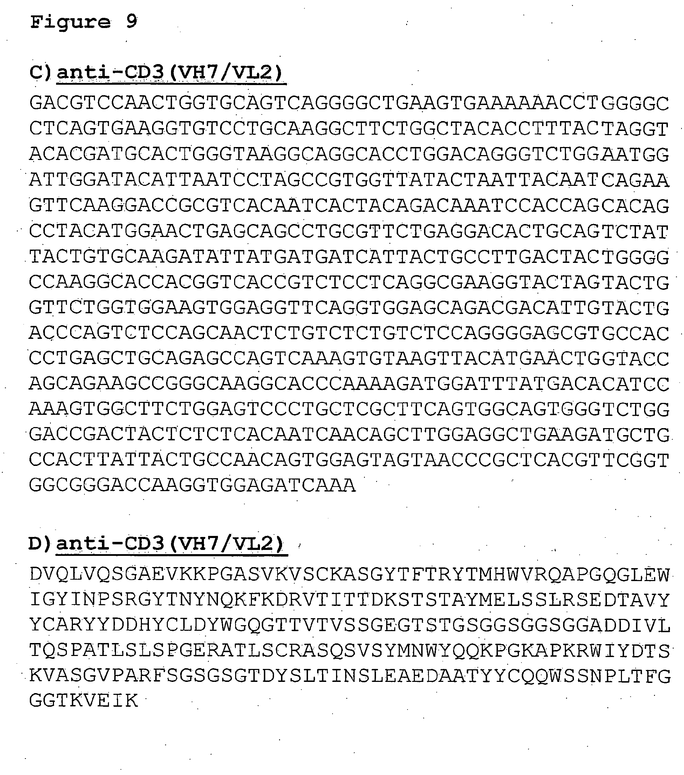

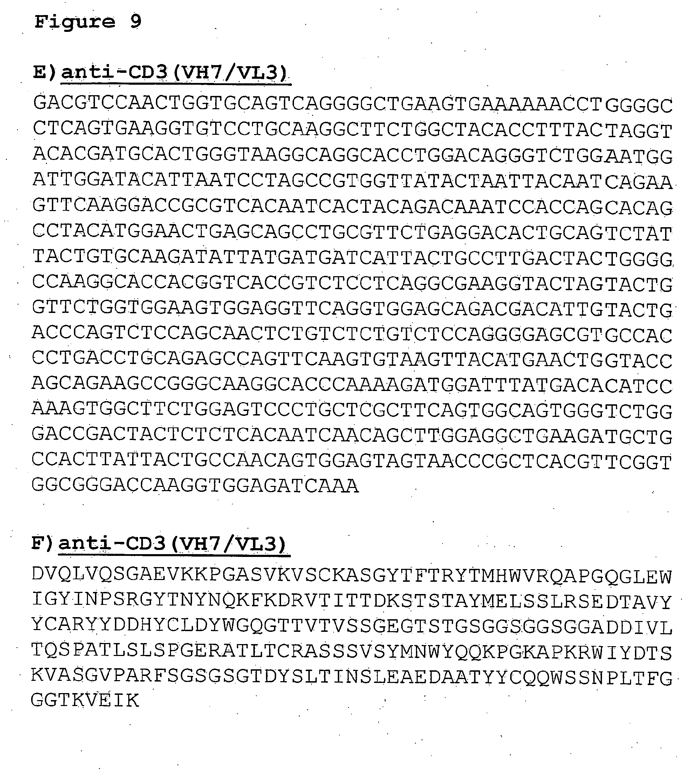

**Figure 9.** DNA and amino acid sequences of deimmunized CD3 specific binding molecules. (A) Nucleotide sequence of anti-CD3 (VH7/VL1) (SEQ ID NO.:40) B) Amino acid sequence of anti-CD3 (VH7/VL1) (SEQ ID NO.: 41) C) Nucleotide sequence of anti-CD3 (VH7/VL2) (SEQ ID NO.:42) D) Amino acid sequence of anti-CD3 (VH7/VL2) (SEQ ID NO.:43) E) Nucleotide sequence of anti-CD3 (VH7/VL3) (SEQ ID NO.:44) F) Amino acid sequence of anti-CD3 (VH7/VL3) (SEQ ID NO.:45).

The following Examples illustrate the invention:

In the following examples a number of single-chain anti-human CD3 antibodies have been engineered to show reduced immunogenicity in man. The different deimmunized anti-human CD3 antibodies comprise 21 combinations of 7 different VH-chains (VH1 (SEQ ID No.:49, 50) VH2 (SEQ ID NO.:51, 52), VH3 (SEQ ID NO.:53, 54), VH4 (SEQ ID NO.:55, 56), VH5 (SEQ ID NO.:57, 58), VH6 (SEQ ID NO.:59, 60) and VH7 (SEQ ID NO.:61, 62)) and 3 different VL (VL1 (SEQ ID NO.:63, 64), VL2 (SEQ ID NO.:65, 66) and VL3 (SEQ ID NO.:67, 68)) regions joined together. The amino acid and nucleic acid sequences of the combinations of the above-mentioned VH and VL regions are shown in Figures 3-9.

**Example 1. Cloning and expression of deimmunized CD3 specific binding molecules**

**1.1.Transfer of cDNA encoding single-chain antibody**

The DNA encoding the anti-CD3 single-chain antibody, which was deimmunized, is referred herein as the anti-CD3 cassette. This anti-CD3 cassette consists of a SGGGGS linker, the anti-CD3 VH region (SEQ ID NO.:70), a 14 amino acid GS linker (VEGGSGGSGGSGGSGGVD linker (SEQ ID NO.:48)), and the anti-CD3 VL region (SEQ ID NO.:72) followed by 6 histidine residues. The afore-mentioned DNA was cloned into the vector p-PCR-Script-Amp SK (+) (Stratagene) at the Srf1 site. The DNA and amino acid sequence of the anti-CD3 cassette is shown in SEQ ID NO.: 1, SEQ ID NO.:2 and Figure 1.

**1.2 Computer analysis of sequences for immunogenic T cell epitopes and design of deimmunized single-chain antibody sequences**

The amino acid sequence of the anti-CD3 cassette (SEQ ID NO.:2) was analyzed by peptide threading program to identify potential T cell epitopes. The program analyzed sequential 13mer peptides through the molecule, assigning each a score for the potential to bind in the binding groove of 18 different human MHC class II allotypes. Deimmunized anti-CD3 VH and VL regions were designed retaining, where required, critical murine amino acids. As generation of the deimmunized sequences required a small number of amino acid substitutions that might affect the binding of the final deimmunized molecules other variant VHs and 2 other VLs were designed. Potential T cell epitopes were also mapped to the linker region between the VH and VL, and substitutions were made to remove these epitopes. SEQ ID NO.3 shows the deimmunized linker sequence and SEQ ID NO.:48 the original linker sequence.

**1.3 Construction of deimmunized single-chain antibody sequences**

The deimmunized versions of the anti-CD3 cassette were constructed by the method of overlapping PCR recombination. The anti-CD3 cassette (SEQ ID NO.:1, 2) in pPCR-S-Amp SK+ was used as the template for mutagenesis to the required deimmunized sequences. Sets of mutagenic primer pairs were synthezised encompassing the regions to be altered. The deimmunized sequences produced, including 7 different VH and 3 different VL regions, were cloned as Not1 to Hind111 fragments into the vector pPCR-S-Amp SK+ and the entire DNA sequence was confirmed by sequencing. The 7 different VH and 3 different VK regions were joined in all combinations (a total of 21), either by PCR or using a unique BstE11 site introduced at the 3' end of the VH region. The entire DNA sequence of each combination was confirmed by sequencing. The different deimmunized VH regions (SEQ ID NO.:50, 52, 54, 56, 58, 60, 62) and VL regions (SEQ ID NO.:64, 66, 68) with the corresponding original non-deimmunized sequences (VH: SEQ ID NO.:70; VL:SEQ ID NO.:72) of the anti-CD3 constructs are summarized in table 1.

Table 1.

| SEQ ID Nos. of deimmunized VH and VL regions | | |
|---|---|---|
| | SEQ ID NO.: | |
| | Nucleic acid | Amino acid |
| Deimmunized VH1 | 49 | 50 |
| Deimmunized VH2 | 51 | 52 |
| Deimmunized VH3 | 53 | 54 |
| Deimmunized VH4 | 55 | 56 |
| Deimmunized VH5 | 57 | 58 |
| Deimmunized VH6 | 59 | 60 |
| Deimmunized VH7 | 61 | 62 |
| VH of the non-deimmunized CD3 | 69 | 70 |
| Deimmunized VL1 | 63 | 64 |
| Deimmunized VL2 | 65 | 66 |
| Deimmunized VL3 | 67 | 68 |

Table 1. (continued)

| SEQ ID Nos. of deimmunized VH and VL regions | | |
|---|---|---|
| | SEQ ID NO.: | |
| | Nucleic acid | Amino acid |
| VL of the non-deimmunized CD3 | 71 | 72 |

**Example 2. T-Cell Proliferation Assay**

Twenty healthy donors were selected for screening in T cell assays based on HLA-DR typing (Table 2). This enables the screening of peptides in the T cell assay against greater than 80% of DR alleles expressed in the world population.

Table 2:

| HLA DR haplotypes of 20 healthy donors used to test the immunogenicity of peptides obtained from deimmunized and non-deimmunized anti-CD3 scAb. | |
|---|---|
| | **HLA DR Allotype** |
| 1 | DRB1*07, DRB1*15, DRB4*01, DRB5 |
| 2 | DRB1*03, DRB1*04, DRB3, DRB4*01 |
| 3 | DRB1*04, DRB1*07 and DRB4*01 |
| 4 | DRB1*07, DRB1*11, DRB4*01 |
| 5 | DRB1*04, DRB1*07, DRB4*01 |
| 6 | DRB1*01, DRB1*04, DRB4*01 |
| 7 | DRB1*03, DRB1*07, DRB3, DRB4*01 |
| 8 | DRB1*07, DRB1*11, DRB3, DRB4*01 |
| 9 | DRB1*12. DRB1*15, DRB3, DRB5 |
| 10 | DRB1*01, DRB1*09, DRB4*01 |
| 11 | DRB1*03, DRB1*15, DRB3, DRB5 |
| 12 | DRB1*10, DRB1*13, DRB3 |
| 13 | DRB1*03, DRB1*15, DRB3, DRB5 |
| 14 | DRB1*04, DRb1*15, DRB4*01, DRB5 |
| 15 | DRB1*04, DRB1*13, DRB3, DRB4*01 |
| 16 | DRB1*01, DRB1*13, DRB3 |
| 17 | DRB1*01, DRB1*04, DRB4*01 |
| 18 | DRB1*07, DRB1*13, DRB3, DRB4*01 |
| 19 | DRB1*07, DRB1*16, DRB4*01, DRB5 |
| 20 | DRB1*04, DRB1*15, DRB4*01, DRB5 |

Peptides were obtained from Pepscan (Netherlands) at a purity of greater than 90%. Peripheral blood mononuclear cells (PBMC) from the 20 selected healthy donors were used to screen individual peptides in triplicate wells at 1 and 5 μM. Two positive control peptides (C32 and C49) and keyhole limpet hemocyanin (KLH) were included in the assay. After 7 days incubation of cells and peptides, an 18 hour pulse with 3H-thymidine at 1μCi/well was used to assess T cell proliferation. These data are expressed as stimulation index where:

$$\text{Stimulation Index} = \text{CPM of test peptide} / \text{CPM of untreated control}$$

A T cell epitope is defined as a peptide giving a stimulation index (SI) greater than 2.

The results from two independent runs indicated that 5 of the 22 MHC binding peptides in the non-deimmunized anti-CD3 sequence had the capacity to induce human T cell proliferation (SI>2). In contrast, none of the deimmunized molecules induced T cell proliferation. Table 3 summarizes the T cell proliferation assay results showing Mean SI values of 2 independent runs.

The data also showed a specific peptide dependent effect whereby each of the non-deimmunized binding molecules showed SI's > 2 in only one of the two concentrations (1µm or 5µm) used. The difference in response at different concentrations is explained by the fact that individual peptides will have optimum concentrations at which they induced T cell proliferation. If this concentration is exceeded, then proliferation can drop off (high peptide concentrations can have an inhibitory effect on T cell proliferation). This explains why, in some instances, proliferation is seen at the lower concentration and not at the higher. From experience, T cell proliferation will be observed at one or two of the peptide concentrations used if a peptide contains a T cell epitope. These data demonstrated that deimmunization had successfully removed T cell epitopes from anti CD3 (VH5/VL2) (SEQ ID NO.:31) and anti CD3 (VH7/VL2) (SEQ ID NO.:43). The fact that about 75% of MHC binding peptides from the non-deimmunized anti-CD3 sequence did not induce T cell proliferation can be explained either by tolerance of the human immune system to these peptides or an inability of the human T cell repertoire to recognize these particular peptides.

Table 3:

| **Summary of data comparing positive (SI>2) mouse peptides and corresponding deimmunized peptides.** | | | | |
|---|---|---|---|---|
| | | | ***Non-deimmunized Anti-CD3*** | ***Deimmunized Anti-CD3*** |
| **Peptide** | **Allotype** | **Concentration** | **Mean SI** | **Mean SI** |
| **Region** | | (µM) | | |
| 6-20 | 5 | 5 | 2.51 | 0.77 |
| | | | | |
| 74-86 | 5 | 1 | 2.52 | 0.97 |
| | | | | 0.96 |
| 90-102 | 5 | 5 | 2.21 | 0.56 |
| | | | | 1.38 |
| 90-102 | 6 | 5 | 2.24 | 0.90 |
| | | | | 0.82 |
| 90-102 | 11 | 5 | 2.23 | 0.83 |
| | | | | 0.78 |
| 162-174 | 5 | 1 | 3.82 | 0.59 |
| | | | | |
| 216-230 | 10 | 1 | 2.12 | 1.03 |

**Claims**

1. CD3 specific binding molecules selected from the group consisting of

   (a) a polypeptide having the amino acid sequence of SEQ ID NO.:5, 7, 9, 11,13,15,17,19,21,23,25,27,29,31,33,35,37,39,41,43 and 45,
   (b) a polypeptide encoded by a nucleic acid sequence selected from the group consisting of SEQ ID NO: 4, 6, 8, 10, 12, 14, 16, 18, 20, 22, 24, 26, 28, 30, 32, 34, 36, 38, 40, 42 and 44;
   (c) a polypeptide encoded by a nucleic acid sequence which is degenerated as a result of the genetic code to a nucleic acid sequence of (b).

2. A nucleic acid sequence encoding a CD3 specific binding molecule according to claim 1.

3. The nucleic acid sequence according to claim 2 selected from the group consisting of SEQ ID NO.:4, 6, 8, 10, 12, 14, 16, 18, 20, 22, 24, 26, 28, 30, 32, 34, 36, 38, 40, 42 and 44.

4. A vector comprising a nucleic acid sequence according to claim 2 or 3.

5. The vector of claim 4, which further comprises a nucleic acid sequence which is a regulatory sequence operable linked to said nucleic acid sequence according to claim 2 or 3.

6. The vector of claim 4 or 5, wherein the vector is an expression vector.

7. A host transformed or transfected with a vector according to any of claims 4 to 6.

8. A process for the production of a CD3 specific binding molecule according to claim 1 said process comprising culturing a host of claim 7 under conditions allowing the expression of the CD3 specific binding molecule and recovering the produced CD3 specific binding molecule from the culture.

9. A composition comprising a CD3 specific binding molecule according to claim 1 or as produced by the process of claim 8, a nucleic acid molecule of claim 2 or 3, a vector of any one of claims 4 to 6 or a host of claim 7 and, optionally, a proteinaceous compound capable of providing an activation signal for immune effector cells.

10. The composition of claim 9, which is a pharmaceutical composition further comprising, optionally, suitable formulations of carrier, stabilizers and/or excipients.

11. The composition of claim 9, which is a diagnostic composition further comprising, optionally, means and methods for detection.

12. Use of a CD3 specific binding molecule according to any of claim 1 or as produced by the process of claim 8, a nucleic acid molecule of claim 2 or 3, a vector of any one of claims 4 to 6 or a host of claim 7 for the preparation of a pharmaceutical composition for the prevention, treatment or amelioration of a proliferative disease, a tumorous disease, an inflammatory disease, an immunological disorder, an autoimmune disease, an infectious disease, viral disease, allergic reactions, parasitic reactions, graft-versus-host diseases or host-versus-graft diseases.

**13.** A kit comprising a CD3 specific binding molecule according to claim 1 or as produced by the process of claim 8, a nucleic acid molecule of claim 2 or 3, a vector of any one of claims 4 to 6 or a host of claim 7.

# Figure 1

## Anti-CD3 WT

GATATCAAACTGCAGCAGTCAGGGGCTGAACTGGCAAGACCTGGGGCCTCAGTGAAGATGTCCT
GCAAGACTTCTGGCTACACCTTTACTAGGTACACGATGCACTGGGTAAAACAGAGGCCTGGACA
GGGTCTGGAATGGATTGGATACATTAATCCTAGCCGTGGTTATACTAATTACAATCAGAAGTTC
AAGGACAAGGCCACATTGACTACAGACAAATCCTCCAGCACAGCCTACATGCAACTGAGCAGCC
TGACATCTGAGGACTCTGCAGTCTATTACTGTGCAAGATATTATGATGATCATTACTGCCTTGA
CTACTGGGGCCAAGGCACCACTCTCACAGTCTCCTCAGTCGAAGGTGGAAGTGGAGGTTCTGGT
GGAAGTGGAGGTTCAGGTGGAGTCGACGACATTCAGCTGACCCAGTCTCCAGCAATCATGTCTG
CATCTCCAGGGGAGAAGGTCACCATGACCTGCAGAGCCAGTTCAAGTGTAAGTTACATGAACTG
GTACCAGCAGAAGTCAGGCACCTCCCCCAAAAGATGGATTTATGACACATCCAAAGTGGCTTCT
GGAGTCCCTTATCGCTTCAGTGGCAGTGGGTCTGGGACCTCATACTCTCTCACAATCAGCAGCA
TGGAGGCTGAAGATGCTGCCACTTATTACTGCCAACAGTGGAGTAGTAACCCGCTCACGTTCGG
TGCTGGGACCAAGCTGGAGCTGAAA

## AA Sequence

DIKLQQSGAELARPGASVKMSCKTSGYTFTRYTMHWVKQRPGQGLEWIGYINPSRGYTNYNQKF
KDKATLTTDKSSSTAYMQLSSLTSEDSAVYYCARYYDDHYCLDYWGQGTTLTVSSVEGGSGGSG
GSGGSGGVDDIQLTQSPAIMSASPGEKVTMTCRASSSVSYMNWYQQKSGTSPKRWIYDTSKVAS
GVPYRFSGSGSGTSYSLTISSMEAEDAATYYCQQWSSNPLTFGAGTKLELK

# Fig. 2 A

**VH1**

DVQLVQSGAEVKKPGASVKVSCKASGYTATRYTMHWVRQAPGQGLEWIGYINPSR
GYTNYADSVKGRFTITTDKSTSTAYMELSSLRSEDTATYYCARYYDDHYCLDYWG
QGTTVTVSS

**VH2**

DVQLVQSGAEVKKPGASVKVSCKASGYTATRYTMHWVRQAPGQGLEWIGYINPSR
GYTNYAQKLQGRVTMTTDTSTSTAYMELSSLRSEDTATYYCARYYDDHYCLDYWG
QGTTVTVSS

**VH3**

DVQLVQSGAEVKKPGASVKVSCKASGYTATRYTMHWVRQAPGQGLEWIGYINPSR
GYTNYAQKLQGRVTMTTDTSTSTAYLQMNSLKTEDTAVYYCARYYDDHYCLDYWG
QGTTVTVSS

# Fig. 2 A (cont.)

**VH4**

DVQLVQSGAEVKKPGASVKVSCKASGYTATRYTMHWVRQAPGQGLEWIGYINPSR
GYTNYADSVKGRFTITTDKSTSTAYLQMNSLKTEDTAVYYCARYYDDHYCLDYWG
QGTTVTVSS

**VH5**

DVQLVQSGAEVKKPGASVKVSCKASGYTFTRYTMHWVRQAPGQGLEWIGYINPSR
GYTNYADSVKGRFTITTDKSTSTAYMELSSLRSEDTATYYCARYYDDHYCLDYWG
QGTTVTVSS

**VH6**

DVQLVQSGAEVKKPGASVKVSCKASGYTFTRYTMHWVRQAPGQGLEWIGYINPSR
GYTNYAQKLQGRVTMTTDTSTSTAYMELSSLRSEDTATYYCARYYDDHYCLDYWG
QGTTVTVSS

**VH7**

DVQLVQSGAEVKKPGASVKVSCKASGYTFTRYTMHWVRQAPGQGLEWIGYINPSR
GYTNYNQKFKDRVTITTDKSTSTAYMELSSLRSEDTAVYYCARYYDDHYCLDYWG
QGTTVTVSS

# Fig. 2 A (cont.)

**VL1**

DIQMTQSPSSLSASVGDRVTITCRASQSVSYMNWYQQKPGKAPKRWIYDTSKVAS
GVPARFSGSGSGTDYSLTINSLEAEDAATYYCQQWSSNPLTFGGGTKVEIK

**VL2**

DIVLTQSPATLSLSPGERATLSCRASQSVSYMNWYQQKPGKAPKRWIYDTSKVAS
GVPARFSGSGSGTDYSLTINSLEAEDAATYYCQQWSSNPLTFGGGTKVEIK

**VL3**

DIVLTQSPATLSLSPGERATLTCRASSSVSYMNWYQQKPGKAPKRWIYDTSKVAS
GVPARFSGSGSGTDYSLTINSLEAEDAATYYCQQWSSNPLTFGGGTKVEIK

# Fig. 2 B

**VH1**

GACGTCCAACTGGTGCAGTCAGGGGCTGAAGTGAAAAAACCTGGGGCCTCAGTGA
AGGTGTCCTGCAAGGCTTCTGGCTACACCGCTACTAGGTACACGATGCACTGGGT
AAGGCAGGCACCTGGACAGGGTCTGGAATGGATTGGATACATTAATCCTAGCCGT
GGTTATACTAATTACGCAGACAGCGTCAAGGGCCGCTTCACAATCACTACAGACA
AATCCACCAGCACAGCCTACATGGAACTGAGCAGCCTGCGTTCTGAGGACACTGC
AACCTATTACTGTGCAAGATATTATGATGATCATTACTGCCTTGACTACTGGGGC
CAAGGGACCACGGTCACCGTCTCCTCA

**VH2**

GACGTCCAACTGGTGCAGTCAGGGGCTGAAGTGAAAAAACCTGGGGCCTCAGTGA
AGGTGTCCTGCAAGGCTTCTGGCTACACCGCTACTAGGTACACGATGCACTGGGT
AAGGCAGGCACCTGGACAGGGTCTGGAATGGATTGGATACATTAATCCTAGCCGT
GGTTATACTAATTACGCACAGAAGTTGCAGGGCCGCGTCACAATGACTACAGACA
CTTCCACCAGCACAGCCTACATGGAACTGAGCAGCCTGCGTTCTGAGGACACTGC
AACCTATTACTGTGCAAGATATTATGATGATCATTACTGCCTTGACTACTGGGGC
CAAGGCACCACGGTCACCGTCTCCTCA

# Fig. 2 B (cont.)

**VH3**

GACGTCCAACTGGTGCAGTCAGGGGCTGAAGTGAAAAAACCTGGGGCCTCAGTGA
AGGTGTCCTGCAAGGCTTCTGGCTACACCGCTACTAGGTACACGATGCACTGGGT
AAGGCAGGCACCTGGACAGGGTCTGGAATGGATTGGATACATTAATCCTAGCCGT
GGTTATACTAATTACGCACAGAAGTTGCAGGGCCGCGTCACAATGACTACAGACA
CTTCCACCAGCACAGCCTACCTGCAAATGAACAGCCTGAAAACTGAGGACACTGC
AGTCTATTACTGTGCAAGATATTATGATGATCATTACTGCCTTGACTACTGGGGC
CAAGGCACCACGGTCACCGTCTCCTCA

**VH4**

GACGTCCAACTGGTGCAGTCAGGGGCTGAAGTGAAAAAACCTGGGGCCTCAGTGA
AGGTGTCCTGCAAGGCTTCTGGCTACACCGCTACTAGGTACACGATGCACTGGGT
AAGGCAGGCACCTGGACAGGGTCTGGAATGGATTGGATACATTAATCCTAGCCGT
GGTTATACTAATTACGCAGACAGCGTCAAGGGCCGCTTCACAATCACTACAGACA
AATCCACCAGCACAGCCTACCTGCAAATGAACAGCCTGAAAACTGAGGACACTGC
AGTCTATTACTGTGCAAGATATTATGATGATCATTACTGCCTTGACTACTGGGGC
CAAGGCACCACGGTCACCGTCTCCTCA

# Fig. 2 B (cont.)

**VH5**

GACGTCCAACTGGTGCAGTCAGGGGCTGAAGTGAAAAAACCTGGGGCCTCAGTGA
AGGTGTCCTGCAAGGCTTCTGGCTACACCTTTACTAGGTACACGATGCACTGGGT
AAGGCAGGCACCTGGACAGGGTCTGGAATGGATTGGATACATTAATCCTAGCCGT
GGTTATACTAATTACGCAGACAGCGTCAAGGGCCGCTTCACAATCACTACAGACA
AATCCACCAGCACAGCCTACATGGAACTGAGCAGCCTGCGTTCTGAGGACACTGC
AACCTATTACTGTGCAAGATATTATGATGATCATTACTGCCTTGACTACTGGGGC
CAAGGCACCACGGTCACCGTCTCCTCA

**VH6**

GACGTCCAACTGGTGCAGTCAGGGGCTGAAGTGAAAAAACCTGGGGCCTCAGTGA
AGGTGTCCTGCAAGGCTTCTGGCTACACCTTTACTAGGTACACGATGCACTGGGT
AAGGCAGGCACCTGGACAGGGTCTGGAATGGATTGGATACATTAATCCTAGCCGT
GGTTATACTAATTACGCACAGAAGTTGCAGGGCCGCGTCACAATGACTACAGACA
CTTCCACCAGCACAGCCTACATGGAACTGAGCAGCCTGCGTTCTGAGGACACTGC
AACCTATTACTGTGCAAGATATTATGATGATCATTACTGCCTTGACTACTGGGGC
CAAGGCACCACGGTCACCGTCTCCTCA

# Fig. 2 B (cont.)

**VH7**

GACGTCCAACTGGTGCAGTCAGGGGCTGAAGTGAAAAAACCTGGGGCCTCAGTGA
AGGTGTCCTGCAAGGCTTCTGGCTACACCTTTACTAGGTACACGATGCACTGGGT
AAGGCAGGCACCTGGACAGGGTCTGGAATGGATTGGATACATTAATCCTAGCCGT
GGTTATACTAATTACAATCAGAAGTTCAAGGACCGCGTCACAATCACTACAGACA
AATCCACCAGCACAGCCTACATGGAACTGAGCAGCCTGCGTTCTGAGGACACTGC
AGTCTATTACTGTGCAAGATATTATGATGATCATTACTGCCTTGACTACTGGGGC
CAAGGCACCACGGTCACCGTCTCCTCA

# Fig. 2 B (cont.)

**VL1**

GACATTCAGATGACCCAGTCTCCATCTAGCCTGTCTGCATCTGTCGGGGACCGTG
TCACCATCACCTGCAGAGCCAGTCAAAGTGTAAGTTACATGAACTGGTACCAGCA
GAAGCCGGGCAAGGCACCCAAAAGATGGATTTATGACACATCCAAAGTGGCTTCT
GGAGTCCCTGCTCGCTTCAGTGGCAGTGGGTCTGGGACCGACTACTCTCTCACAA
TCAACAGCTTGGAGGCTGAAGATGCTGCCACTTATTACTGCCAACAGTGGAGTAG
TAACCCGCTCACGTTCGGTGGCGGGACCAAGGTGGAGATCAAA

**VL2**

GACATTGTACTGACCCAGTCTCCAGCAACTCTGTCTCTGTCTCCAGGGGAGCGTG
CCACCCTGAGCTGCAGAGCCAGTCAAAGTGTAAGTTACATGAACTGGTACCAGCA
GAAGCCGGGCAAGGCACCCAAAAGATGGATTTATGACACATCCAAAGTGGCTTCT
GGAGTCCCTGCTCGCTTCAGTGGCAGTGGGTCTGGGACCGACTACTCTCTCACAA
TCAACAGCTTGGAGGCTGAAGATGCTGCCACTTATTACTGCCAACAGTGGAGTAG
TAACCCGCTCACGTTCGGTGGCGGGACCAAGGTGGAGATCAAA

# Fig. 2 B (cont.)

**VL3**

GACATTGTACTGACCCAGTCTCCAGCAACTCTGTCTCTGTCTCCAGGGGAGCGTG
CCACCCTGACCTGCAGAGCCAGTTCAAGTGTAAGTTACATGAACTGGTACCAGCA
GAAGCCGGGCAAGGCACCCAAAAGATGGATTTATGACACATCCAAAGTGGCTTCT
GGAGTCCCTGCTCGCTTCAGTGGCAGTGGGTCTGGGACCGACTACTCTCTCACAA
TCAACAGCTTGGAGGCTGAAGATGCTGCCACTTATTACTGCCAACAGTGGAGTAG
TAACCCGCTCACGTTCGGTGGCGGGACCAAGGTGGAGATCAAA

**Figure 3**

**A) anti-CD3 (VH1/VL1)**

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCAGACAG
CGTCAAGGGCCGCTTCACAATCACTACAGACAAATCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGGACCACGGTCACCGTCTCCTCAGGCGAAGGTACTAGTACTG
GTTCTGGTGGAAGTGGAGGTTCAGGTGGAGCAGACGACATTCAGATG
ACCCAGTCTCCATCTAGCCTGTCTGCATCTGTCGGGGACCGTGTCAC
CATCACCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**B) anti-CD3 (VH1/VL1)**

DVQLVQSGAEVKKPGASVKVSCKASGYTATRYTMHWVRQAPGQGLEW
IGYINPSRGYTNYADSVKGRFTITTDKSTSTAYMELSSLRSEDTATY
YCARYYDDHYCLDYWGQGTTVTVSSGEGTSTGSGGSGGSGGADDIQM
TQSPSSLSASVGDRVTITCRASQSVSYMNWYQQKPGKAPKRWIYDTS
KVASGVPARFSGSGSGTDYSLTINSLEAEDAATYYCQQWSSNPLTF-
GGGTKVEIK

**Figure 3**

**C) anti-CD3 (VH1/VL2)**

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCAGACAG
CGTCAAGGGCCGCTTCACAATCACTACAGACAAATCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGGACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGAGCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**D) anti-CD3 (VH1/VL2)**

DVQLVQSGAEVKKPGASVKVSCKASGYTATRYTMHWVRQAPGQGLEW
IGYINPSRGYTNYADSVKGRFTITTDKSTSTAYMELSSLRSEDTATY
YCARYYDDHYCLDYWGQGTTVTVSSGEGTSTGSGGSGGSGGADDIVL
TQSPATLSLSPGERATLSCRASQSVSYMNWYQQKPGKAPKRWIYDTS
KVASGVPARFSGSGSGTDYSLTINSLEAEDAATYYCQQWSSNPLTF-
GGGTKVEIK

**Figure 3**

**E) anti-CD3 (VH1/VL3)**

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCAGACAG
CGTCAAGGGCCGCTTCACAATCACTACAGACAAATCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGGACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGAGCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**F) anti-CD3 (VH1/VL3)**

DVQLVQSGAEVKKPGASVKVSCKASGYTATRYTMHWVRQAPGQGLEW
IGYINPSRGYTNYADSVKGRFTITTDKSTSTAYMELSSLRSEDTATY
YCARYYDDHYCLDYWGQGTTVTVSSGEGTSTGSGGSGGSGGADDIVL
TQSPATLSLSPGERATLTCRASSSVSYMNWYQQKPGKAPKRWIYDTS
KVASGVPARFSGSGSGTDYSLTINSLEAEDAATYYCQQWSSNPLTFG
GGTKVEIK

**Figure 4**

**A) anti-CD3 (VH2/VL1)**

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCACAGAA
GTTGCAGGGCCGCGTCACAATGACTACAGACACTTCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTCAGATG
ACCCAGTCTCCATCTAGCCTGTCTGCATCTGTCGGGGACCGTGTCAC
CATCACCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**B) anti-CD3 (VH2/VL1)**

DVQLVQSGAEVKKPGASVKVSCKASGYTATRYTMHWVRQAPGQGLEW
IGYINPSRGYTNYAQKLQGRVTMTTDTSTSTAYMELSSLRSEDTATY
YCARYYDDHYCLDYWGQGTTVTVSSGEGTSTGSGGSGGSGGADDIQM
TQSPSSLSASVGDRVTITCRASQSVSYMNWYQQKPGKAPKRWIYDTS
KVASGVPARFSGSGSGTDYSLTINSLEAEDAATYYCQQWSSNPLTF-
GGGTKVEIK

**Figure 4**

**C) anti-CD3 (VH2/VL2)**

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCACAGAA
GTTGCAGGGCCGCGTCACAATGACTACAGACACTTCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGAGCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**D) anti-CD3 (VH2/VL2)**

DVQLVQSGAEVKKPGASVKVSCKASGYTATRYTMHWVRQAPGQGLEW
IGYINPSRGYTNYAQKLQGRVTMTTDTSTSTAYMELSSLRSEDTATY
YCARYYDDHYCLDYWGQGTTVTVSSGEGTSTGSGGSGGSGGADDIVL
TQSPATLSLSPGERATLSCRASQSVSYMNWYQQKPGKAPKRWIYDTS
KVASGVPARFSGSGSGTDYSLTINSLEAEDAATYYCQQWSSNPLTF-
GGGTKVEIK

**Figure 4**

**E) anti-CD3 (VH2/VL3)**

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCACAGAA
GTTGCAGGGCCGCGTCACAATGACTACAGACACTTCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGACCTGCAGAGCCAGTTCAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**F) anti-CD3 (VH2/VL3)**

DVQLVQSGAEVKKPGASVKVSCKASGYTATRYTMHWVRQAPGQGLEW
IGYINPSRGYTNYAQKLQGRVTMTTDTSTSTAYMELSSLRSEDTATY
YCARYYDDHYCLDYWGQGTTVTVSSGEGTSTGSGGSGGSGGADDIVL
TQSPATLSLSPGERATLTCRASSSVSYMNWYQQKPGKAPKRWIYDTS
KVASGVPARFSGSGSGTDYSLTINSLEAEDAATYYCQQWSSNPLTFG
GGTKVEIK

**Figure 5**

**A) anti-CD3 (VH3/VL1)**

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCACAGAA
GTTGCAGGGCCGCGTCACAATGACTACAGACACTTCCACCAGCACAG
CCTACCTGCAAATGAACAGCCTGAAAACTGAGGACACTGCAGTCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTCAGATG
ACCCAGTCTCCATCTAGCCTGTCTGCATCTGTCGGGGACCGTGTCAC
CATCACCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**B) anti-CD3 (VH3/VL1)**

DVQLVQSGAEVKKPGASVKVSCKASGYTATRYTMHWVRQAPGQGLEW
IGYINPSRGYTNYAQKLQGRVTMTTDTSTSTAYLQMNSLKTEDTAVY
YCARYYDDHYCLDYWGQGTTVTVSSGEGTSTGSGGSGGSGGADDIQM
TQSPSSLSASVGDRVTITCRASQSVSYMNWYQQKPGKAPKRWIYDTS
KVASGVPARFSGSGSGTDYSLTINSLEAEDAATYYCQQWSSNPLTFG
GGTKVEIK

**Figure 5**

**C) anti-CD3(VH3/VL2)**

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCACAGAA
GTTGCAGGGCCGCGTCACAATGACTACAGACACTTCCACCAGCACAG
CCTACCTGCAAATGAACAGCCTGAAAACTGAGGACACTGCAGTCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGAGCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**D) anti-CD3(VH3/VL2)**

DVQLVQSGAEVKKPGASVKVSCKASGYTATRYTMHWVRQAPGQGLEW
IGYINPSRGYTNYAQKLQGRVTMTTDTSTSTAYLQMNSLKTEDTAVY
YCARYYDDHYCLDYWGQGTTVTVSSGEGTSTGSGGSGGSGGADDIVL
TQSPATLSLSPGERATLSCRASQSVSYMNWYQQKPGKAPKRWIYDTS
KVASGVPARFSGSGSGTDYSLTINSLEAEDAATYYCQQWSSNPLTF-
GGGTKVEIK

**Figure 5**

**E) anti-CD3 (VH3/VL3)**

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCACAGAA
GTTGCAGGGCCGCGTCACAATGACTACAGACACTTCCACCAGCACAG
CCTACCTGCAAATGAACAGCCTGAAAACTGAGGACACTGCAGTCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGACCTGCAGAGCCAGTTCAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**F) anti-CD3 (VH3/VL3)**

DVQLVQSGAEVKKPGASVKVSCKASGYTATRYTMHWVRQAPGQGLEW
IGYINPSRGYTNYAQKLQGRVTMTTDTSTSTAYLQMNSLKTEDTAVY
YCARYYDDHYCLDYWGQGTTVTVSSGEGTSTGSGGSGGSGGADDIVL
TQSPATLSLSPGERATLTCRASSSVSYMNWYQQKPGKAPKRWIYDTS
KVASGVPARFSGSGSGTDYSLTINSLEAEDAATYYCQQWSSNPLTFG
GGTKVEIK

# Figure 6

## A) anti-CD3 (VH4/VL1)

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCAGACAG
CGTCAAGGGCCGCTTCACAATCACTACAGACAAATCCACCAGCACAG
CCTACCTGCAAATGAACAGCCTGAAAACTGAGGACACTGCAGTCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTCAGATG
ACCCAGTCTCCATCTAGCCTGTCTGCATCTGTCGGGGACCGTGTCAC
CATCACCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

## B) anti-CD3 (VH4/VL1)

DVQLVQSGAEVKKPGASVKVSCKASGYTATRYTMHWVRQAPGQGLEW
IGYINPSRGYTNYADSVKGRFTITTDKSTSTAYLQMNSLKTEDTAVY
YCARYYDDHYCLDYWGQGTTVTVSSGEGTSTGSGGSGGSGGADDIQM
TQSPSSLSASVGDRVTITCRASQSVSYMNWYQQKPGKAPKRWIYDTS
KVASGVPARFSGSGSGTDYSLTINSLEAEDAATYYCQQWSSNPLTFG
GGTKVEIK

**Figure 6**

**C) anti-CD3 (VH4/VL2)**

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCAGACAG
CGTCAAGGGCCGCTTCACAATCACTACAGACAAATCCACCAGCACAG
CCTACCTGCAAATGAACAGCCTGAAAACTGAGGACACTGCAGTCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGAGCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**D) anti-CD3 (VH4/VL2)**

DVQLVQSGAEVKKPGASVKVSCKASGYTATRYTMHWVRQAPGQGLEW
IGYINPSRGYTNYADSVKGRFTITTDKSTSTAYLQMNSLKTEDTAVY
YCARYYDDHYCLDYWGQGTTVTVSSGEGTSTGSGGSGGSGGADDIVL
TQSPATLSLSPGERATLSCRASQSVSYMNWYQQKPGKAPKRWIYDTS
KVASGVPARFSGSGSGTDYSLTINSLEAEDAATYYCQQWSSNPLTFG
GGTKVEIK

**Figure 6**

**E) anti-CD3 (VH4/VL3)**

GACGTCCAACTGGTGCAGTCAGGGGCTGAAGTGAAAAAACCTGGGGC
CTCAGTGAAGGTGTCCTGCAAGGCTTCTGGCTACACCGCTACTAGGT
ACACGATGCACTGGGTAAGGCAGGCACCTGGACAGGGTCTGGAATGG
ATTGGATACATTAATCCTAGCCGTGGTTATACTAATTACGCAGACAG
CGTCAAGGGCCGCTTCACAATCACTACAGACAAATCCACCAGCACAG
CCTACCTGCAAATGAACAGCCTGAAAACTGAGGACACTGCAGTCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGACCTGCAGAGCCAGTTCAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**F) anti-CD3 (VH4/VL3)**

DVQLVQSGAEVKKPGASVKVSCKASGYTATRYTMHWVRQAPGQGLEW
IGYINPSRGYTNYADSVKGRFTITTDKSTSTAYLQMNSLKTEDTAVY
YCARYYDDHYCLDYWGQGTTVTVSSGEGTSTGSGGSGGSGGADDIVL
TQSPATLSLSPGERATLTCRASSSVSYMNWYQQKPGKAPKRWIYDTS
KVASGVPARFSGSGSGTDYSLTINSLEAEDAATYYCQQWSSNPLTFG
GGTKVEIK

**Figure 7**

**A) CD3 (VH5/VL1)**

GACGTCCAACTGGTGCAGTCAGGGGCTGAAGTGAAAAAACCTGGGGC
CTCAGTGAAGGTGTCCTGCAAGGCTTCTGGCTACACCTTTACTAGGT
ACACGATGCACTGGGTAAGGCAGGCACCTGGACAGGGTCTGGAATGG
ATTGGATACATTAATCCTAGCCGTGGTTATACTAATTACGCAGACAG
CGTCAAGGGCCGCTTCACAATCACTACAGACAAATCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTCAGATG
ACCCAGTCTCCATCTAGCCTGTCTGCATCTGTCGGGGACCGTGTCAC
CATCACCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**B) CD3 (VH5/VL1)**

DVQLVQSGAEVKKPGASVKVSCKASGYTFTRYTMHWVRQAPGQGLEW
IGYINPSRGYTNYADSVKGRFTITTDKSTSTAYMELSSLRSEDTATY
YCARYYDDHYCLDYWGQGTTVTVSSGEGTSTGSGGSGGSGGADDIQM
TQSPSSLSASVGDRVTITCRASQSVSYMNWYQQKPGKAPKRWIYDTS
KVASGVPARFSGSGSGTDYSLTINSLEAEDAATYYCQQWSSNPLTF-
GGGTKVEIK

**Figure 7**

**C) anti-CD3 (VH5/VL2)**

GACGTCCAACTGGTGCAGTCAGGGGCTGAAGTGAAAAAACCTGGGGC
CTCAGTGAAGGTGTCCTGCAAGGCTTCTGGCTACACCTTTACTAGGT
ACACGATGCACTGGGTAAGGCAGGCACCTGGACAGGGTCTGGAATGG
ATTGGATACATTAATCCTAGCCGTGGTTATACTAATTACGCAGACAG
CGTCAAGGGCCGCTTCACAATCACTACAGACAAATCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGAGCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**D) anti-CD3 (VH5/VL2)**

DVQLVQSGAEVKKPGASVKVSCKASGYTFTRYTMHWVRQAPGQGLEW
IGYINPSRGYTNYADSVKGRFTITTDKSTSTAYMELSSLRSEDTATY
YCARYYDDHYCLDYWGQGTTVTVSSGEGTSTGSGGSGGSGGADDIVL
TQSPATLSLSPGERATLSCRASQSVSYMNWYQQKPGKAPKRWIYDTS
KVASGVPARFSGSGSGTDYSLTINSLEAEDAATYYCQQWSSNPLTF-
GGGTKVEIK

**Figure 7**

**E) anti-CD3 (VH5/VL3)**

GACGTCCAACTGGTGCAGTCAGGGGCTGAAGTGAAAAAACCTGGGGC
CTCAGTGAAGGTGTCCTGCAAGGCTTCTGGCTACACCTTTACTAGGT
ACACGATGCACTGGGTAAGGCAGGCACCTGGACAGGGTCTGGAATGG
ATTGGATACATTAATCCTAGCCGTGGTTATACTAATTACGCAGACAG
CGTCAAGGGCCGCTTCACAATCACTACAGACAAATCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGACCTGCAGAGCCAGTTCAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**F) anti-CD3 (VH5/VL3)**

DVQLVQSGAEVKKPGASVKVSCKASGYTFTRYTMHWVRQAPGQGLEW
IGYINPSRGYTNYADSVKGRFTITTDKSTSTAYMELSSLRSEDTATY
YCARYYDDHYCLDYWGQGTTVTVSSGEGTSTGSGGSGGSGGADDIVL
TQSPATLSLSPGERATLTCRASSSVSYMNWYQQKPGKAPKRWIYDTS
KVASGVPARFSGSGSGTDYSLTINSLEAEDAATYYCQQWSSNPLTFG
GGTKVEIK

Figure 8

A) anti-CD3 (VH6/VL1)

GACGTCCAACTGGTGCAGTCAGGGGCTGAAGTGAAAAAACCTGGGGC
CTCAGTGAAGGTGTCCTGCAAGGCTTCTGGCTACACCTTTACTAGGT
ACACGATGCACTGGGTAAGGCAGGCACCTGGACAGGGTCTGGAATGG
ATTGGATACATTAATCCTAGCCGTGGTTATACTAATTACGCAGACAG
CGTCAAGGGCCGCTTCACAATCACTACAGACAAATCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTCAGATG
ACCCAGTCTCCATCTAGCCTGTCTGCATCTGTCGGGGACCGTGTCAC
CATCACCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

B) anti-CD3 (VH6/VL1)

DVQLVQSGAEVKKPGASVKVSCKASGYTFTRYTMHWVRQAPGQGLEW
IGYINPSRGYTNYAQKLQGRVTMTTDTSTSTAYMELSSLRSEDTATY
YCARYYDDHYCLDYWGQGTTVTVSSGEGTSTGSGGSGGSGGADDIQM
TQSPSSLSASVGDRVTITCRASQSVSYMNWYQQKPGKAPKRWIYDTS
KVASGVPARFSGSGSGTDYSLTINSLEAEDAATYYCQQWSSNPLTFG
GGTKVEIK

**Figure 8**

**C) anti-CD3 (VH6/VL2)**

GACGTCCAACTGGTGCAGTCAGGGGCTGAAGTGAAAAAACCTGGGGC
CTCAGTGAAGGTGTCCTGCAAGGCTTCTGGCTACACCTTTACTAGGT
ACACGATGCACTGGGTAAGGCAGGCACCTGGACAGGGTCTGGAATGG
ATTGGATACATTAATCCTAGCCGTGGTTATACTAATTACGCACAGAA
GTTGCAGGGCCGCGTCACAATGACTACAGACACTTCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGAGCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**D) anti-CD3 (VH6/VL2)**

DVQLVQSGAEVKKPGASVKVSCKASGYTFTRYTMHWVRQAPGQGLEW
IGYINPSRGYTNYAQKLQGRVTMTTDTSTSTAYMELSSLRSEDTATY
YCARYYDDHYCLDYWGQGTTVTVSSGEGTSTGSGGSGGSGGADDIVL
TQSPATLSLSPGERATLSCRASQSVSYMNWYQQKPGKAPKRWIYDTS
KVASGVPARFSGSGSGTDYSLTINSLEAEDAATYYCQQWSSNPLTFG
GGTKVEIK

**Figure 8**

**E) anti-CD3 (VH6/VL3)**

GACGTCCAACTGGTGCAGTCAGGGGCTGAAGTGAAAAAACCTGGGGC
CTCAGTGAAGGTGTCCTGCAAGGCTTCTGGCTACACCTTTACTAGGT
ACACGATGCACTGGGTAAGGCAGGCACCTGGACAGGGTCTGGAATGG
ATTGGATACATTAATCCTAGCCGTGGTTATACTAATTACGCACAGAA
GTTGCAGGGCCGCGTCACAATGACTACAGACACTTCCACCAGCACAG
CCTACATGGAACTGAGCAGCCTGCGTTCTGAGGACACTGCAACCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGACCTGCAGAGCCAGTTCAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**F) anti-CD3 (VH6/VL3)**

DVQLVQSGAEVKKPGASVKVSCKASGYTFTRYTMHWVRQAPGQGLEW
IGYINPSRGYTNYAQKLQGRVTMTTDTSTSTAYMELSSLRSEDTATY
YCARYYDDHYCLDYWGQGTTVTVSSGEGTSTGSGGSGGSGGADDIVL
TQSPATLSLSPGERATLTCRASSSVSYMNWYQQKPGKAPKRWIYDTS
KVASGVPARFSGSGSGTDYSLTINSLEAEDAATYYCQQWSSNPLTFG
GGTKVEIK

## Figure 9

**A) anti-CD3 (VH7/VL1)**

GACGTCCAACTGGTGCAGTCAGGGGCTGAAGTGAAAAAACCTGGGGC
CTCAGTGAAGGTGTCCTGCAAGGCTTCTGGCTACACCTTTACTAGGT
ACACGATGCACTGGGTAAGGCAGGCACCTGGACAGGGTCTGGAATGG
ATTGGATACATTAATCCTAGCCGTGGTTATACTAATTACAATCAGAA
GTTCAAGGACCGCGTCACAATCACTACAGACAAATCCACCAGCACAG
CCTACATGGAACTGAGCAGCCTGCGTTCTGAGGACACTGCAGTCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTCAGATG
ACCCAGTCTCCATCTAGCCTGTCTGCATCTGTCGGGGACCGTGTCAC
CATCACCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**B) anti-CD3 (VH7/VL1)**

DVQLVQSGAEVKKPGASVKVSCKASGYTFTRYTMHWVRQAPGQGLEW
IGYINPSRGYTNYNQKFKDRVTITTDKSTSTAYMELSSLRSEDTAVY
YCARYYDDHYCLDYWGQGTTVTVSSGEGTSTGSGGSGGSGGADDIQM
TQSPSSLSASVGDRVTITCRASQSVSYMNWYQQKPGKAPKRWIYDTS
KVASGVPARFSGSGSGTDYSLTINSLEAEDAATYYCQQWSSNPLTFG
GGTKVEIK

**Figure 9**

**C) anti-CD3(VH7/VL2)**

GACGTCCAACTGGTGCAGTCAGGGGCTGAAGTGAAAAAACCTGGGGC
CTCAGTGAAGGTGTCCTGCAAGGCTTCTGGCTACACCTTTACTAGGT
ACACGATGCACTGGGTAAGGCAGGCACCTGGACAGGGTCTGGAATGG
ATTGGATACATTAATCCTAGCCGTGGTTATACTAATTACAATCAGAA
GTTCAAGGACCGCGTCACAATCACTACAGACAAATCCACCAGCACAG
CCTACATGGAACTGAGCAGCCTGCGTTCTGAGGACACTGCAGTCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGAGCTGCAGAGCCAGTCAAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**D) anti-CD3(VH7/VL2)**

DVQLVQSGAEVKKPGASVKVSCKASGYTFTRYTMHWVRQAPGQGLEW
IGYINPSRGYTNYNQKFKDRVTITTDKSTSTAYMELSSLRSEDTAVY
YCARYYDDHYCLDYWGQGTTVTVSSGEGTSTGSGGSGGSGGADDIVL
TQSPATLSLSPGERATLSCRASQSVSYMNWYQQKPGKAPKRWIYDTS
KVASGVPARFSGSGSGTDYSLTINSLEAEDAATYYCQQWSSNPLTFG
GGTKVEIK

Figure 9

**E) anti-CD3 (VH7/VL3)**

GACGTCCAACTGGTGCAGTCAGGGGCTGAAGTGAAAAAACCTGGGGC
CTCAGTGAAGGTGTCCTGCAAGGCTTCTGGCTACACCTTTACTAGGT
ACACGATGCACTGGGTAAGGCAGGCACCTGGACAGGGTCTGGAATGG
ATTGGATACATTAATCCTAGCCGTGGTTATACTAATTACAATCAGAA
GTTCAAGGACCGCGTCACAATCACTACAGACAAATCCACCAGCACAG
CCTACATGGAACTGAGCAGCCTGCGTTCTGAGGACACTGCAGTCTAT
TACTGTGCAAGATATTATGATGATCATTACTGCCTTGACTACTGGGG
CCAAGGCACCACGGTCACCGTCTCCTCAGGCGAAGGTACTAGTACTG
GTTCTGGTGGAAGTGGAGGTTCAGGTGGAGCAGACGACATTGTACTG
ACCCAGTCTCCAGCAACTCTGTCTCTGTCTCCAGGGGAGCGTGCCAC
CCTGACCTGCAGAGCCAGTTCAAGTGTAAGTTACATGAACTGGTACC
AGCAGAAGCCGGGCAAGGCACCCAAAAGATGGATTTATGACACATCC
AAAGTGGCTTCTGGAGTCCCTGCTCGCTTCAGTGGCAGTGGGTCTGG
GACCGACTACTCTCTCACAATCAACAGCTTGGAGGCTGAAGATGCTG
CCACTTATTACTGCCAACAGTGGAGTAGTAACCCGCTCACGTTCGGT
GGCGGGACCAAGGTGGAGATCAAA

**F) anti-CD3 (VH7/VL3)**

DVQLVQSGAEVKKPGASVKVSCKASGYTFTRYTMHWVRQAPGQGLEW
IGYINPSRGYTNYNQKFKDRVTITTDKSTSTAYMELSSLRSEDTAVY
YCARYYDDHYCLDYWGQGTTVTVSSGEGTSTGSGGSGGSGGADDIVL
TQSPATLSLSPGERATLTCRASSSVSYMNWYQQKPGKAPKRWIYDTS
KVASGVPARFSGSGSGTDYSLTINSLEAEDAATYYCQQWSSNPLTFG
GGTKVEIK